Europäisches Patentamt
European Patent Office
Office européen des brevets

(19) (11) Publication number: **0 307 837 A2**

(12) **EUROPEAN PATENT APPLICATION**

(21) Application number: **88114867.0**

(22) Date of filing: **12.09.88**

(51) Int. Cl.4: **C07D 521/00 , A61K 31/33 , C07K 5/06 , C07K 5/02 , A61K 37/64**

The title of the invention has been amended (Guidelines for Examination in the EPO, A-III, 7.3).

(30) Priority: **16.09.87 US 97553**
**16.08.88 US 231869**

(43) Date of publication of application: **22.03.89 Bulletin 89/12**

(84) Designated Contracting States: **AT BE CH DE ES FR GB GR IT LI LU NL SE**

(71) Applicant: **ABBOTT LABORATORIES**

**Abbott Park Illinois 60064(US)**

(72) Inventor: **Rosenberg, Saul Howard**
**1110 Fairlawn Drive**
**Libertyville Illinois 60048(US)**
Inventor: **Sham, Hing Leung**
**5109 Pembrook Court**
**Gurnee Illinois 60031(US)**
Inventor: **Baker, William R.**
**1408 Juliet Lane**
**Libertyville Illinois 60048(US)**
Inventor: **Dellaria, Joseph F., Jr.**
**2512 Timber Lane**
**Lindenhurst Illinois 60046(US)**
Inventor: **Kempf, Dale J.**
**205 Chelsea Circle**
**Lake Villa Illinois 60046(US)**

(74) Representative: **Modiano, Guido et al**
**MODIANO, JOSIF, PISANTY & STAUB**
**Modiano & Associati Baaderstrasse 3**
**D-8000 München 5(DE)**

(54) **Renin-inhibiting peptidyl heterocycles.**

(57) A renin inhibiting compound of the formula:

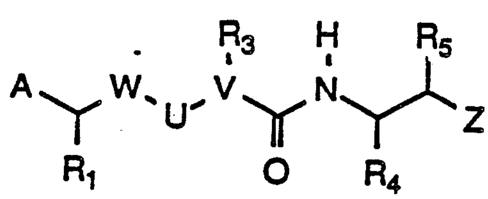

wherein A is a substituent; W is C=O, CHOH or $NR_2$ wherein $R_2$ is hydrogen or loweralkyl; U is C=O, $CH_2$ or $NR_2$ wherein $R_2$ is hydrogen or loweralkyl, with the proviso that when W is CHOH then U is $CH_2$ and with the proviso that U is C=O or $CH_2$ when W is $NR_2$; V is CH, C(OH) or C(halogen) with the proviso that V is CH when U is $NR_2$; $R_1$ is loweralkyl, cycloalkylalkyl, benzyl, (alpha, alpha)-dimethylbenzyl, 4-methoxybenzyl, halobenzyl, 4-hydroxybenzyl, (1-naphthyl)methyl, (2-naphthyl)methyl, (unsubstituted heterocyclic)-

EP 0 307 837 A2

methyl, (substituted heterocyclic)methyl, phenethyl, 1-benzyloxyethyl, phenoxy, thiophenoxy or anilino, provided that B is $CH_2$ or CHOH or A is hydrogen when $R_1$ is phenoxy, thiophenoxy or anilino; $R_3$ is loweralkyl, loweralkenyl, ((alkoxy)alkoxy)alkyl, carboxyalkyl, (thioalkoxy)alkyl, azidoalkyl, aminoalkyl, (alkyl)-aminoalkyl, dialkylaminoalkyl, (alkoxy)(alkyl)aminoalkyl, (alkoxy)aminoalkyl, benzyl or heterocyclic ring substituted methyl; $R_4$ is loweralkyl, cycloalkylmethyl or benzyl; $R_5$ is OH or $NH_2$; and Z is a substituent.

Also disclosed are compositions for and a method of treating hypertension, methods of making the renin inhibiting compounds and intermediates useful in making the renin inhibiting compounds.

# PEPTIDYLHETEROCYCLES

## Technical Field

This is a continuation-in-part of U.S. patent application Serial No. 097,553, filed September 16, 1987.

The present invention relates to novel organic compounds and compositions which inhibit renin, processes for making such compounds, synthetic intermediates employed in these processes and a method of treating hypertension with such compounds.

## Background Art

Renin is a proteolytic enzyme synthesized and stored principally in a specific part of the kidney called the juxtaglomerular apparatus. Any of three different physiologic circumstances may cause the release of renin into the circulation: (a) a decrease in the blood pressure entering or within the kidney itself; (b) a decrease in the blood volume in the body; or (c) a fall in the concentration of sodium in the distal tubules of the kidney.

When renin is released into the blood from the kidney, the renin-angiotensin system is activated, leading to vasoconstriction and conservation of sodium, both of which result in increased blood pressure. The renin acts on a circulating protein, angiotensinogen, to cleave out a fragment called angiotensin I (AI). AI itself has only slight pharamacologic activity but, after additional cleavage by a second enzyme, angiotensin converting enzyme (ACE), forms the potent molecule angiotensin II (AII). The major pharmacological effects of AII are vasoconstriction and stimulation of the adrenal cortex to release aldosterone, a hormone which causes sodium retention. Sodium retention causes blood volume to increase, which leads to hypertension. AII is cleaved by an aminopeptidase to form angiotensin III (AIII), which, compared to AII, is a less potent vasoconstrictor but a more potent inducer of aldosterone release.

Inhibitors of renin have been sought as agents for control of hypertension and as diagnostic agents for identification of cases of hypertension due to renin excess.

With these objectives in mind, the renin-angiotensin system has been modulated or manipulated, in the past, with ACE inhibitors. However, ACE acts on several substrates other than angiotensin I (AI), most notably the kinins which cause such undesirable side effects as pain, "leaky" capillaries, prostaglandin release and a variety of behavorial and neurologic effects. Further, ACE inhibition leads to the accumulation of AI. Although AI has much less vasoconstrictor activity than AII, its presence may negate some of the hypotensive effects of the blockade of AII synthesis.

Inhibition of other targets in the renin-angiotensin system such as AII with compounds such as saralasin can block AII activity, but would leave unimpaired and perhaps enhance the hypertensive effects of AIII.

On the other hand, there are no known side effects which result when renin is inhibited from acting on its substrate. Considerable research efforts have thus been carried out to develop useful inhibitors of renin. Past research efforts have been directed to renin antibodies, pepstatin, phospholipids and substrate analogs such as tetrapeptides and octapeptides to tridecapeptides. These inhibitors either demonstrate poor activity in inhibiting renin production or poor specificity for inhibiting renin only. However, Boger et al. have reported that statine-containing peptides possess potent and specific renin-inhibiting activity (Nature, Vol. 303, p. 81, 1983). In addition, Szelke and co-workers have described polypeptide analogs containing a non-peptide link (Nature, Vol. 299, p. 555, 1982) which also cause potent renin inhibition and show a high specificity for this enzyme.

Disclosure of the Invention

In accordance with the present invention, there are renin inhibiting compounds of the formula:

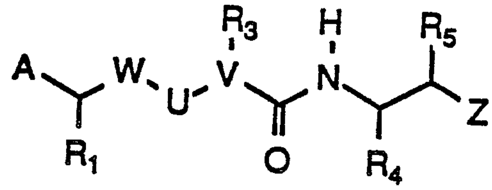

wherein A is hydrogen, loweralkyl, aminoalkyl, (alkyl)aminoalkyl, dialkylaminoalkyl, (alkoxy)aminoalkyl, (alkoxy)(alkyl)aminoalkyl, phenylalkyl, (substituted phenyl)alkyl wherein the phenyl ring is substituted with one, two or three substituents independently selected from loweralkoxy, loweralkyl, amino, alkylamino, dialkylamino, hydroxy, halo, mercapto, nitro, thioalkoxy, carboxaldehyde, carboxy, alkoxycarbonyl and carboxamide, naphthylalkyl, (substituted naphthyl)alkyl wherein the naphthyl ring is substituted with one, two or three substituents, independently selected from loweralkoxy, loweralkyl, amino, alkylamino, dialkylamino, hydroxy, halo, mercapto, nitro, thioalkoxy, carboxaldehyde, carboxy, alkoxycarbonyl and carboxamide, substituted or unsubstituted heterocyclic, where saturated heterocyclics may be unsubstituted, monosubsituted or disubstituted with hydroxy, oxo, amino, alkylamino, dialkylamino, alkoxy, polyalkoxy, loweralkyl, haloalkyl or polyhaloalkyl; unsaturated heterocyclics may be unsubstituted or monosubstituted with hydroxy, amino, alkylamino, dialkylamino, alkoxy, polyalkoxy, loweralkyl, haloalkyl or polyhaloalkyl, or A is (unsubstituted heterocyclic)alkyl or (substituted heterocyclic)alkyl wherein unsubstituted or substituted heterocyclic is as defined above, or A is $-OR_7$ or $-SR_7$ wherein $R_7$ is hydrogen, loweralkyl, aminoalkyl, (alkyl)aminoalkyl, dialkylaminoalkyl, (alkoxy)aminoalkyl, (alkoxy)(alkyl)aminoalkyl, phenylalkyl, (substituted phenyl)alkyl wherein substituted phenyl is as defined above, naphthylalkyl, (substituted naphthyl)alkyl wherein the substituted naphthyl is as defined above, substituted or unsubstituted heterocyclic as defined above (unsubstituted heterocyclic)alkyl or (substituted heterocyclic)alkyl wherein unsubstituted or substituted heterocyclic is as defined above, (unsubstituted heterocyclic)C(O)- or (substituted heterocyclic)C(O)- wherein unsubstituted or substituted heterocyclic is as defined above; or A is $-NR_8R_9$ wherein $R_8$ and $R_9$ are independently selected from hydrogen, hydroxy, alkoxy, loweralkyl, aminoalkyl, cyanoalkyl and hydroxyalkyl; or A is

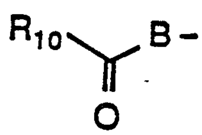

or

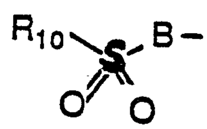

wherein B is NH, alkylamino, S, O, $CH_2$, $NHCH_2$ or $CH(OR_{52})$ wherein $R_{52}$ is hydrogen, loweralkyl or loweralkylcarbonyl, and $R_{10}$ is hydrogen, loweralkyl, cycloalkyl, phenyl, substituted phenyl as defined above, naphthyl, substituted naphthyl as defined above, alkoxy, alkenyloxy, hydroxyalkoxy, dihydroxyalkoxy, phenylalkoxy, (substituted phenyl)alkoxy wherein substituted phenyl is as defined above, naphthylalkoxy, (substituted naphthyl)alkoxy wherein substituted naphthyl is as defined above, phenylalkoxyalkyl, (substituted phenyl)alkoxyalkyl wherein substituted phenyl is as defined above, naphthylalkoxyalkyl, (substituted naphthyl)alkoxyalkyl wherein substituted naphthyl is as defined above, thioalkoxyalkyl, loweralkylsulfinylalkyl, loweralkylsulfonylalkyl, phenylthioalkyl, (substituted phenyl)thioalkyl wherein substituted phenyl is as defined above, naphthylthioalkyl, (substituted naphthyl)thioalkyl wherein substituted naphthyl is as defined above, phenylsulfonylalkyl, (substituted phenyl)sulfonylalkyl wherein substituted phenyl is as defined above, naphthylsulfonylalkyl, (substituted naphthyl)sulfonylalkyl wherein substituted naphthyl is as defined above, amino, alkylamino, dialkylamino, (hydroxyalkyl)(alkyl)amino, (dihydroxyalkyl)(alkyl)amino, aminoalkyl, alkoxycarbonylalkyl, carboxyalkyl, (N-protected)aminoalkyl, alkylaminoalkyl, (N-protected)(alkyl)-aminoalkyl, dialkylaminoalkyl, (heterocyclic)alkyl, a substituted or unsubstituted heterocyclic as defined above, aminocycloalkyl, aminoalkylamino, (dialkylaminoalkyl)(alkyl)amino, phenylalkylamino, (substituted phenyl)alkylamino wherein substituted phenyl is as defined above, naphthylalkylamino, (substituted naphthyl)alkylamino wherein substituted naphthyl is as defined above, (phenylalkyl)(alkyl)amino, ((substituted phenyl)alkyl)(alkyl)amino wherein substituted phenyl is as defined above, (naphthylalkyl)(alkyl)amino, (-

(substituted naphthyl)alkyl)(alkyl)amino wherein substituted naphthyl is as defined above, alkoxyalkyl(alkyl)-amino, (polyalkoxy)alkyl(alkyl)amino, di-(alkoxyalkyl)amino, di-(hydroxyalkyl)amino, di-((polyalkoxy)alkyl)-amino, ((heterocyclic)alkyl)(alkyl)amino, ((heterocyclic)alkyl)amino, (heterocyclic)(alkyl)amino, (alkylaminoalkyl)(alkyl)amino, (dialkylaminoalkyl)(alkyl)amino, ((alkoxy)(alkyl)aminoalkyl)(alkyl)amino, (-(alkoxy)aminoalkyl)(alkyl)amino, polyalkoxy or (polyalkoxy)alkyl; or A is $R_{41}CH(OH)CH_2$- or $R_{41}CH(OH)CH(OH)$- wherein $R_{41}$ is loweralkyl, cycloalkyl, phenyl, substituted phenyl as defined above, naphthyl, substituted naphthyl as defined above, phenylalkyl, (substituted phenyl)alkyl wherein substituted phenyl is as defined above, naphthylalkyl, (substituted naphthyl)alkyl wherein substituted naphthyl is as defined above, phenylalkoxyalkyl, (substituted phenyl)alkoxyalkyl wherein substituted phenyl is as defined above, naphthylalkoxyalkyl, (substituted naphthyl)alkoxyalkyl wherein substituted naphthyl is as defined above, thioalkoxyalkyl, loweralkylsulfinylalkyl, loweralkylsulfonylalkyl, phenylthioalkyl, (substituted phenyl)thioalkyl wherein substituted phenyl is as defined above, naphthylthioalkyl, (substituted naphthyl)thioalkyl wherein substituted naphthyl is as defined above, phenylsulfonylalkyl, (substituted phenyl)sulfonylalkyl wherein substituted phenyl is as defined above, naphthylsulfonyalkyl, (substituted naphthyl)sulfonylalkyl wherein substituted naphthyl is as defined above, aminoalkyl, alkoxycarbonylalkyl, carboxyalkyl, (N-protected)-aminoalkyl, alkylaminoalkyl, (N-protected)(alkyl)aminoalkyl, dialkylaminoalkyl, heterocyclicalkyl, a substituted or unsubstituted heterocyclic as defined above, aminocycloalkyl or (polyalkoxy)alkyl;

W is C=O, CHOH or $NR_2$ wherein $R_2$ is hydrogen or loweralkyl;

U is C=O, $CH_2$ or $NR_2$ wherein $R_2$ is hydrogen or loweralkyl, with the proviso that when W is CHOH then U is $CH_2$ and with the proviso that U is C=O or $CH_2$ when W is $NR_2$;

V is CH, C(OH) or C(halogen) with the proviso that V is CH when U is $NR_2$;

$R_1$ is loweralkyl, cycloalkylalkyl, benzyl, (alpha, alpha)-dimethylbenzyl, 4-methoxybenzyl, halobenzyl, 4-hydroxybenzyl, (1-naphthyl)methyl (2-naphthyl)methyl, (unsubstituted heterocyclic)methyl, (substituted heterocyclic)methyl wherein unsubstituted or substituted heterocyclic is as defined above, phenethyl, 1-benzyloxyethyl, phenoxy, thiophenoxy or anilino, provided that B is $CH_2$ or CHOH or A is hydrogen when $R_1$ is phenoxy, thiophenoxy or anilino;

$R_3$ is loweralkyl, loweralkenyl, ((alkoxy)alkoxy)alkyl, carboxyalkyl, (thioalkoxy)alkyl, azidoalkyl, aminoalkyl, (alkyl)aminoalkyl, dialkylaminoalkyl, (alkoxy)(alkyl)aminoalkyl, (alkoxy)aminoalkyl, benzyl or heterocyclic ring substituted methyl;

$R_4$ is loweralkyl, cycloalkylmethyl or benzyl;

$R_5$ is Oh or $NH_2$; and

Z is

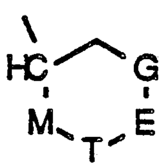

or

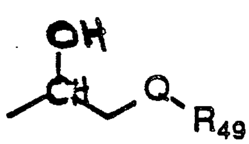

wherein M is O, S or NH, T is C=O, C=S, S(O), $S(O)_2$ or $CH_2$, E is O, S, $NR_6$ wherein $R_6$ is hydrogen, loweralkyl, hydroxyalkyl, hydroxy, alkoxy, amino, or alkylamino, or E is $CR_6R_{42}$ wherein $R_6$ is as defined above and $R_{42}$ is hydrogen or loweralkyl or E is $C=CR_{43}R_{44}$ wherein $R_{43}$ and $R_{44}$ are independently selected from hydrogen and loweralkyl, G is absent, $CH_2$, or $NR_{11}$ wherein $R_{11}$ is hydrogen or loweralkyl, with the proviso that when G is $NR_{11}$ then $R_6$ is loweralkyl or hydroxyalkyl, Q is $CR_{45}R_{46}$ wherein $R_{45}$ and $R_{46}$ are independently selected from hydrogen and loweralkyl or Q is $C=CR_{47}R_{48}$ wherein $R_{47}$ and $R_{48}$ are independently selected from hydrogen and loweralkyl, and $R_{49}$ is $-CH_2OH$, carboxy, alkoxycarbonyl or $-CONR_{50}R_{51}$ wherein $R_{50}$ is hydrogen or loweralkyl and $R_{51}$ is hydrogen, loweralkyl, aminoalkyl, alkylaminoalkyl, dialkylaminoalkyl or alkoxyalkyl; or pharmaceutically acceptable salts or esters thereof.

The chiral centers of the compounds of the invention may have either the "R" or "S" configuration. The

terms "R" and "S" configuration are as defined by IUPAC 1974 Recommendations for Section E, Fundamental Stereochemistry, Pure Appl. Chem. (1976) 45, 13-30.

The term "loweralkyl" as used herein refers to straight or branched chain alkyl radicals containing from 1 to 7 carbon atoms including but not limited to methyl, ethyl, n-propyl, iso-propyl, n-butyl, iso-butyl, sec-butyl, n-pentyl, 2-methylbutyl, 2,2-dimethylpropyl, n-hexyl, 2-methylpentyl, 2,2-dimethylbutyl, n-heptyl, and the like.

The term "cycloalkyl" as used herein refers to an alicyclic ring having 3 to 7 carbon atoms.

The term "cycloalkylalkyl" as used herein refers to a cycloalkyl residue appended to a loweralkyl radical and includes but is not limited to cyclohexylmethyl and cyclopentylmethyl.

The term "phenylalkyl" as used herein refers to a phenyl group appended to a loweralkyl radical, including, but not limited to benzyl, phenethyl and the like.

The term "(substituted phenyl)alkyl" as used herein refers to a substituted phenyl group appended to a loweralkyl radical wherein the phenyl ring is substituted with one, two or three substituents chosen from the group loweralkoxy, loweralkyl, amino, loweralkylamino, hydroxy, halo, mercapto, nitro, thioalkoxy, carboxaldehyde, carboxy, carboalkoxy and carboxamide, including, but not limited to halobenzyl, alkoxybenzyl and the like.

The term "naphthylalkyl" as used herein refers to a naphthyl group appended to a loweralkyl radical, including, but not limited to 1-naphthylmethyl, 2-naphthylmethyl and the like.

The term "(substituted naphthyl)alkyl" as used herein refers to a substituted naphthyl group appended to a loweralkyl radical wherein the naphthyl ring is substituted with one, two or three substituents chosen from the group loweralkoxy, loweralkyl, amino, loweralkylamino, hydroxy, halo, mercapto, nitro, thioalkoxy, carboxaldehyde, carboxy, carboalkoxy and carboxamide, including, but not limited to halonaphthylmethyl, alkoxynaphthylmethyl and the like.

The term "heterocyclicalkyl" as used herein refers to an unsubstituted or substituted heterocyclic ring as defined below appended to a lower alkyl radical, including, but not limited to imidazolylmethyl, thiazolylmethyl and the like.

The term "hydroxyalkyl" as used herein refers to -OH appended to a loweralkyl radical.

The term "alkoxyalkyl" as used herein refers to an alkoxy group appended to a loweralkyl radical.

The term "thioalkoxyalkyl" as used herein refers to a thioalkoxy group appended to a loweralkyl radical.

The term "phenylalkoxyalkyl" as used herein refers to a phenylalkoxy group appended to a loweralkyl radical, including, but not limited to phenylmethoxymethyl and the like.

The term "(substituted phenyl)alkoxyalkyl" as used herein refers to a (substituted phenyl)alkoxy group appended to a loweralkyl radical, including, but not limited to 4-chlorophenylmethoxymethyl.

The term "naphthylalkoxyalkyl" as used herein refers to a naphthylalkoxy group appended to a loweralkyl radical, including, but not limited to 1-naphthylmethoxymethyl and the like.

The term "(substituted naphthyl)alkoxyalkyl" as used herein refers to a (substituted naphthyl)alkoxy group appended to a loweralky radical, including, but not limited to halonaphthylmethoxymethyl and the like.

The term "((alkoxy)alkoxy)alkyl" as used herein refers to an alkoxy group appended to an alkoxy group which is appended to a loweralkyl radical, including, but not limited to methoxymethoxymethyl and the like.

The term "polyalkoxyalkyl" as used herein refers to a polyalkoxy residue appended to a loweralkyl radical, including, but not limited to methoxyethoxymethoxymethyl and the like.

The term "aminoalkyl" as used herein refers to $-NH_2$ appended to a loweralkyl radical.

The term "alkylaminoalkyl" as used herein refers to $-NHR_{12}$ appended to a loweralkyl radical, wherein $R_{12}$ is a loweralkyl radical.

The term "dialkylaminoalkyl" as used herein refers to a dialkylamino group appended to a loweralkyl radical.

The term "aminocycloalkyl" as used herein refers to an $-NH_2$ appended to a cycloalkyl radical.

The terms "(N-protected)aminoalkyl" as used herein refers to $-NHR_{13}$ appended to a loweralkyl group, wherein $R_{13}$ is an N-protecting group.

The term "(N-protected)(alkyl)aminoalkyl" as used herein refers to $-NR_{13}R_{14}$, which is appended to a loweralkyl radical, wherein $R_{13}$ is defined as above and $R_{14}$ is a loweralkyl group.

The term "alkoxycarbonylalkyl" as used herein refers to $R_{15}COR_{16}$-, wherein $R_{15}$ is an alkoxy group and $R_{16}$ is a loweralkyl radical.

The term "carboxyalkyl" as used herein refers to a carboxylic acid group (-COOH) appended to a loweralkyl radical.

The term "cyanoalkyl" as used herein refers to -CN appended to a loweralkyl radical.

The term "(alkoxy)aminoalkyl" as used herein refers to an alkoxy group appended to an amino group

which in turn is appended to a loweralkyl radical.

The term "(alkoxy)(alkyl)aminoalkyl" as used herein refers to an $-NR_{59}R_{60}$ group appended to a loweralkyl radical wherein $R_{59}$ is an alkoxy group and $R_{60}$ is a loweralkyl group.

The term "haloalkyl" as used herein refers to a loweralkyl radical substituted with a halogen, including, but not limited to fluoromethyl, 2-chloroethyl and the like.

The term "polyhaloalkyl" as used herein refers to a loweralkyl radical substituted with two or more halogens, including, but not limited to trifluoromethyl, 2,2-dichloroethyl and the like.

The term "azidoalkyl" as used herein refers to a $-N_3$ group appended to a loweralkyl radical.

The term "loweralkylsulfinylalkyl" as used herein refers to a $R_{61}S(O)$- group appended to a loweralkyl radical wherein $R_{61}$ is a loweralkyl group.

The term "loweralkylsulfonylalkyl" as used herein refers to a $R_{62}S(O)_2$- group appended to a loweralkyl radical wherein $R_{62}$ is a loweralkyl group.

The term "phenylthioalkyl" as used herein refers to a $R_{63}S$- group appended to a loweralkyl radical wherein $R_{63}$ is a phenyl group.

The term "(substituted phenyl)thioalkyl" as used herein refers to a $R_{64}S$- group appended to a loweralkyl radical wherein $R_{64}$ is a substituted phenyl group.

The term "naphthyl thioalkyl" as used herein refers to a $R_{65}S$- group appended to a loweralkyl radical wherein $R_{65}$ is a naphthyl group.

The term "(substituted naphthyl)thioalkyl" as used herein refers to a $R_{66}S$- group appended to a loweralkyl radical wherein $R_{66}$ is a substituted naphthyl group.

The term "phenylsulfonylalkyl" as used herein refers to a $R_{67}S(O)_2$- group appended to a loweralkyl radical wherein $R_{67}$ is a phenyl group.

The term "(substituted phenyl)sulfonylalkyl" as used herein refers to a $R_{68}S(O)_2$- group appended to a loweralkyl radical wherein $R_{68}$ is a substituted phenyl group.

The term "naphthylsulfonylalkyl" as used herein refers to a $R_{69}S(O)_2$- group appended to a loweralkyl group wherein $R_{69}$ is a naphthyl group.

The term "(substituted naphthyl)sulfonylalkyl" as used herein refers to a $R_{70}S(O)_2$- group appended to a loweralkyl group wherein $R_{70}$ is a substituted naphthyl group.

The term "amino" as used herein refers to an $-NH_2$ substituent.

The term "alkylamino" as used herein refers to $-NHR_{17}$, wherein $R_{17}$ is a loweralkyl group.

The term "dialkylamino" as used herein refers to $-NR_{18}R_{19}$, wherein $R_{18}$ and $R_{19}$ are independently selected from loweralkyl groups.

The term "phenylalkylamino" as used herein refers to a phenylalkyl group appended to an amino radical, including, but not limited to benzylamino and the like.

The term "(substituted phenyl)alkylamino" as used herein refers to a (substituted phenyl)alkyl group appended to an amino radical, including, but not limited to 4-chlorobenzylamino and the like.

The term "naphthylalkylamino" as used herein refers to a naphthylalkyl group appended to an amino radical, including, but not limited to 1-naphthylmethylamino and the like.

The term "(substituted naphthyl)alkylamino" as used herein refers to a (substituted naphthyl)alkyl group appended to an amino radical.

The term "(phenylalkyl)(alkyl)amino" as used herein refers to $R_{21}R_{22}N$-, wherein $R_{21}$ is a phenylalkyl residue and $R_{22}$ is a loweralkyl residue.

The term "((substituted phenyl)alkyl)(alkyl)amino" as used herein refers to $R_{53}R_{54}N$-wherein $R_{53}$ is a (substituted phenyl)alkyl group and $R_{54}$ is a loweralkyl group.

The term "(naphthylalkyl)(alkyl)amino" as used herein refers to $R_{55}R_{56}N$- wherein $R_{55}$ is a naphthylalkyl group and $R_{56}$ is a loweralkyl group.

The term "((substituted naphthyl)alkyl)(alkyl)amino" as used herein refers to $R_{57}R_{58}N$-wherein $R_{57}$ is a (substituted naphthyl)alkyl group and $R_{58}$ is a loweralkyl group.

The term "aminoalkylamino" as used herein refers to $R_{23}NH$-, where $R_{23}$ is an aminoalkyl residue.

The term "(dialkylaminoalkyl)(alkyl)amino" as used herein refers to $R_{24}R_{25}N$-, wherein $R_{24}$ is a dialkylamino residue appended to a loweralkyl group and $R_{25}$ is a loweralkyl group.

The term "(hydroxyalkyl)(alkyl)amino" as used herein refers to $-NR_{26}R_{27}$ wherein $R_{26}$ is a hydroxyalkyl group and $R_{27}$ is a loweralkyl group.

The term "(di-hydroxyalkyl)(alkyl)amino" as used herein refers to a loweralkyl group which is disubstituted with -OH radicals appended to an amino group, which amino group also has appended another loweralkyl group.

The term "di-(hydroxyalkyl)amino" as used herein refers to $R_{28}R_{29}N$-, wherein $R_{28}$ and $R_{29}$ are hydroxyalkyl residues.

The term "(alkoxyalkyl)(alkyl)amino" as used herein refers to $R_{30}R_{31}N$-, wherein $R_{30}$ is an alkoxyalkyl group and $R_{31}$ is a loweralkyl group.

The term "di-(alkoxyalkyl)amino" as used herein refers to $R_{32}R_{33}N$-, wherein $R_{32}$ and $R_{33}$ are alkoxy groups appended to a loweralkyl residue.

The term "di-(polyalkoxyalkyl)amino" as used herein refers to $R_{34}R_{35}N$-, wherein $R_{34}$ and $R_{35}$ are polyalkoxy residues appended to loweralkyl residues.

The term "((polyalkoxy)alkyl)(alkyl)amino" as used herein refers to $R_{36}R_{37}N$-, wherein $R_{36}$ is a polyalkoxy residue appended to a loweralkyl radical and $R_{37}$ is a loweralkyl residue.

The term "((heterocyclic)alkyl)(alkyl)amino" as used herein refers to $-NR_{71}R_{72}$ wherein $R_{71}$ is a heterocyclicalkyl group and $R_{72}$ is a loweralkyl group.

The term "(heterocyclicalkyl)amino" as used herein refers to $-NHR_{73}$ wherein $R_{73}$ is a heterocyclic alkyl group.

The term "(heterocyclic)(alkyl)amino" as used herein refers to $-NR_{74}R_{75}$ wherein $R_{74}$ is a substituted or unsubstituted heterocyclic group and $R_{75}$ is a loweralkyl group.

The term "(alkylaminoalkyl)(alkyl)amino" as used herein refers to $-NR_{76}R_{77}$ wherein $R_{76}$ is an alkylaminoalkyl group and $R_{77}$ is a loweralkyl group.

The term "(dialkylaminoalkyl)(alkyl)amino" as used herein refers to $-NR_{78}R_{79}$ wherein $R_{78}$ is a dialkylaminoalkyl group and $R_{79}$ is a loweralkyl group.

The term "((alkoxy)(alkyl)aminoalkyl)(alkyl)amino" as used herein refers to $-NR_{80}R_{81}$ wherein $R_{80}$ is $-NR_{82}R_{83}$ appended to a loweralkyl radical wherein $R_{82}$ is an alkoxy group and $R_{83}$ is a loweralkyl group and $R_{81}$ is a loweralkyl group.

The term "((alkoxy)aminoalkyl)(alkyl)amino" as used herein refers to $-NR_{84}R_{85}$ wherein $R_{84}$ is $-NHR_{86}$ appended to a loweralkyl group and wherein $R_{86}$ is an alkoxy group and $R_{85}$ is a loweralkyl group.

The term "loweralkylcarbonyl" as used herein refers to $R_{87}C(O)$- wherein $R_{87}$ is a loweralkyl group, including, but not limited to acetyl, propionyl and the like.

The terms "alkoxy" and "thioalkoxy" as used herein refer to $R_{38}O$- and $R_{38}S$-, respectively, wherein $R_{38}$ is a loweralkyl group.

The term "alkenyloxy" as used herein refers to $R_{39}O$-, wherein $R_{39}$ is an alkyl group of 1 to 7 carbon atoms which contains at least one double bond.

The term "hydroxyalkoxy" as used herein refers to -OH appended to an alkoxy radical.

The term "dihydroxyalkoxy" as used herein refers to an alkoxy radical which is disubstituted with -OH radicals.

The term "phenylalkoxy" as used herein refers to a phenyl group appended to an alkoxy radical, including, but not limited to benzyloxy and the like.

The term "(substituted phenyl)alkoxy" as used herein refers to a substituted phenyl group appended to an alkoxy radical, including, but not limited to 4-chlorobenzyloxy and the like.

The term "naphthylalkoxy" as used herein refers to a naphthyl group appended to an alkoxy radical.

The term "(substituted naphthyl)alkoxy" as used herein refers to a substituted naphthyl group appended to an alkoxy radical.

The term "polyalkoxy" as used herein refers to $R_{40}O$-, wherein $R_{40}$ is a straight or branched chain containing 1-5, $C_n$-O-$C_{n'}$ linkages wherein n and n′ are independently 1-3.

The term "halo" as used herein refers to Cl, Br, F or I substituents.

The term "halobenzyl" as used herein refers to a halo substituent appended to the phenyl ring of a benzyl radical.

The term "halophenyl" as used herein refers to a halo substituent appended to a phenyl radical.

The term "substituted phenyl" as used herein refers to a phenyl ring substituted with one, two or three substituents chosen from the group loweralkoxy, loweralkyl, amino, loweralkylamino, hydroxy, halo, mercapto, nitro, thioalkoxy, carboxaldehyde, carboxy, carboalkoxy and carboxamide, including, but not limited to halophenyl, loweralkylphenyl, alkoxyphenyl and the like.

The term "substituted naphthyl" as used herein refers to a naphthyl ring substituted with one, two or three substituents chosen from the group loweralkoxy, loweralkyl, amino, loweralkylamino, hydroxy, halo, mercapto, nitro, thioalkoxy, carboxaldehyde, carboxy, carboalkoxy and carboxamide, including, but not limited to halonaphthyl, alkoxynaphthyl and the like.

The term "heterocyclic group" or "heterocyclic" as used herein refers to any 5-, 6-membered ring containing from one to three heteroatoms selected from the group consisting of nitrogen, oxygen, and sulfur; wherein the 5-membered ring has 0-2 double bonds and the 6-membered ring has 0-3 double bonds; wherein the nitrogen and sulfur heteroatoms may optionally be oxidized; wherein the nitrogen heteroatom may optionally be quaternized; and including any bicyclic group in which any of the above heterocyclic

rings are fused to a benzene ring. Heterocyclics in which nitrogen is the heteroatom are preferred. Fully saturated heterocyclics are also preferred. Preferred heterocyclics include: pyrryl, pyrrolinyl, pyrrolidinyl, pyrazolyl, pyrazolinyl, pyrazolidinyl, imidazolyl, imidazolinyl, imidazolidinyl, pyridyl, piperidinyl, pyrazinyl, piperazinyl, pyrimidinyl, pyridazinyl, oxazolyl, oxazolidinyl, isoxazolyl, isoxazolidinyl, morpholinyl, thiazolyl, thiazolidinyl, isothiazolyl, isothiazolidinyl, indolyl, quinolinyl, isoquinolinyl, benzimidazolyl, benzothiazolyl, benzoxazolyl, furyl, thienyl and benzothienyl.

Saturated heterocyclics may be unsubstituted, monosubstituted or disubstituted with hydroxy, oxo, amino, alkylamino, dialkylamino, alkoxy, polyalkoxy, loweralkyl, haloalkyl or polyhaloalkyl. Unsaturated heterocyclics may be unsubstituted or monosubstituted with hydroxy, amino, alkylamino, dialkylamino, alkoxy, polyalkoxy, loweralkyl, haloalkyl or polyhaloalkyl.

The most preferred heterocyclics include the following:

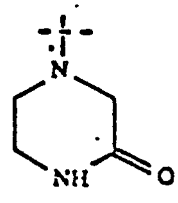 , 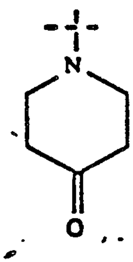 , 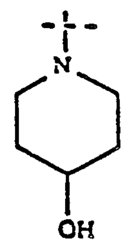 , 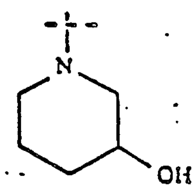 ,

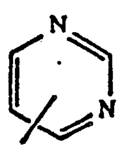 , 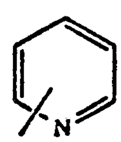 , 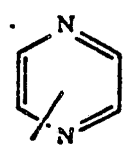 , 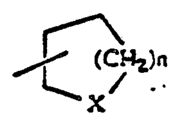 , 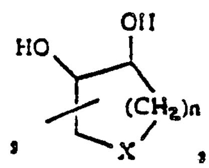 ,

wherein n is 1 or 2 and X is N, NH, O, S, provided that X is the point of connection only when X is N,

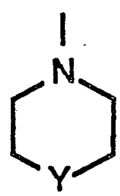

wherein Y is NH, N-loweralkyl, O, S, or $SO_2$, or

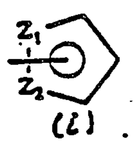 , 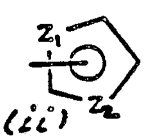 , 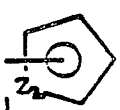 ,

(i) (ii) (iii)

wherein the symbols (i), (ii) and (iii) represent 5-membered heterocycles containing one or more heteroatoms and containing 2 double bonds; wherein $Z_1$ is N, O, or S and not the point of connection and $Z_2$ is N when it is the point of connection and NH, O or S when it is not the point of connection.

The term "N-protecting group" or "N-protected" as used herein refers to those groups intended to protect the N-terminus of an amino acid or peptide or to protect an amino group against undesirable reactions during synthetic procedures or to prevent the attack of exopeptidases on the compounds or to increase the solubility of the compounds and includes but is not limited to sulfonyl, acyl, acetyl, pivaloyl, t-butyloxycarbonyl (Boc), carbonylbenzyloxy (Cbz), benzoyl or an L- or D-aminoacyl residue, which may itself be N-protected similarly.

The term O-protecting group" as used herein refers to a substituent which protects hydroxyl groups against undesirable reactions during synthetic procedures and includes but is not limited to substituted methyl ethers, for example methoxymethyl, benzylozymethyl, 2-methoxyethoxymethyl, 2-(trimethylsilyl)-ethoxymethyl, benzyl and triphenylmethyl; tetrahydropyranyl ethers; substituted ethyl ethers, for example,

2,2,2-trichloroethyl and t-butyl; silyl ethers, for example, trimethylsilyl, t-butyldimethylsilyl and t-butyldiphenylsilyl; cyclic acetals and ketals, for example, methylene acetal, acetonide and benzylidene acetal; cyclic ortho esters, for example, methoxymethylene; cyclic carbonates; and cyclic boronates.

The terms "Ala", "His", "Leu", "Phe", "Tyr", "Cys", "Gly", "Lys", "Sar", and "Pro" as used herein refer to alanine, histidine, leucine, phenylalanine, tyrosine, cysteine, glycine, lysine, sarcosine and proline, respectively. In general, the amino acid abbreviations follow the IUPAC-IUB Joint Commission on Biochemical Nomenclature for amino acids and peptides (Eur. J. Biochem. 1984, 158, 9-31).

The compounds of the invention may be made as shown in Scheme 1, Scheme 2 and Scheme 3. Compound (IV) can serve as a common intermediate for the synthetic processes disclosed.

In particular, the process shown in Scheme 1 discloses an N-protected amino acid ester (I) which is reduced to the aldehyde and condensed with a vinyl anion equivalent to provide allylic alcohol (II) as an RS mixture at the hydroxyl center. Closure to the oxazolidone and isomer separation, followed by basic hydrolysis, affords the isomerically pure allylic alcohol (III). O- and N- protection of (III) yields (IV).

The process shown in Scheme 2 discloses bis-hydroxylation of allylic alcohol (IV) to provide a diol intermediate which is reacted with a diactivated carbonyl reagent to provide carbonate 11. Alternatively, allylic alcohol (IV) may be epoxidized to (V) which is reacted with cyanide to give (VI) as an RS mixture at the free hydroxyl center. One isomer of (VI) is reduced to the free amine and then reacted with a diactivated carbonyl reagent to provide urethane 17. The other isomer of (VI) may also be reduced and the hydroxyl converted to an amine with inversion of stereochemistry. This amine is then reacted with a diactivated carboxyl reagent to provide urea 21. Epoxide (V) can also be reacted with monosubstituted amines to give intermediate amino-alcohols which are reacted with diactivated carbonyl reagents to provide urethanes 7, 8, 9, 28 and 104. The intermediate amino-alcohol can also be converted to a 1, 2-diamine via azide (VII). The 1, 2-diamine gives urea 14 after reaction with a diactivated carbonyl reagent. Reaction of epoxide (V) with a 1-substituted - 2-protected hydrazine derivative provides intermediate (VIII), which is deprotected and reacted with a diactivated carbonyl reagent to provide urethane 24.

Heterocycles 7, 8, 9, 11, 14, 17, 21, 24, 28 and 104 are deprotected and coupled to peptide segments, which are described in Examples 30-103 or generally disclosed herein. The peptide segments are either purchased commercially or are made by analogy to the examples disclosed herein). The coupling reaction is accomplished using either the mixed anhydride method (employing isobutyl chloroformate and N-methylmorpholine) or the carbodiimide method (employing N-ethyl-N′-(3-dimethylaminopropyl) carbodiimide, 1-hydroxybenzotriazole, and N-methylmorpholine in dimethylformamide) to give renin inhibitors of this invention. Deprotection of certain of these compounds yields still further renin inhibitors of this invention.

The process shown in Scheme 3 discloses the conversion of allylic alcohol (II) to aldehyde (IX) via a three step procedure comprising (a) oxazolidine formation using 2-methoxypropene and pyridinium p-toluenesulfonate, (b) ozonolysis, and (c) equilibration of diastereomers using potassium carbonate in methanol. Aldehyde (IX) is condensed with methyl 2-(bromomethyl)acrylate in the presence of zinc to give the unsaturated lactone (X). Hydrogenation of (X) over palladium on carbon leads to saturated lactone (XI). Reduction of (XI) with sodium borohydride gives diol (XII), which can be cyclized to the substituted tetrahydrofuran (XIII) using a two step procedure including (a) mesylation with methanesulfonyl chloride and triethylamine and (b) ring closure with sodium hydride. Alternatively, (XI) can be alkylated using lithium diisopropylamide and an alkyl halide to provide (XIV). Reduction or (XIV) using sodium borohydride leads to diol (XV). Intermediates (X), (XI), (XII), (XIII), (XIV) and (XV) are deprotected and coupled to peptide segments in the manner disclosed in Scheme 2 to give the novel renin inhibiting compounds of this invention.

Particularly useful intermediates for the preparation of the novel compounds of this invention are compounds of the formula:

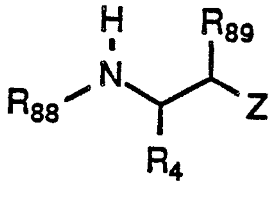

wherein $R_4$ is loweralkyl, cycloalkylmethyl or benzyl;

$R_{88}$ is hydrogen or an N-protecting group;

$R_{89}$ is $-OR_{90}$ or $-NHR_{91}$ wherein $R_{90}$ is hydrogen or an O-protecting group and $R_{91}$ is hydrogen or an N-protecting group; and

Z is

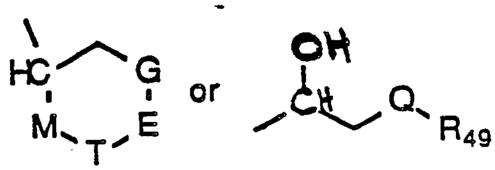

wherein M is O, S or NH, T is C=O, C=S, S, S(O), $S(O)_2$ or $CH_2$, E is O, S, $NR_6$ wherein $R_6$ is hydrogen, loweralkyl, hydroxyalkyl, hydroxy, alkoxy, amino, or alkylamino, or E is $CR_6R_{42}$ wherein $R_6$ is as defined above and $R_{42}$ is hydrogen or loweralkyl or E is $C=CR_{43}R_{44}$ wherein $R_{43}$ and $R_{44}$ are independently selected from hydrogen and loweralkyl, G is absent, $CH_2$, or $NR_{11}$ wherein $R_{11}$ is hydrogen or loweralkyl, with the proviso that when G is $NR_{11}$ then $R_6$ is loweralkyl or hydroxyalkyl, Q is $CR_{45}R_{46}$ wherein $R_{45}$ and $R_{46}$ are independently selected from hydrogen and loweralkyl or Q is $C=CR_{47}R_{48}$ wherein $R_{47}$ and $R_{48}$ are independently selected from hydrogen and loweralkyl, and $R_{49}$ is $-CH_2OH$, carboxy, alkoxycarbonyl or $-CONR_{50}R_{51}$ wherein $R_{50}$ is hydrogen or loweralkyl and $R_{51}$ is hydrogen, loweralkyl, aminoalkyl, alkylaminoalkyl, dialkylaminoalkyl or alkoxyalkyl.

Other intermediates useful for the preparation of the novel compounds of this invention include compounds of the formula:

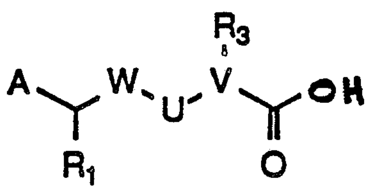

wherein A is hydrogen, loweralkyl, aminoalkyl, (alkyl)aminoalkyl, dialkylaminoalkyl, (alkoxy)aminoalkyl, (alkoxy)(alkyl)aminoalkyl, phenylalkyl, (substituted phenyl)alkyl wherein the phenyl ring is substituted with one, two or three substituents independently selected from loweralkoxy, loweralkyl, amino, alkylamino, dialkylamino, hydroxy, halo, mercapto, nitro, thioalkoxy, carboxaldehyde, carboxy, alkoxycarbonyl and carboxamide, naphthylalkyl, (substituted naphthyl)alkyl wherein the naphthyl ring is substituted with one, two or three substituents independently selected from loweralkoxy, loweralkyl, amino, alkylamino, dialkylamino, hydroxy, halo, mercapto, nitro, thioalkoxy, carboxaldehyde, carboxy, alkoxycarbonyl and carboxamide, substituted or unsubstituted heterocyclic, where saturated heterocyclics may be unsubstituted, monosubsituted or disubstituted with hydroxy, oxo, amino, alkylamino, dialkylamino, alkoxy, polyalkoxy, loweralkyl, haloalkyl or polyhaloalkyl; unsaturated heterocyclics may be unsubstituted or monosubstituted with hydroxy, amino, alkylamino, dialkylamino, alkoxy, polyalkoxy, loweraklyl, haloalkyl or polyhaloalkyl, or A is (unsubstituted heterocyclic)alkyl or (substituted heterocyclic)alkyl wherein unsubstituted or substituted heterocyclic is as defined above, or A is $-OR_7$ or $-SR_7$ wherein $R_7$ is hydrogen, loweralkyl, aminoalkyl, (alkyl)aminoalkyl, dialkylaminoalkyl, (alkoxy)aminoalkyl, (alkoxy)(alkyl)aminoalkyl, phenylalkyl, (substituted phenyl)alkyl wherein substituted phenyl is as defined above, naphthylalkyl, (substituted naphthyl)alkyl wherein the substituted naphthyl is as defined above, substituted or unsubstituted heterocyclic as defined above, (unsubstituted heterocyclic)alkyl or (substituted heterocyclic)alkyl wherein unsubstituted or substituted heterocyclic is as defined above, (unsubstituted heterocyclic)C(O)- or (substituted heterocyclic)C(O)- wherein unsubstituted or substituted heterocyclic is as defined above; or A is $-NR_8R_9$ wherein $R_8$ and $R_9$ are independently selected from hydrogen, hydroxy, alkoxy, loweralkyl, aminoalkyl, cyanoalkyl and hydroxyalkyl; or A is

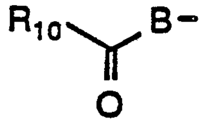

or

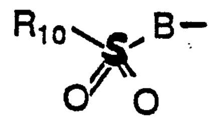

wherein B is NH, alkylamino, S, O, $CH_2$, $NHCH_2$ or $CH(OR_{52})$ wherein $R_{52}$ is hydrogen, loweralkyl or loweralkylcarbonyl, and $R_{10}$ is hydrogen, loweralkyl, cycloalkyl, phenyl, substituted phenyl as defined above, naphthyl, substituted naphthyl as defined above, alkoxy, alkenyloxy, hydroxyalkoxy, dihydroxyalkoxy, phenylalkoxy, (substituted phenyl)alkoxy wherein substituted phenyl is as defined above, naphthylalkoxy, (substituted naphthyl)alkoxy wherein substituted naphthyl is as defined above, phenylalkoxyalkyl, (substituted phenyl)alkoxyalkyl wherein substituted phenyl is as defined above, naphthylalkoxyalkyl, (substituted naphthyl)alkoxyalkyl wherein substituted naphthyl is as defined above, thioalkoxyalkyl, loweralkylsulfinylalkyl, loweralkylsulfonylalkyl, phenylthioalkyl, (substituted phenyl)thioalkyl wherein substituted phenyl is as defined above, naphthylthioalkyl, (substituted naphthyl)thioalkyl wherein substituted naphthyl is as defined above, phenylsulfonylalkyl, (substituted phenyl)sulfonylalkyl wherein substituted phenyl is as defined above, naphthylsulfonylalkyl, (substituted naphthyl)sulfonylalkyl wherein substituted naphthyl is as defined above, amino, alkylamino, dialkylamino, (hydroxyalkyl)(alkyl)amino, (dihydroxyalkyl)(alkyl)amino, aminoalkyl, alkoxycarbonylalkyl, carboxyalkyl, (N-protected)aminoalkyl, alkylaminoalkyl, (N-protected)(alkyl)-aminoalkyl, dialkylaminoalkyl, (heterocyclic)alkyl, a substituted or unsubstituted heterocyclic as defined above, aminocycloalkyl, aminoalkylamino, (dialkylaminoalkyl)(alkyl)amino, phenylalkylamino, (substituted phenyl)alkylamino wherein substituted phenyl is as defined above, naphthylalkylamino, (substituted naphthyl)alkylamino wherein substituted naphthyl is as defined above, (phenylalkyl)(alkyl)amino, ((substituted phenyl)alkyl)(alkyl)amino wherein substituted phenyl is as defined above, (naphthylalkyl)(alkyl)amino, ((substituted naphthyl)alkyl)(alkyl)amino wherein substituted naphthyl is as defined above, alkoxyalkyl(alkyl)-amino, (polyalkoxy)alkyl(alkyl)amino, di-(alkoxyalkyl)amino, di-(hydroxyalkyl)amino, di-((polyalkoxy)alkyl)-amino, ((heterocyclic)alkyl)(alkyl)amino, ((heterocyclic)alkyl)amino, (heterocyclic)(alkyl)amino, (alkylaminoalkyl)(alkyl)amino, (dialkylaminoalkyl)(alkyl)amino, ((alkoxy)(alkyl)aminoalkyl)(alkyl)amino, ((alkoxy)aminoalkyl)(alkyl)amino, polyalkoxy or (polyalkoxy)alkyl; or A is $R_{41}CH(OH)CH_2$- or $R_{41}CH(OH)CH(OH)$- wherein $R_{41}$ is loweralkyl, cycloalkyl, phenyl, substituted phenyl as defined above, naphthyl, substituted naphthyl as defined above, phenylalkyl, (substituted phenyl)alkyl wherein substituted phenyl is as defined above, naphthylalkyl, (substituted naphthyl)alkyl wherein substituted naphthyl is as defined above, phenylalkoxyalkyl, (substituted phenyl)alkoxyalkyl wherein substituted phenyl is as defined above, naphthylalkoxyalkyl, (substituted naphthyl)alkoxyalkyl wherein substituted naphthyl is as defined above, thioalkoxyalkyl, loweralkylsulfinylalkyl, loweralkylsulfonylalkyl, phenylthioalkyl, (substituted phenyl)thioalkyl wherein substituted phenyl is as defined above, naphthylthioalkyl, (substituted naphthyl)thioalkyl wherein substituted naphthyl is as defined above, phenylsulfonylalkyl, (substituted phenyl)sulfonylalkyl wherein substituted phenyl is as defined above, naphthylsulfonylalkyl, (substituted naphthyl)sulfonylalkyl wherein substituted naphthyl is as defined above, aminoalkyl, alkoxycarbonylalkyl, carboxyalkyl, (N-protected)-aminoalkyl, alkylaminoalkyl, (N-protected)(alkyl)aminoalkyl, dialkylaminoalkyl, heterocyclicalkyl, a substituted or unsubstituted heterocyclic as defined above, aminocycloalkyl or (polyalkoxy)alkyl;

W is C=O, CHOH or $NR_2$ wherein $R_2$ is hydrogen or loweralkyl;

U is C=O, $CH_2$ or $NR_2$ wherein $R_2$ is hydrogen or loweralkyl, with the proviso that when W is CHOH then U is $CH_2$ and with the proviso that U is C=O or $CH_2$ when W is $NR_2$;

V is CH, C(OH) or C(halogen) with the proviso that V is CH when U is $NR_2$;

$R_1$ is loweralkyl, cycloalkylalkyl, benzyl, (alpha, alpha)-dimethylbenzyl, 4-methoxybenzyl, halobenzyl, 4-hydroxybenzyl, (1-naphthyl)methyl, (2-naphthyl)methyl, (unsubstituted heterocyclic)methyl, (substituted heterocyclic)methyl wherein unsubstituted or substituted heterocyclic is as defined above, phenethyl, 1-benzyloxyethyl, phenoxy, thiophenoxy or anilino, provided that B is $CH_2$ or CHOH or A is hydrogen when $R_1$ is phenoxy, thiophenoxy or anilino; and

$R_3$ is loweralkyl, loweralkenyl, ((alkoxy)alkyoxy)alkyl, carboxyalkyl, (thioalkoxy)alkyl, azidoalkyl, aminoalkyl, (alkyl)aminoalkyl, dialkylaminoalkyl, (alkoxy)(alkyl)aminoalkyl, (alkoxy)aminoalkyl, benzyl or heterocyclic ring substituted methyl; or an acid halide or activated ester derivative thereof.

Acid halide derivatives of the above intermediates include the acid chloride. Activated ester derivatives of the above intermediates include activated esters commonly used by those skilled in the art for activating carboxylic acid groups for coupling with an amine to form a peptide bond, including, but not limited to formic and acetic acid derived anhydrides, anhydrides derived from alkoxycarbonyl halides such as isobutyloxycarbonylchloride and the like, N-hydroxysuccinimide derived esters, N-hydroxyphthalimide derived esters, N-hydroxybenzotriazole derived esters, N-hydroxy-5-norbornene-2,3-dicarboxamide derived esters, 4-nitrophenol derived esters, 2,4,5-trichlorophenol derived esters and the like.

SCHEME 1

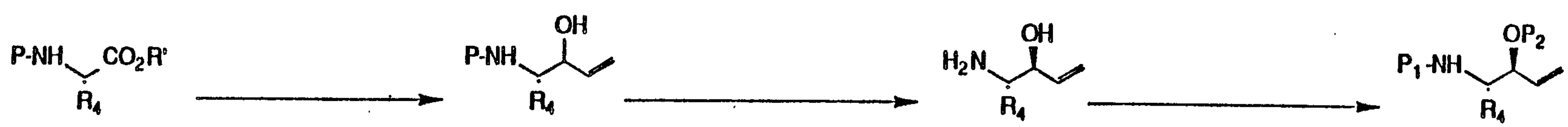

(I) (II) (III) (IV)

P, $P_1$, and $P_2$ represent protecting groups.
R' represents loweralkyl or arylalkyl.

SCHEME 2

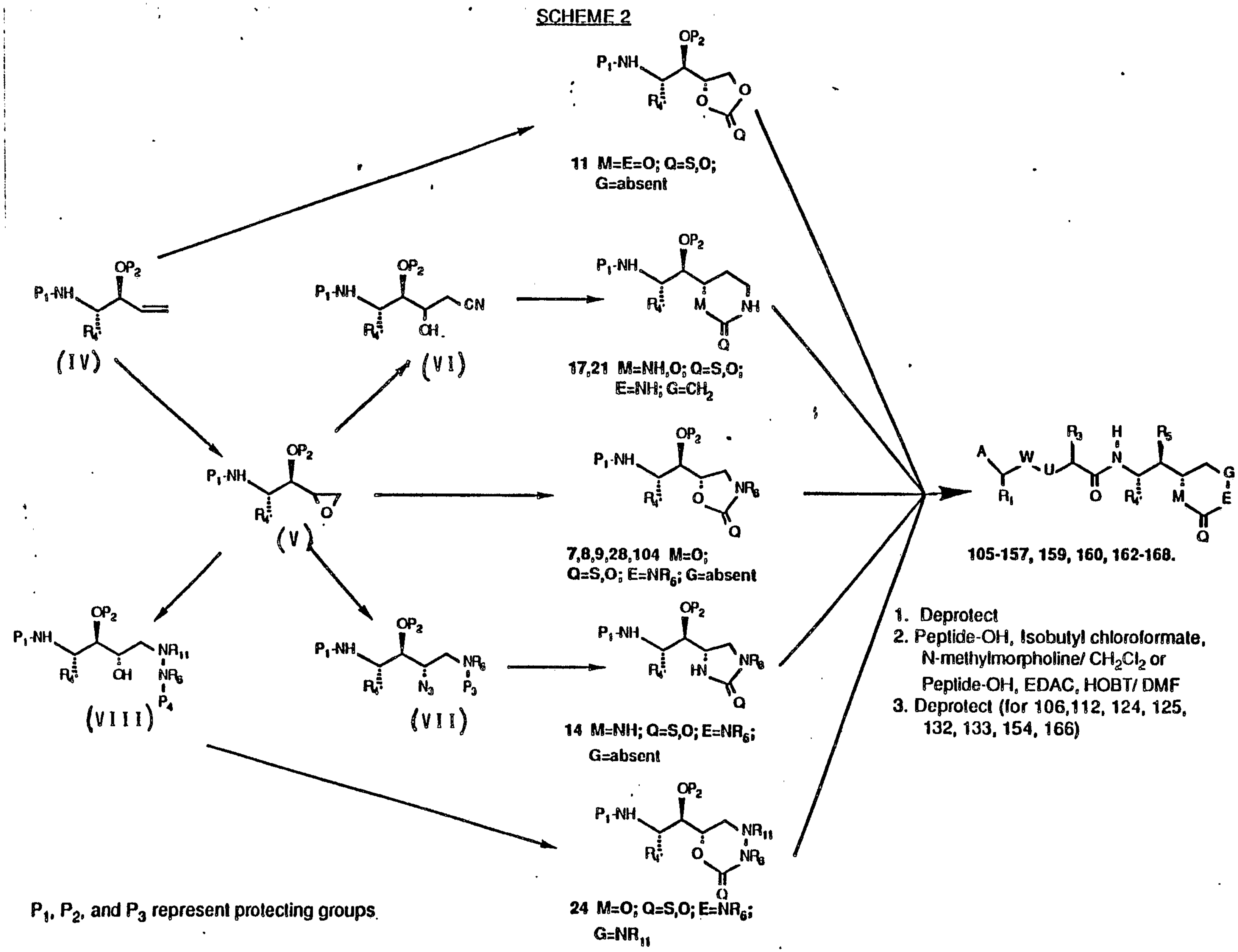


(IV)

(VI)

(V)

(VIII)

(VII)

11 M=E=O; Q=S,O; G=absent

17,21 M=NH,O; Q=S,O; E=NH; G=$CH_2$

7,8,9,28,104 M=O; Q=S,O; E=$NR_6$; G=absent

14 M=NH; Q=S,O; E=$NR_6$; G=absent

24 M=O; Q=S,O; E=$NR_6$; G=$NR_{11}$

105-157, 159, 160, 162-168.

$P_1$, $P_2$, and $P_3$ represent protecting groups.

Scheme 3

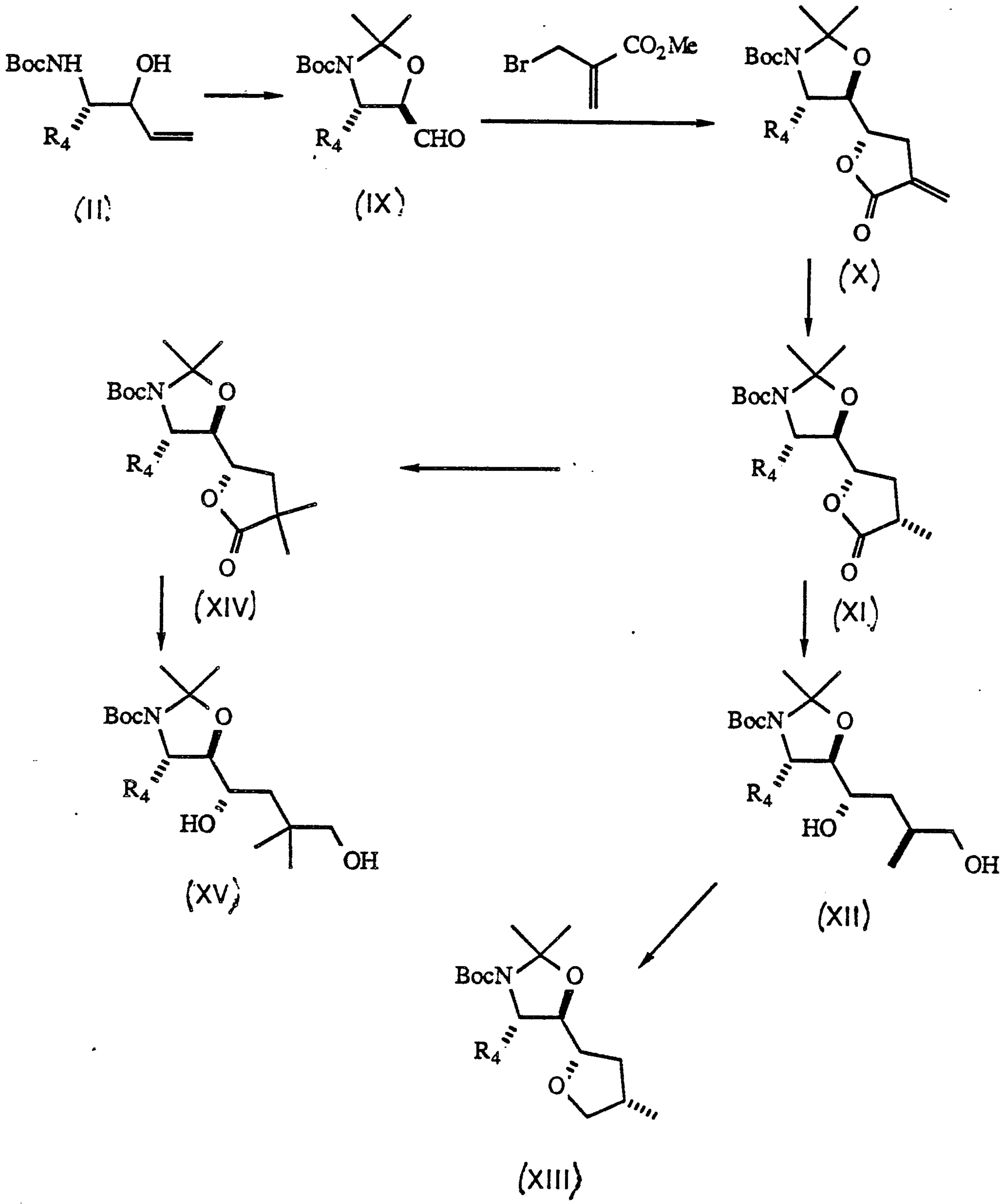

(II) (IX) (X) (XI) (XII) (XIII) (XIV) (XV)

The following examples will serve to further illustrate preparation of the novel compounds of the invention.

## Example 1

### 4(S)-t-Butyloxycarbonylamino-5-cyclohexyl-3(R,S)-hydroxyl-1-pentene.

To a stirred -78° C solution of Boc-cyclohexylalanine methyl ester (10.2 g, 35.8 mmol) in dry toluene (60 ml) was added diisobutylaluminum hydride (34 ml of a 1.5 M solution in toluene). After 30 min, vinyl magnesium bromide (108 ml of 1 M solution in tetrahydrofuran (THF)) was added. After stirring for 15 h at 0° C, the mixture was carefully quenched with methanol, treated with Rochelle salts (22 ml of saturated aqueous solution in 140 ml $H_2O$), and filtered. After extracting the solids 5 times with ethyl acetate, the extracts and filtrate were combined and the organic phase was washed with brine, dried, filtered and evaporated to an oil (10.2 g). Chromatogrphy on silica gel eluting with hexane/ethyl acetate mixtures provided 6.1 g (60%) of the desired product.
Anal. Calcd. for $C_{16}H_{29}NO_3 \bullet \frac{1}{4} H_20$:
C, 66.8; H, 10.3; N, 4.9.
Found:
C, 66.9; H, 10.2; N, 4.7.

## Example 2

### 4(S)-Cyclohexylmethyl-5(R,S)-vinyl-2-oxazolidinone.

The resultant product of Example 1 (2.80 g, 9.88 mmol) in dry dimethylformamide (DMF) (50 ml) was added to a stirred suspension of NaH (593 mg of a 60% dispersion in oil, 14.8 mmol, hexane washed) in dry DMF (50 ml). After 3 h, the mixture was quenched (750 ml water + 100 ml brine) and extracted with ether (5 x 100 ml). The combined organic phase was washed with brine (3 x 50 ml), dried ($MgSO_4$), filtered and evaporated to an oil (2.23 g). The NMR spectrum of the crude product revealed an 82:18 mixture of 5S:5R diastereomers. Silica gel chromatography gave 80% recovery of pure diastereomers. 5S:
Anal. Calcd. for $C_{12}H_{19}NO_2$:
C, 68.9; H, 9.1; N, 6.7.
Found:
C, 68.4; H, 9.2; N, 6.5. Mass spectrum: $(M+1)^+$ = 210. 5R: Mass spectrum: $(M+1)^+$ = 210.

## Example 3

### (3S,4S)-3-Hydroxy-4-amino-5-cyclohexyl-1-pentene.

To the resultant 5S-diasteriomer from Example 2 (2.06 g, 9.84 mmol) in dioxane (180 ml) and water (120 ml) was added barium hydroxide octahydrate (6.24 g, 19.8 mmol). The mixture was refluxed for 18 h cooled, filtered, concentrated, taken up in water and extracted with ethyl acetate which was dried over $Na_2SO_4$ and evaporated to afford 1.64 g (91%) of the desired product, m.p.: 59-61° C.
Anal. Calcd. for $C_{11}H_{21}NO$:
C, 72.08; H, 11.55; N, 7.64.
Found:
C, 71.67; H, 11.68; N, 7.36.

Example 4

(3S,4S)-3-Hydroxy-4-tert-butyloxycarbonylamino-5-cyclohexyl-1-pentene.

To the resultant compound from Example 3 (1.62 g, 8.84 mmol) in methylene chloride (20 ml) was added di-tert-butyldicarbonate (1.93 g, 8.84 mmol). The mixture was stirred for 14 h, diluted with ethyl acetate, washed sequentially with 0.5 M $H_3PO_4$, saturated $NaHCO_3$ solution and brine, then dried over $Na_2SO_4$ and evaporated to afford 2.51 g (100%) of the desired compound.

Example 5

(3S,4S)-3-Methoxyethoxymethoxy-4-tert-butyloxycarbonylamino-5-cyclohexyl-1-pentene.

To the resultant compound from Example 4 (2.51 g, 8.84 mmol) in methylene chloride (20 ml) was added diisopropyl ethylamine (4.60 ml, 26.4 mmol) and methoxyethoxy chloromethane (3.00 ml, 26.3 mmol). After stirring at room temperature for 24 h the mixture was concentrated, diluted with ethyl acetate, washed with 0.5 M $H_3PO_4$, saturated $NaHCO_3$ solution, then brine, dried over $Na_2SO_4$, and evaporated. Chromatography on silica gel with ethyl acetate/hexane mixtures afforded 2.63 g (80%) of the desired product as an oil. EI-MS: $M^+$ = 371.

Example 6

(2RS,3R,4S)-3-Methoxyethoxymethoxy-4-tert-butyloxycarbonylamino-5-cyclohexyl-1,2-oxopentane.

To the resultant compound from Example 5 (5.41 g, 14.56 mmol) in methylene chloride (50 ml) was added 3-chloroperbenzoic acid (6.28 g). After stirring at room temperature for 60 h the mixture was concentrated, diluted with ethyl acetate, washed with cold 1:1 15% aqueous $Na_2SO_3$ solution/saturated $NaHCO_3$ solution (2 x 200 ml), saturated $NaHCO_3$ solution (3 x 100 ml) then brine (1 x 100 ml), dried over $Na_2SO_4$, and evaporated to afford 4.57 g (81%) product as an oil. EI-MS: $M^+$ = 387.

Example 7

(2′S,1′R,5S)-3-Ethyl-5-(1′-methoxyethoxymethoxy-2′-tert-butyloxycarbonylamino-3′-oxazolidin-2-one.

To the resultant compound from Example 6 (310 mg, 0.80 mmol) in isopropanol (5 ml) was added ethyl amine (200 mg, 4 mmol). The mixture was heated at 70° C for 48 h, evaporated and dissolved in methylene chloride (5 ml). To this solution was added triethylamine (0.34 ml, 2.4 mmol) and phosgene in toluene (1.0 ml, 1.2 mmol 12.5% solution). After 2 h the mixture was diluted with ethyl acetate, washed with 0.5 M $H_3PO_4$, saturated $NaHCO_3$ solution then brine, dried over $Na_2SO_4$ and evaporated. Chromatography of the residue on silica gel with 1:1 ethyl acetate/hexane provided 14.3 mg (4%) of the 5R isomer followed by 63.0 mg (17%) of the desired 5S isomer, both as oils.
5S-Isomer: $^1$H-NMR ($CDCl_3$) δ 4.83 (d,1H), 4.80 (d,1H), 4.58 (m,1H), 3.49 (s,3H), 1.43 (s,9H), 1.15 (t,3H).
5R-Isomer: MS $(M+H)^+$ = 459.

Example 8

(2′S,1′R,5S)-3-Methoxy-5-(1′-methoxyethoxymethoxy-2′-tert-butyloxycarbonylamino-3′-cyclohexylpropyl)-oxazolidin-2-one.

Prepared from the resultant compound of Example 6 using the procedure of Example 7 and replacing the ethyl amine with equal parts of methoxylamine hydrochloride and sodium bicarbonate.

Example 9

(2′S,1′R,5S)-3-(Benzyloxycarbonylmethylamino)-5-(1′-methoxyethoxymethoxy)-2′-tert-butyloxycarbonylamino-3′-cyclohexylpropyl)-oxazolidin-2-one.

Prepared from the resultant compound of Example 6 using the procedure of Example 7 and replacing the ethyl amine with one equivalent of 1-methyl-1-benzyloxycarbonylhydrazine.

Example 10

(2RS,3R,4S)-1,2-Dihydroxy-3-methoxyethoxymethoxy-4-tert-butyloxycarbonylamino-5-cyclohexylpentane.

To the resultant compound from Example 5 (1.00 g, 2.69 mmol) in tetrahydrofuran (20 ml) at 0° C was added osmium tetroxide (0.75 ml of a 2.5% solution in tert-butanol) and N-methylmorpholine N-oxide (347 mg, 2.95 mmol). The mixture was stirred at room temperature 16 h, diluted with ethyl acetate, washed with $NaHSO_3$ solution, saturated $NaHCO_3$ solution and brine, then dried over $Na_2SO_4$ and evaporated. Chromatography of the residue on silica gel with methanol/methylene chloride mixtures provided 887 mg (81%) of the desired product.
Anal. Calcd. for $C_{20}H_{39}NO_7 \bullet 0.3\ H_2O$:
C, 58.46; H, 9.71; N, 3.41.
Found:
C, 58.69; H, 9.53; N, 3.41.

Example 11

(2′S,1′R,5S)-2-Oxo-4-(1′-methoxyethoxymethoxy-2′-tert-butyloxycarbonylamino-3′-cyclohexylpropyl)-dioxolane.

The resultant compound of Example 10 was treated with phosgene according to the procedure of Example 17 to provide the desired product as an oil.

Example 12

(2R,3R,4S)-1-Benzyloxycarbonylethylamino-2-hydroxy-3-methoxyethoxymethoxy-4-tert-butyloxycarbonylamino-5-cyclohexylpentane.

Using the procedure of Example 7 with the resultant compound from Example 6 and replacing the phosgene with benzyl chloroformate provided the desired 2R isomer preceeded by the 2S isomer.
2R-Isomer: $^1$H-NMR ($CDC1_3$, TMS) $\delta$ 7.34 (m,5H), 5.13 (s,2H), 4.95 (d,1H), 4.79 (m,2H), 3.37 (s,3H), 1.43 (s,9H), 1.14 (m,3H).
2S-Isomer: $^1$H-NMR ($CDC1_3$, TMS) $\delta$ 7.35 (m,5H), 5.14 (d,1H), 5.12 (d,1H), 4.93 (d,1H), 4.80 (m,2H), 3.38 (s,3H), 1.43 (s,9H), 1.13 (t,3H).

## Example 13

(2S,3R,4S)-1-Benzyloxycarbonylethylamino-2-azido-3-methoxyethoxymethoxy-4-tert-butyloxycarbonylamino-5-cyclohexylpentane.

To triphenylphosphine (100.0 mg, 0.381 mmol) in tetrahydrofuran (THF, 0.6 ml) at -78°C was added diethyl azodicarboxylate (60 µl, 0.38 mmol) in THF (1 ml). To this mixture was added a solution of hydrazoic acid (0.46 mmol) in benzene (1 ml) then the resultant compound from Example 12 (180.0 mg, 0.318 mmol) in THF (1.4 ml) was added. After one hour the mixture was warmed to room temperature, stirred for 16 h, evaporated and chromatographed on silica gel with 20% ethyl acetate in hexane to afford 103.5 mg (55%) of the desired product as an oil. $^1$H-NMR ($CDC1_3$, TMS) $\delta$ 7.35 (m,5H), 5.15 (m,2H), 3,38 (s,3H), 1.45 (s,9H), 1.15 (m,3H).

## Example 14

(2′S,1′R,5S)-3-Ethyl-5-(1′-methoxyethoxymethoxy-2′-tert-butyloxycarbonylamino-3′-cyclohexylpropyl)-imidazolidin-2-one.

To the resultant compound from Example 13 (99.0 mg, 0.167 mmol) in methanol (2 ml) was added triethylamine (75 µl, 0.54 mmol) and propane 1,3-dithiol (50 µl, 0.50 mmol). After 72 h the mixture was filtered and evaporated, and the crude amino compound was dissolved in toluene (5 ml) and heated to reflux for 72 h. Evaporation and chromatography on silica gel with ethyl acetate/hexane mixtures provided the desired product as an oil.

## Example 15

(3S,4R,5S)-3-Hydroxy-4-methoxyethoxymethoxy-5-tert-butyloxycarbonylamino-6-cyclohexylhexane nitrile.

To the resultant compound from Example 6 (627.0 mg, 1.62 mmol) in methanol (9 ml) was added potassium cyanide (320 mg, 4.91 mmol) in water (3 ml). The mixture was stirred for 32 h, concentrated, diluted with ethyl acetate, washed with water and brine, then dried over $Na_2SO_4$ and evaporated. Chromatography on silica gel with 2:1 hexane/ethyl acetate afforded 209 mg (31%) of the desired 3S-isomer as a solid followed by 150 mg (22%) of the 3R-isomer.
3S-Isomer:
Anal. Calcd. for $C_{21}H_{38}N_2O_6$:

C, 60.85; H, 9.24; N, 6.76.
Found:
C, 60.76; H, 9.22; N, 6.72.
3R-Isomer:
$^1H$-NMR ($CDCl_3$, TMS) δ 4.92 (d,1H), 4.75 (d,1H), 4.69 (d,1H), 3.40 (s,3H), 2.80 (d,2H), 1.45 (s,9H).

Example 16

(3S,4R,5S)-1-Amino-3-hydroxy-4-methoxyethoxymethoxy-5-tert-butyloxycarbonylamino-6-cyclohexylhexane.

The resultant compound from Example 15 in 5:1 methanol/ammonia was treated with Raney-Nickel and stirred under 4 atmospheres of hydrogen for 18 h. The mixture was evaporated, dissolved in ethyl acetate, filtered, and evaporated to provide the desired product as an oil. $^1H$-NMR ($CDCl_3$, TMS) δ 4.97 (d,1H), 4.80 (d,1H), 4.76 (d,1H), 4.03 (m,1H), 3.90 (s,3H), 1.43 (s,9H).

Example 17

(6S,1′R,2′S)-3-Aza-2-oxo-6-(1′-methoxyethoxymethoxy-2′-tert-butyloxycarbonylamino-3′-chexylpropyl)-tetrahydropyran.

The resultant compound from Example 16 (59.0 mg, 0.141 mmol) in methylene chloride (2 ml) at 0 °C was treated with triethylamine (100 µl, 0.71 mmol) then phosgene in toluene (0.22 mmol in 180 µl). The mixture was stirred at 0 °C for 1 h, then at room temperature for 3 h, poured into ethyl acetate, washed with 0.5 M $H_3PO_4$, saturated $NaHCO_3$ solution and brine, then dried over $Na_2SO_4$ and evaporated to afford 53.6 mg (86%) of the desired product as an oil. $^1H$-NMR ($CDCl_3$, TMS) δ 5.09 (m,1H), 4.90 (d,1H), 4.85 (s,2H), 4.35 (m,1H), 3.40 (s,3H), 1.47 (s,9H).

Example 18

(3R,4R,5S)-1-Amino-3-hydroxy-4-methoxyethoxymethoxy-5-tert -butyloxycarbonylamino-6-cyclohexylhexane.

The resultant 3R-isomer from Example 15 was treated according to the procedure for Example 16 to provide the desired product.

Example 19

(3R,4R,5S)-1-Benzlyloxycarbonylamino-3-hydroxy-4-methoxyethoxymethoxy-5-tert-butyloxycarbonylamino-6-cyclohexylhexane.

The resultant compound from Example 18 was treated according to the procedure for Example 17 except that the phosgene in toluene was replaced with benzyl chloroformate to provide the desired product.

Example 20

(3S,4R,5S)-1-Benzyloxycarbonylamino-3-azido-4-methoxyethoxymethoxy-5-tert -butyloxycarbonylamino-6-cyclohoxylpentane.

The resultant compound from Example 19 was treated according to the procedure of Example 13 to provide the desired product.

Example 21

(4S,1′R,2′S)-3-Aza-4-(1′-methoxyethoxymethoxy-2′-tert-butyloxycarbonylamino-3′-cyclohexylpropyl)-piperidin-2-one.

The resultant compound from Example 20 was treated according to the procedure of Example 14 to provide the desired product.

Example 22

(2S,3R,4S)-1-(Benzyloxycarbonylamino)methylamino -2-hydroxy-3-methoxyethoxymethoxy-4-tert-butyloxycarbonylamino-5-cyclohexylpentane.

Prepared from the resultant compound of Example 6 using the procedure of Example 7 with the exceptions that the ethyl amine was replaced with one equivalent of 1-methyl-2- benzyloxycarbonylhydrazine and that the phosgene step was omitted. The desired product was isolated by chromatography on silica gel.

Example 23

(2S,3R,4S)-1-(Amino)methylamino-2-hydroxy-3-methoxyethoxymethoxy-4-tert-butyloxycarbonylamino-5-cyclohoxylpentane.

The resultant compound from Example 22 and an equal weight of 10% palladium on carbon in methanol were stirred under a hydrogen atmosphere for 6 h. The mixture was filtered and evaporated to provide the desired product.

Example 24

(6S,1′R,2′S)-3,4-Diaza-2-oxo-4-methyl-6-(1′-methoxyethoxymethoxy-2′-tert-butyloxycarbonylamino-3′-cyclohexylpropyl)tetrahydropyran.

The resultant compound from Example 23 was treated according to the procedure of Example 17 to provide the desired product.

Example 25

Benzyloxycarbonyl-2-aminoethanol.

To ethanolamine (9.0 ml, 149 mmol) in methylene chloride (100 ml) at 0°C was added benzyl chloroformate (10.0 ml, 70 mmol). The mixture was stirred at 0°C for 30 min, then at room temperature for 1 h, poured into ethyl acetate, washed with 2 M HCl, saturated $NaHCO_3$ solution, and brine, then dried over $Na_2SO_4$ and evaporated to provide 12.91 g (94%) of the desired compound as a white solid. $^1$H-NMR ($CDC1_3$, TMS) $\delta$ 7.47 (m,5H), 5.11 (s,2H), 3.73 (m,2H), 3.38 (m,2H).

Example 26

1-Methoxymethoxy-2-benzyloxycarbonylaminoethane.

To the resultant compound from Example 25 (12.91 g, 66.1 mmol) in methylene chloride (100ml) was added diisopropylethylamine (24.0 ml, 138 mmol) and chloromethylmethylether (10.0 ml, 132 mmol). After 4 h the mixture was evaporated, dissolved in ethyl acetate, washed with 0.5 M $H_3PO_4$, saturated $NaHCO_3$ solution, and brine, then dried over $Na_2SO_4$, and evaporated to afford 15.27 g (97%) of the desired product as an oil. $^1$H-NMR ($CDC1_3$, TMS) $\delta$ 7.47 (m,5H), 5.12 (s,2H), 4.62 (s,2H), 3.62 (m,2H), 3.42 (m.2H), 3.35 (s,3H).

Example 27

1-Methoxymethoxy-2-aminoethane.

The resultant compound from Example 26 (7.60 g, 31.2 mmol) and 10% palladium on carbon (3 g) in methanol (60 ml) were stirred under a hydrogen atmosphere for 24 h. The mixture was filtered, evaporated and distilled to afford 2.02 g (60%) of the desired product as an oil. b.p. 60-70°C, 45 mm; $^1$H-NMR ($CDCl_3$, TMS) $\delta$ 4.65 (s,2H), 3.56 (t,3H), 3.37 (s,3H), 2.88 (t,3H).

Example 28

(2′S,1′R,5S)-3-(2-methoxymethoxyethyl)-5′-(1′-methoxyethoxymethoxy-2′-tert-butyloxycarbonylamino-3′-cyclohexylpropyl)-oxazolidin-2-one.

The resultant compound from Example 6 was treated accordingly to the procedure for Example 7 except that the ethyl amine was replaced with three equivalents of the resultant compound from Example 27 to provide the desired product as an oil.

## Example 29

(2′S,1′R,5S)-3-Ethyl-5-(1′-hydroxy-2′-amino-3′-cyclohexylpropyl)-oxazolidin-2-one.

The resultant compound from Example 7 was stirred for 1 h in 4.5 M ethanolic HCl. The mixture was evaporated, dissolved in saturated $NaHCO_3$ solution and extracted into chloroform which was dried over $Na_2SO_4$ and evaporated to provide the desired product as a solid.
Anal. Calcd. for $C_{14}H_{25}N_2O_3$:
C, 62.19; H, 9.69; N, 10.36.
Found:
C, 62.35; H, 9.74; N, 10.28.

## Example 30

Boc-His Amide of (2′S,1′R,5S)-3-Ethyl-5-(1′-hydroxy-2′-amino-3′-cyclohexylpropyl)-oxazolidin-2-one.

The resultant compound from Example 7 (2.23 g, 4.87 mmol) was stirred for 1 h in 4.5 M ethanolic HCl (15 ml). The solvent was evaporated with ether and toluene chasers and the residue was dissolved in dimethylformamide (25 ml) and treated with Boc-His-OH (1.37 g, 5.36 mmol) and 1-hydroxybenzotriazole (1.98 g, 14.6 mmol). The mixture was cooled to -23 °C and N-methylmorpholine (600µl, 5.46 mmol) was added followed by 1-(3-dimethylaminopropyl)-3-ethylcarbodiimide (1.05 g, 5.46 mmol). The mixture was stirred at -23 °C for 2 h and at room temperature 16 h, and then was poured into saturated $NaHCO_3$ solution and extracted into ethyl acetate which was washed with water and brine, then dried over $Na_2SO_4$ and evaporated. Chromatography of the residue on silica gel with 4% methanol in chloroform provided 1.785 g (72%) of the desired product as a white solid. $^1$H-NMR ($CDC1_3$, TMS) δ 7.58 (s,1H), 6.89 (s,1H), 6.26 (m,1H), 4.39 (m,1H), 4.13 (m,1H), 4.40 (m,1H), 3.30 (m,2H), 3.16 (dd,1H), 3.01 (dd.1H), 1.47 (s,9H), 1.17 (t,3H).

## Example 31

Boc-Leu Amide of (2′S,1′R,5S)-3-Ethyl-5-(1′-hydroxy-2′-amino-3′-cyclohexylpropyl)-oxazolidin-2-one.

The resultant compound from Example 7 (131 mg, 0.285 mmol) was stirred for 1 h in 4.5 M ethanolic HCl (2 ml). The solvent was evaporated with ether and toluene chasers and the residue was taken up in methylene chloride (4 ml) and treated with N-methylmorpholine (65 µl, 0.59 mmol). To Boc-Leu-OH (81.6 mg, 0.327 mmol) in methylene chloride (2 ml) at -10 °C was added N-methylmorpholine (40 µl, 0.34 mmol) then isobutyl chloroformate (45 µl, 0.34 mmol). After 3 min the amine solution was added, the reaction was stirred at -10 °C for 15 min and then at room temperature for 2 h. The mixture was poured into ethyl acetate

which was washed with 0.5 M $H_3PO_4$, saturated $NaHCO_3$ solution, and brine, then dried over $Na_2SO_4$ and evaporated to provide 140 mg (100%) of the desired product as a solid. $^1H$-NMR ($CDCl_3$, TMS) $\delta$ 6.72 (d,1H), 4.75 (d,1H), 4.30 (m,1H), 4.08 (m,1H), 3.80 (m,1H), 3.61 (m,3H), 3.31 (m,2H), 1.45 (s,9H), 1.17 (t,3H), 0.97 (t,6H).

## Example 32

### Boc-(Me)His Amide of (2′S,1′R,5S)-3-Ethyl-5-(1′-hydroxy-2′-amino-3′-cyclohexylpropyl)-oxazolidin-2-one.

To a stirred solution of $N^{\alpha}$-(t-butyloxycarbonyl)-$N^{\alpha}$-methyl-$N^{im}$-tosyl-L-histidine [J. Med. Chem. 29, 2088 (1986), 9.15 mmol] and the product from Example 29 (6.1 mmol) in dichloromethane (75 ml) was added 1.28 ml (9.18 mmol) of triethylamine, followed by the slow addition of diethoxyphosphoryl cyanide (1.36 ml, 8.87 mmol). After being stirred at room temperature for 16 h, the reaction mixture was diluted with dichloromethane and then washed with saturated aqueous $NaHCO_3$. The organic phase was dried ($MgSO_4$) and then concentrated. The residue was chromatographed on silica gel eluting with ethyl acetate/hexane mixtures to give a 75% yield of the coupled product.

The above product was stirred in $CH_3OH$ with 5 equivalents of HOBT for 16 h. The reaction mixture was filtered. The filtrate was evaporated to a solid which was taken up in $CHCl_3$, washed with dil $NaHCO_3$, brine, dried and filtered. The resultant residue after evaporation was chromatographed eluting with 5% $CH_3OH/CHCl_3$. The desired product was obtained in 60% yield.

## Example 33

### 3-Benzyloxycarbonylamino-3-methylbutanoic Acid.

A solution of 2,2-dimethyl-3-carbomethyoxypropionic acid [LeMaul, Bull. Soc. Chim. Fr., 828 (1965), 20 g, 0.125 mol], diphenylphosphorylazide (34.3 g, 0.125 mol) and triethylamine was heated in toluene (150 ml) at 100 °C for 2 h. After cooling to 5 °C, the toluene solution was washed successively with 0.5 M HCl, aqueous $NaHCO_3$ and brine. Evaporation of the dried solution gave a residue which was chroma tographed on silica gel eluting with 60/40 hexane-ether. There was obtained 13 g of methyl 3-isocyanato-3-methylbutanoate as a mobile liquid. A solution of this material in toluene (20 ml) was treated with benzyl alcohol (13 ml) and the resulting mixture heated at reflux for 40 h. Evaporation of the toluene left a residue which was dissolved in methanol (125 ml) and then treated with a solution of NaOH (6.6 g, 0.165 mmol) in 22 ml of water. After 5 h, the reaction mixture was partially evaporated, washed with ether and acidified with 6N HCl. Extraction with methylene chloride and evaporation gave 21 g of the desired product. NMR (300 MHz, $CDCl_3$): 1.42 (s,6H), 2.78 (s,2H), 5.08 (s,2H).

## Example 34

### Cbz-[($\beta$,$\beta$-di-Me)-$\beta$-Ala]-Phe-$OCH_3$.

A 4.0 g sample of 3-benzyloxycarbonylamino-3-methylbutanoic acid was coupled to phenylalanine methyl ester hydrochloride (3.43 g) using the mixed anhydride procedure described in Example 31. Purification of the crude product by flash chromatography eluting with 65/35 ether/hexane gave an 86% yield of product. NMR (300 MHz, $CDCl_3$): 1.32 (s,3H), 1.34 (s,3H), 2.46 (d,1H), 2.63 (d,1H), 2.98 (dd,1H), 3.09 (dd,1H), 3.70 (s,3H), 4.86 (dd,1H), 4.97 (d,1H), 5.2 (d,1H), 5.3 (s,1H), 6.13 (d,1H).

Example 35

Cbz[β,β-di-Me)-β-Ala]-Phe-OH.

To a 0 °C solution of Cbz-[(β,β-di-Me)-β-Ala]-Phe-OMe (1.5 g, 3.63 mmol) in dioxane (15 ml) was added a solution of lithium hydroxide (0.174 g, 4.15 mmol) in water (7.5 ml). After stirring for 1 h at 0-5 °C, the reaction mixture was diluted with cold water and extracted two times with ether. The aqueous portion was acidified with 6N HCl and extracted with ether. The organic extract was washed with brine and evaporated to give an 87% yield of product as a viscous liquid.

Example 36

Cbz-[(β,β-di-Me)-β-Ala]-(Me)Tyr-$OCH_3$.

Using the procedure of Example 34 and replacing phenylalanine methyl ester hydrochloride with 0-methyltyrosine methyl ester hydrochloride provided the desired material.

Example 37

Cbz-[(β,β-di-Me)-β-Ala]-(Me)Tyr-OH.

Using the procedure of Example 35 with the resultant compound from Example 36 gave the desired material.

Example 38

2(S)-[[(4-Morpholinyl)carbonyl]oxy]-3-phenylpropionic Acid Methyl Ester.

To L-phenyllactic acid methyl ester (3.2 g) was added 150 ml of 12.5% phosgene in toluene and 25 drops of dimethylformamide. After stirring for 16 h at room temperature, the solvent was evaporated and the residue chased several times with benzene. The resulting product was dissolved in methylene chloride (50 ml), cooled to 0 °C and treated by dropwise addition with 3.86 g (0.044 mmol) of morpholine. The reaction mixture was stirred for 2 h at 0-5 °C and then distributed between 0.5 N HCl and methylene chloride. The organic phase was washed with aqueous $NaHCO_3$ and brine and evaporated to a residue. Flash chromatography on silica gel eluting with 2:1 ether/hexane gave a 65% yield of product. NMR (300 MHz): 3.08 (dd,1H), 3.20 (dd,1H), 3.8 (s,3H), 5.19 (dd.1H).

Example 39

2(S)-[(4-Morpholinyl)carbonyl]oxy-3-phenylpropionic Acid.

Using the hydrolysis procedure of Example 35, the title compound was obtained in 90% yield.

Example 40

(4R)-3-(3-Phenylpropionyl)-4-(2-propyl)-oxazolidine-2-one.

To a stirred solution of 4-(2-propyl)-oxazolidine-2-one in anhydrous tetrahydrofuran (250 ml) under a nitrogen atmosphere at -78°C were added in a dropwise fashion a solution of n-butyllithium in hexane (50 ml, 77.4 mmol) over 5 to 10 min. After stirring an additional 20 min at -78°C 3-phenylpropionyl chloride (12.7 ml, 85.2 mmol) was added neat. The reaction was warmed to room temperature and stirred 1 to 2 h. The reaction was quenched by adding 100 ml of saturated aqueous ammonium chloride and the volatiles removed by rotary evaporation. The resulting aqueous residue was extracted three times with ether and the combined organic phases were washed with brine, dried ($Na_2SO_4$), filtered and concentrated in vacuo. Recrystallization from hexanes/ethyl acetate provided the title compound (16.6 g, 82%). m.p. = 86.5 to 87.5°C. Mass spectrum: $(M+NH_4)^+$ = 279, $(M+H)^+$ = 262.

Example 41

(4R)-3-[(2R)-3-t-butyloxycarbonyl-2-benzylpropionyl]-4-(2-propyl)-oxazolidine-2-one.

To a stirred solution of the product resulting from Example 40 (2.28 g, 8.72 mmol), in anhydrous tetrahydrofuran (30 ml) under a nitrogen atmosphere at -78°C was added a solution of sodium hexamethyldisilylamide (9.6 ml, 9.59 mmol) in tetrahydrofuran. After stirring for 30 min at -78°C, t-butyl bromoacetate (2.21 g, 11.34 mmol) was added in anhydrous tetrahydrofuran and the resulting solution stirred 1 h at -78°C. The reaction was quenched by adding 20 ml of saturated aqueous ammonium chloride and partitioned between water and ether. The aqueous layer was drawn off and extracted with ether. The combined organic phases were washed with 10% aqueous HCl, saturated aqueous $NaHCO_3$, and brine, dried ($Na_2SO_4$), filtered, and concentrated in vacuo. Recrystallization from acetone/hexanes provided the desired purified product (2.59 g, 79%). m.p. = 167-168°C. Mass spectrum: $(M+NH_4)^+$ = 393, $(M+H)^+$ = 376.

Example 42

Benzyl-(2R)-3-t-butyloxycarbonyl-2-benzylpropionate.

To a stirred solution of dry benzyl alcohol (0.55 ml, 5.33 mmol) in anhydrous tetrahydrofuran (18 ml) under a nitrogen atmosphere at 0°C was added a hexane solution of N-butyllithium (2.58 ml; 4.00 mmol). To this solution was added the product from Example 41 in anhydrous tetrahydrofuran (10 ml). After stirring 1 h at 0°C the reaction was quenched by adding excess saturated aqueous ammonium chloride. The volatiles were removed by rotary evaporation and the resulting aqueous residue extracted two times with ether. The combined organic layers were washed with brine, dried ($Na_2SO_4$), filtered, and concentrated in vacuo provided an oil which was purified by chromatography on $SiO_2$ (15% ethyl acetate/hexanes) to provide the desired product (0.89 g 94%) as a colorless oil. Mass spectrum: $(M)^+$ = 354.

Example 43

Benzyl-(2R)-3-carboxy-2-benzylpropionate.

The product from Example 42 (0.52 g, 1.47 mmol) was dissolved in a 1:1 (v:v) solution (6 ml) of trifluoroacetic acid and dichloromethane and stirred at room temperature for 1 h. The volatiles were removed in vacuo to provide the title compound (0.437 g, 100%) as an oil which crystallized on standing. The unpurified material was of sufficient purity to employ in subsequent steps. Mass spectrum: $(M)^+$ = 298.

Example 44

Benzyl-(2R)-2-benzyl-3-(N-morpholinocarbamoyl)-propionate.

The product from Example 43 (0.438 g, 1.47 mmol), diphenylphosphoryl azide (317 µl, 1.47 mmol), and triethylamine (205 µl, 1.47 mmol) in dry benzene (6 ml) were refluxed for 3 to 5 h to provide a solution of the derived isocyanate which was cooled to 0° C and treated with morpholine (141 µl, 1.62 ml). The cooling bath was removed and the reaction stirred for 1 h. The reaction mixture was poured into 10% aqueous HCl and extracted two times with ether. The combined organic layers were washed successively with saturated aqueous $NaHCO_3$ and brine, dried ($Na_2SO_4$), filtered and concentrated in vacuo to provide the unpurified product. The desired product (0.403 g, 72%) was obtained in pure form after chromatography on $SiO_2$ (3% methanol/chloro form) as a thick oil which formed an amorphous solid on standing. Mass spectrum: $(M)^+$ = 382. NMR (300 MHz, $CDCl_3$, ppm, TMS as internal standard) 7.12-7.40 (m,1OH), 5.18 (AB; J=12.6 Hz; 2H), 4.8 (dd; J=5.7 Hz; 1H), 3.59 (d,d; J=6.0, 6.0 Hz; 4H), 3.55 (d,d,d; J=3.0, 6.0 14.4 Hz; 1H), 3.37 (d,d,d; J=5.4, 8.4 14.4 Hz; 1H), 3.13 (d,d; J=6.0, 6.0 Hz; 4H), 2.8-3.10 (m,3H).

Example 45

Benzyl-(2R)-2-benzyl-3-(ethoxycarbamoyl)-propionate.

The procedure as described in Example 44 was followed except absolute ethanol was employed in lieu of morpholine. Mass spectrum: $(M)^+$ = 341. NMR (300 MHZ, $CDCl_3$, ppm, TMS as internal standard) 7.1-7.4 (m,1OH, 5.17 (s,2H), 4.96 (br s,1H), 4.07 (d,d,d; J=6.6, 6.6, 6.6 Hz,2H), 3.25-3.5 (2 br ABX,2H), 2.9-3.05 (m,2H), 2.75-2.88 (br m,1H), 1.23 (d,d; J=6.6, 6.6 Hz; 3H).

Example 46

(2R)-2-Benzyl-3-(morpholinocarbamoyl)-propionic Acid.

The product from Example 44 (0.315 g, 0.86 mmol) was dissolved in ethyl acetate (5 ml) and syringed into a flask charged with 10% Pd/C (~0.3 g). The resulting suspension was exposed to 1 atm of gaseous hydrogen for 2 to 4 h. The catalyst was removed by filtration through a celite pad. The filtrate was concentrated in vacuo to provide the desired compound (0.21 g, 88%) as a cream colored foam which was employed without further purification. Mass spectrum: $(M+H)^+$ = 278.

Example 47

(2R)-2-Benzyl-3-ethoxycarbamoylpropionic Acid.

The procedure as described in Example 46 was followed employing the product from Example 45 in lieu of that from Example 44. Mass spectrum: $(M+H)^+$ = 252.

Example 48

Benzyl (2R)-2-Benzyl-3-morpholinocarbonylpropionate.

The product of Example 43 and morpholine were converted to the title compound using the mixed anhydride method of coupling as described in Example 31. Mass spectrum: $(M)^+$ = 367.

Example 49

(2R)-2-Benzyl-3-morpholinocarbonylpropionic Acid.

The product from Example 48 was converted to the title compound following the procedure described in Example 46. Mass spectrum: $(M)^+$ = 277.

Example 50

(2R)-3-t-Butyloxycarbonyl-2-benzylpropionic Acid.

The resultant compound from Example 42 and an equal weight of 10% palladium on carbon in methanol were stirred under a hydrogen atmosphere for 1 h. The mixture was filtered and evaporated to provide the desired product. $^1$H-NMR ($CDCl_3$, TMS) δ 7.25 (m,5H), 3.07 (m,2H), 2.73 (dd,1H), 2.56 (dd,1H), 2.33 (dd,1H), 1.40 (s,9H).

Example 51

Boc-Phe-dl-3-pyrazolylalanine Methyl Ester.

To dl-3-pyrazolylalaine methyl ester dihydrochloride (dl connotes 50/50 mixture of dextrorotatory/levorotatory) (2.05 g, 8.5 mmol) in dimethylformamide (10 ml) at -10 °C was added Boc-Phe N-hydroxysuccinimide ester (2.50 g, 6.90 mmol) and N-methylmorpholine (2.8 ml, 25 mmol). The mixture was stirred at -10 °C for 1 h and then at 25 °C for 12 h. The mixture was partitioned between ethyl acetate and saturated $NaHCO_3$ solution, and extracted with ethyl acetate which was washed with water, dried over

$Na_2SO_4$ and evaporated to afford 2.75 g (95%) of the desired product.
Anal. Calcd. for $C_{21}H_{28}N_4O_5 \bullet 0.25\ H_2O$:
C, 59.92; H, 6.82; N, 13.31.
Found:
C, 59.82; H, 6.75; N, 13.13.

## Example 52

### Boc-Phe-dl-3-pyrazolylalanine.

Boc-Phe-dl-3-pyrazolylalanine methyl ester (0.210 g, 0.505 mmol) in dioxane (1.5 ml) and water (1.0 ml) was treated with lithium hydroxide monohydrate (0.0272 g, 0.648 mmol), stirred at 25° C for 30 min and quenched with 0.32 ml 2 M HCl. The mixture was poured into chloroform, washed with water, dried over $Na_2SO_4$ and evaporated to afford 0.184 g (91%) of the desired compound.
Anal. Calcd. for $C_{20}H_{26}N_4O_5 \bullet 0.25\ H_2O$:
C, 59.03; H, 6.56; N, 13.77.
Found:
C, 58.66; H, 6.70; N, 13.65.

## Example 53

### α-Isocyanato-L-(0-methyl)tyrosine.

A suspension of (0-methyl)tyrosine methyl ester hydrochloride (6 g) in toluene (125 ml) was heated at 100° C while phosgene was bubbled into the reaction mixture. After 2 h the mixture became homogeneous and the phosgene was continued for an additional 15 min. The mixture was cooled and evaporated with several benzene chasers to provide the desired product.

## Example 54

### 1-Benzyloxycarbonylamino-2,3-propanediol.

1-Amino-2,3-propanediol (15.2 g, 167 mmol) and NaOH (8.1 g, 204 mmol) in water (70 ml) at -10° C was treated dropwise with benzyl chloroformate (28.5 ml, 200 mmol) in ether (30 ml) over 20 min. The reaction was stirred at 0° C for 30 min then at room temperature for 2 h. The mixture was acidified with 2 M HCl and extracted with ethyl acetate which was washed with 0.5 M $H_3PO_4$ and brine, then dried over $Na_2SO_4$ and evaporated. Recrystallization of the residue from benzene afforded 16.59 g (44%) of the desired product as a white powder. NMR (300 MHz, $CD_3OD$, ppm): 3.12 (dd,1H), 3.28 (dd,1H), 3.50 (m,2H), 3.68 (m,1H), 5.08 (s,2H), 7.35 (m,5H).

## Example 55

1-Methylamino-2,3-propanediol.

Lithium aluminum hydride (7.20 g, 189 mmol) in tetrahdyrofuran (THF, 300 ml) was heated to reflux and the resultant compound from Example 54 (17.0 g, 75.5 mmol) in THF (150 ml) was added dropwise over 10 min. The mixture was refluxed for 2 h, cooled, quenched sequentially with water (10 ml), 3 M NaOH (40 ml) and water (20 ml), then filtered and concentrated. The residue was dissolved in water which was washed with ether and evaporated. Bulb to bulb distillation of the residue afforded 2.0 g (25%) of the desired compound as an oil. NMR (300 MHz, $CDCl_3$, ppm): 2.45 (s,3H), 2.68 (dd,1H), 2.77 (dd,1H), 3.61 (dd,1H), 3.72 (dd,1H), 3.78 (m,1H).

Example 56

(N-Methyl-2,3-dihydroxypropylamino)carbonyl -(0-methyl)tyrosine Methyl Ester.

To the resultant compound from Example 53 (1.53 g, 6.5 mmol) in dioxane (5 ml) at 0° C was added the resultant compound from Example 55 (0.684 g, 6.5 mmol). The reaction was stirred at 0° C for 1 h then at room temperature for 1 h, evaporated and chromatographed on silica gel with 5% methanol in chloroform to afford 1.855 g (84%) of the desired product as an oil. NMR (300 MHz, $CDCl_3$, ppm), 2.88, 2.89 (2,3H total), 3.05 (m,2H), 3.26-3.60 (m,5H), 3.73 (s,3H), 3.80 (s,3H), 4.70 (m,1H), 5.07 (broad t,1H), 6.83 (dd,1H), 7.02 (dd,1H).

Example 57

(N-Methyl-2,3-dihydroxypropylamino)carbonyl-(0-methyl)tyrosine.

The resultant compound from Example 56 (114 mg, 0.355 mmol) in dioxane (4 ml) and water (2 ml) at 0° C was treated with LiOH monohydrate (42.0 mg, 1 mmol). After 90 min 2 M HCl (0.6 ml. 1.2 mmol) was added and the mixture was evaporated to a foam which was used without further purification; DCI-MS: $(M+H)^+$ = 327.

Example 58

3,3-Dimethylglutaric Acid Mono t-Butyl Ester.

3,3-Dimethylglutaric anhydride (455 mg, 3.2 mmol) in tetrahydrofuran (THF, 5 ml) was treated with sublimed potassium t-butoxide (395 mg, 3.5 mmol). After 30 min the solution was concentrated, poured into saturated $NaHCO_3$ solution and washed with ether. The aqueous phase was acidified to pH 4 with 0.5 M $H_3PO_4$ and extracted with chloroform which was dried over $Na_2SO_4$ and evaporated to afford 179 mg (26%) of the desired product as an oil. NMR (300 MHz, $CDCl_3$, ppm), 1.13 (s,6H), 1.47 (s,9H), 2.33 (s,2H), 2.45 (s,2H).

Example 59

(4-t-Butyloxycarbonyl-3,3-dimethyl)butanoylphenylalanine Benzyl Ester.

Prepared according to the procedure from Example 31 from the resultant compound from Example 58 and phenylalanine benzyl ester p-toluenesulfonic acid salt. NMR (300 MHz, $CDCl_3$ ppm), 0.96 (s,3H), 1.00 (s,3H), 1.44 (s,9H), 1.90 (d,1H), 2.16 (d,1H), 2.25 (d,1H), 2.29 (d,1H), 3.03 (dd,1H), 3.17 (dd,1H), 4.92 (m,1H), 5.12 (d,1H), 5.16 (d,1H), 7.10-7.40 (m,10H).

Example 60

(4-t-Butyloxycarbonyl-3,3-dimethyl)butanoylphenylalanine.

Using the procedure of Example 50 with the resultant compound of Example 59 gave the desired product as an oil. NMR (300 MHz, $CDCl_3$, ppm), 0.93 (s,3H), 0.99 (s,3H), 1.45 (s,9H), 1.77 (d,1H), 2.10 (d,1H), 2.19 (d,1H), 2.25 (d,1H), 3.02 (dd,1H), 3.33 (dd,1H), 4.72 (m,1H), 7.25 (m,5H).

Example 61

2(R,S)-(4-morpholinylcarbonylmethyl)-3-(1′-naphthyl)-propionic Acid.

To 50 ml of absolute ethanol was added 2 g of sodium metal. The suspension was stirred vigorously until all the sodium dissolved and the evolution of hydrogen ceased. To this solution of sodium ethoxide was added a solution of 11.6 g of diethylsuccinate in 10.4 g of 1-naphthaldehyde. The solution was heated to reflux for 3 h, at which time it was cooled to room temperature and concentrated. The residue was dissolved in 320 ml of water and extracted 6 times with 100 ml portions of ether. The aqueous layer was acidified with 2N HCl and extracted with 2 x 300 ml of ether and dried with anhydrous magnesium sulfate. Evaporation of the solvent gave a yellow gummy solid which was hydrogenated to the saturated acid using Pd/C as catalyst. Coupling of the resulting saturated acid to morpholine using the mixed anhydride method described in Example 31 followed by ester hydrolysis using the procedure of Example 52 gave the desired acid. Mass spectrum: $(M+H)^+$ = 328.

Example 62

[(4-Thiomorpholinyl)carbonyl]-Phe Methyl Ester.

A suspension of L-phenylalanine methyl ester hydrochloride (6 g) in toluene (125 ml) was heated to 100° C and phosgene gas was bubbled into the reaction mixture. After approximately 1.5 h, the mixture became homogeneous. The bubbling of phosgene was continued for 10 more min. The solvent was then evaporated and the residue chased with benzene several times. The residue was then dissolved in ~100 ml of methylene chloride and cooled to ~0° C, and 1.1 equivalent of thiomorpholine was added dropwise. After 10 min the solution was washed with 1N HCl and the organic layer was dried with $MgSO_4$. Evaporation of solvent gave 5.5 g of product. Mass spectrum: $M^+$ = 308.

Example 63

[(4-Sulphonylmorpholinyl)carbonyl]-Phe Methyl Ester.

To 2 g of the product from Example 62 in 100 ml of methylene chloride was added 2.94 g of meta-chloroperbenzoic acid at 0 °C. After 30 min the solvent was evaporated, dissolved in ether, and the ether solution was washed with 10% sodium sulfite solution and then with saturated sodium bicarbonate several times. The organic layer was dried with $MgSO_4$ and evaporation of the solvent gave a white solid which was purified by silica gel column chromatography (20% EtOAc/80%/$CH_2Cl_2$) to give 2.10 g (95%) of pure product. Mass spectrum: $M^+$ = 340.

Example 64

[(4-sulfonylmorpholinyl)carbonyl]-Phe-Oh.

Using the procedure described in Example 35, with the resultant compound from Example 63 gave the desired compound.

Example 65

Boc-6-aminohexanoic Acid.

A mixture of 3.0 g (0.02 mol) of 6-aminohexanoic acid, 5.04 g (0.02 mol) of di-t-butyldicarbonate and 3.84 g (0.05 mol) of $NaHCO_3$ in 160 mol of 1:1 $H_2O$/tetrahydrofuran was stirred vigorously for 24 h. After concentration of the solvent, the mixture was acidified with HCl, saturated with NaCl, extracted with ethyl acetate, dried over $MgSO_4$ and concentrated in vacuo to give the desired product ($R_f$ 0.48, 9:1 chloroform/methanol).

Example 66

Boc-6-aminohexanoyl-(Me)Tyr-$OCH_3$.

The resulting compound from Example 65 and the hydrochloride salt of 0-methyltyrosine methyl ester were reacted according to the procedure of Example 31 to provide the desired product as an oil.

Example 67

Boc-6-aminohexanoyl-(Me)Tyr-OH.

The resulting compound from Example 66 was hydrolized according to the procedure of Example 35 to provide the desired product.

## Example 68

N-Methyl-N-(2-(N,N-dimethylamino)ethyl)carbamoyl-(0-methyl)tyrosine Methyl Ester.

A solution of 0.5 g (2.1 mmol) of the resultant compound of Example 53 in 50 ml of dichloromethane was cooled to 0°C and treated with 0.3 ml (2.3 mmol) of N,N,N′-trimethylethylenediamine. After being allowed to stir for 16 h, the solution was concentrated and the desired compound was isolated by flash column chromatography using 1% methanol/2% isopropylamine in chloroform.

## Example 69

N-Methyl-N-(2-(N,N-dimethylamino)ethyl)carbamoyl-(0-methyl)tyrosine Lithium Salt.

A solution of the resultant compound of Example 68 in dioxane was cooled to 0°C, treated with 1.05 equivalent of aqueous lithium hydroxide (0.5 M) and stirred for 1.5 h. The resulting solution was concentrated *in vacuo* to give the desired compound as a white solid.

## Example 70

Isobutyryl (0-methyl)tyrosine.

To (0-methyl)tyrosine (126 mg, 0.647 mmol) in dioxane (3 ml) was added NaOH (52 mg, 1.3 mmol) in water (3 ml). Isobutyric anhydride (0.11 ml, 0.66 mmol) was added and the mixture was stirred at 0°C for 2 h, poured into saturated $NaHCO_3$ solution and extracted with ether.

The aqueous phase was acidified with concentrated HCl then extracted with ethyl acetate which was dried over $Na_2SO_4$ and evaporated. Trituration with hot ethyl acetate afforded 132 mg (77%) of the desired product as a solid.

Anal. Calcd. for $C_{14}H_{19}NO_4 \bullet 0.25\ H_2O$:

C, 62.32; H, 7.28; N, 5.19.

Found:

C, 62.68; H, 7.29; N, 5.18.

## Example 71

Isobutyryl (phenylmethyl)alanine.

Prepared from phenylmethylalanine according to the procedure of Example 70 to give the desired product as an oil.

## Example 72

Isobutyryl (0-benzyl)threonine.

Prepared according to the procedure of Example 70 from (0-benzyl)threonine to give the desired product as a solid.
Anal. Calcd. for $C_{15}H_{21}NO_4$:
C, 64.50; H, 7.58; N, 5.01.
Found:
C, 64.65; H, 7.51; N, 4.75.

Example 73

Morpholinocarbonyl-(0-methyl)tyrosine Methyl Ester.

To the resultant compound from Example 53 in methylene chloride at 0 °C was added one equivalent of morpholine. After 1 h the solvent was evaporated and the residue was chromatographed on silica gel with ethyl acetate/hexane mixtures to provide the desired produce.

Example 74

Morpholinocarbonyl-(0-methyl)tyrosine.

Using the procedure from Example 35 with the resultant compound from Example 73 gave the desired product.
Anal. Calcd. for $C_{15}H_{20}N_2O_5 \bullet 0.25\ H_2O$:
C, 57.59; H, 6.66; N, 8.95.
Found:
C, 57.79; H, 6.57; N, 8.93.

Example 75

Phenethylmethylaminocarbonyl-(0-methyl) tyrosine Methyl Ester.

Prepared according to the procedure of Example 73 replacing morpholine with N-methyl-2-phenylethylamine. $^1$H-NMR ($CDCl_3$, TMS) $\delta$ 7.25 (m,5H), 7.02 (m,2H), 6.82 (m,2H), 4.73 (m,2H), 3.78 (s,3H), 3.73 (s,3H), 3.45 (n,2H), 3.03 (d,2H), 2.81 (d,2H), 2.77 (s,3H).

Example 76

Phenethylmethylaminocarbonyl-(0-methyl)tyrosine.

Using the procedure from Example 35 with the resultant compound from Example 75 gave the desired product. $^{1}H$-NMR ($CDCl_3$, TMS) $\delta$ 7.28 (m,3H), 7.10 (m,4H), 6.82 (m,2H), 4.40 (m,2H), 3.78 (s,3H), 3.38 (m,2H), 2.70 (s,3H).

Example 77

Diisopropylaminocarbonyl-Phe-$OCH_3$.

Using the procedure of Example 62 but replacing the thiomorpholine with diisopropylamine provided the desired product. $^{1}H$-NMR ($CDCl_3$, TMS) $\delta$ 7.10-7.40 (m,5H), 4.83 (m,1H), 4.66 (d,2H), 3.88 (m,2H), 3.74 (s,3H), 3.20 (dd,1H), 3.12 (dd,1H), 1.19 (d,6H), 1.15 (d,6H).

58

Example 78

Diisopropylaminocarbonylphenylalanine.

Using the procedure of Example 35 with the resultant compound of Example 77 gave the desired product. $^{1}H$-NMR ($CDCl_3$, TMS) $\delta$ 7.28 (m,5H), 4.48 (m,2H), 3.72 (s,3H), 3.69 (m,2H), 3.34 (dd,1H), 3.19 (dd,1H), 1.11 (d,6H), 1.05 (d,6H).

Example 79

2-Ethyl-2-methylbutanoyl-Phe-OH.

Using the procedure of Example 70 and replacing isobutyric anhydride with 2-ethyl-2-methylbutanoyl chloride gave the desired compound. $^{1}H$-NMR ($CDCl_3$, TMS) $\delta$ 7.15-7.35 (m,5H), 600 (d,1H), 4.78 (m,1H), 3.27 (dd,1H), 3.08 (dd,1H), 1.55 (m,2H), 1.35 (m,2H), 1.00 (s,3H), 0.70 (m,6H).

Example 80

Benzyl (2R)-2-Benzyl-3-[N-(2-hydroxylethyl)-N-methyl]-aminocarbonylpropionate.

Using the mixed anhydride coupling procedure of Example 31 with the resultant compound from Example 43 and N-methyl-ethanolamine gave the desired compound. $^{1}H$-NMR ($CDCl_3$, TMS) $\delta$ 7.10-7.40 (m,10H), 5.10 (m,2H), 3.00, 2.92 (s,total 3H).

Example 81

(2R)-2-Benzyl-3-]N-(2-hydroxyethyl)-N-methyl]aminocarbonylpropionic Acid.

Using the procedure of Example 46 with the resultant compound of Example 80 gave the desired compound. $^1$H-NMR ($CDCl_3$, TMS) δ 7.25 (m,5H), 2.96,2.93 (s, total 3H).

Example 82

Benzyl (2R)-2-Benzyl-3-methylaminocarbonylpropionate.

Using the mixed anhydride coupling procedure of Example 31 with the resultant compound from Example 43 and methylamine gave the desired product. $^1$H-NMR ($CDCl_3$, TMS) δ 7.05-7.40 (m,10H), 5.42 (broad s,1H), 5.12 (d,1H), 5.06 (d,1H), 3.29 (m,1H), 3.02 (dd,1H), 2.83 (dd,1H), 2.72 (d,3H), 2.48 (dd,1H), 2.27 (dd,1H).

Example 83

(2R)-2-Benzyl-3-methylaminocarbonylpropionic Acid.

Using the procedure of Example 46 with the resultant compound of Example 82 gave the desired product. $^1$H-NMR ($CDCl_3$, TMS) δ 7.15-7.35 (m,5H), 5.83 (m,1H), 2.72 (d,3H).

Example 84

2-N-methyl-benzyloxycarbonylaminoethanol.

Using the procedure of Example 25 and replacing ethanolamine with N-methylethanolamine provided the desired product. $^1$H-NMR ($CDCl_3$, TMS) δ 7.36 (m,5H), 3.78 (m,2H), 3.47 (m,2H), 5.14 (s,2H), 3.01 (s,3H).

Example 85

1-Methoxymethoxy-2-(N-methylbenzyloxycarbonylamino)ethane.

Using the procedure of Example 26 with the resultant compound from Example 84 gave the desired product. $^1$H-NMR ($CDCl_3$, TMS) δ 7.37 (m,5H), 5.14 (s,2H), 4.09 (m,2H), 3.67 (m,2H), 3.50 (m,2H), 3.33 (m,3H), 3.02 (s,3H).

Example 86

1-Methoxyethoxymethoxy-2-(N-methylbenzyloxycarbonylamino)ethane.

Using the procedure of Example 26 with the resultant compound from Example 84 and replacing chloromethyl methyl ether with 2-methoxyethoxy methylchloride gave the desired compound, b.p. 150-170° C (0.3 mm).

Example 87

(3,4-cis-Dihydroxypyrrolidinylcarbonyl)-(0-methyl)tyrosine Methyl Ester.

A suspension of L-(0-methyl)tyrosine methyl ester hydrochloride (10 g) in toluene (~200 ml) was heated to 100° C while phosgene gas was bubbled into the reaction mixture. After approximately 2 h the mixture became homogeneous. The bubbling of phosgene was continued for 15 more minutes keeping the temperature at ~100° C. The toluene was then evaporated and the residue chased with benzene several times. The isocyanate from L-(Me)Tyr-$OCH_3$ was then dissolved in ~100 ml of methylene chloride and 1.1 equivalent of 3-pyrroline (75% pure) was added dropwise at 0° C. After 15 min, the reaction mixture was washed with 0.5 N HC1 and methylene chloride. The organic layer was washed with aqueous $NaHCO_3$ and dried over $MgSO_4$. Evaporation of the solvent gave 3-pyrrolinylcarbonyl-(Me)Tyr-methyl ester which was cis-hydroxylated under the following conditions: 2.5 g of the 3-pyrrolinylcarbonyl-(Me)Tyr-methyl ester was dissolved in 50 ml of THF and 1 ml of a 2.5% solution of $OsO_4$ in t-butanol was added, followed by 1.15 g of N-methylmorpholine-N-oxide. After 1 h, the solvent was evaporated and the residue dissolved in 150 ml of ethyl acetate and washed with dilute $Na_2SO_3$ solution, saturated $NaHCO_3$ solution and then dried with $MgSO_4$. Evaporation of the solvent gave a gummy solid which was purified by $SiO_2$ column chromatography (5% $MeOH/CH_2Cl_2$) to give the desired compound (65% yield). Mass spectrum: $M^+$ = 338.

Example 88

(3,4-cis-Dihydroxypyrrolidinylcarbonyl)-(0-methyl)tyrosine.

Using the procedure from Example 35 with the resultant compound from Example 87 and replacing ether extractions with chloroform extractions gave the desired product. Mass spectrum: $M^+$ = 324.

Example 89

1-Benzyloxycarbonyl-3,4-cis-dihydroxypyrrolidine.

3-Pyrroline (75% pure) was reacted with benzyl chloroformate according to the procedure of Example 25 and the resulting product was cis-hydroxylated according to the procedure of Example 87 to give the desired product.

## Example 90

### 1-Benzyloxycarbonyl-3,4-cis-dimethoxymethoxypyrrolidine.

Using the procedure of Example 26 with the resultant compound from Example 89 gave the desired product. $^1$H-NMR ($CDCl_3$, TMS) δ 7.35 (m,5H), 5.16 (d,1H), 5.13 (d,1H), 4.73 (m,4H), 4.21 (m,2H), 3.60 (m,4H), 3.40 (s,3H), 3.38 (s,3H).

## Example 91

### 1-Methylamino-2-methoxymethoxyethane.

Using the procedure of Example 27 with the resultant compound from Example 85 gave the desired product, b.p. 60-80 °C (60 mm).

## Example 92

### 1-Methylamino-2-methoxyethoxymethoxyethane.

Using the procedure of Example 27 with the resultant compound from Example 86 gave the desired product, b.p. 130-140 °C (45 mm).

## Example 93

### Cis-3,4-dimethoxymethoxypyrrolidine.

Using the procedure of Example 27 with the resultant compound from Example 90 gave the desired product. $^1$H-NMR ($CDCl_3$, TMS) δ 4.71 (m,4H), 4.12 (m,2H), 3.38 (s,6H), 3.09 (m,2H), 2.90 (m,2H).

## Example 94

### Benzyl (2R)-2-Benzyl-3-(3,4-cis-dimethoxymethoxypyrrolidinocarbonyl) propionate.

Using the procedure of Example 31 with the resultant compounds from Example 43 and Example 93 gave the desired product. $^1$H-NMR ($CDCl_3$, TMS) δ 7.10-7.35 (m,10H), 5.10 (m,2H), 4.72 (m,4H), 4.18 (m,2H), 3.37 (m,6H), 3.05 (m,1H), 2.82 (m,1H), 2.62 (m,1H), 2.30 (m,1H).

Example 95

Benzyl (2R)-2-Benzyl-3-(N-Methyl-N-2-methoxyethoxymethoxyethylaminocarbonyl)propionate.

Using the procedure of Example 31 with the resultant compounds from Example 43 and Example 92 gave the desired product.
Anal. Calcd. for $C_{25}H_{33}NO_6$:
C, 67.70; H, 7.50; N, 3.16.
Found:
C, 67.79; H, 7.12; N, 3.15.

Example 96

(2R)-2-Benzyl-3-(3,4-cis-dimethoxymethoxypyrrolidinocarbonyl) propionic Acid.

Using the procedure of Example 46 with the resultant compound from Example 94 gave the desired product. $^1$H-NMR ($CDCl_3$, TMS) δ 7.26 (m,5H), 4.70 (m,4H), 4.20 (m,2H), 2.75 (dd,1H).

Example 97

(2R)-2-Benzyl-3-(N-Methyl-N-2-methoxyethoxymethoxyethylaminocarbonyl) propionic Acid.

Using the procedure of Example 46 with the resultant compound from Example 95 gave the desired product. $^1$H-NMR ($CDCl_3$, TMS) δ 7.27 (m,5H), 4.70,4.55 (m, total 2H), 3.40,3.41 (s, total 3H), 2.97,2.93 (s, total 3H).

Example 98

N-Methyl-(2-methoxymethoxyethyl)aminocarbonyl-(0-methyl)tyrosineMethyl Ester.

Using the procedure of Example 73 and replacing morpholine with the resultant compound from Example 91 gave the desired product. $^1$H-NMR ($CDCl_3$, TMS) δ 7.06 (m,2H), 6.82 (m,2H), 5.52 (broad d,1H), 4.70 (m,1H), 4.53 (s,2H), 3.89 (s,3H), 3.82 (s,3H), 3.29 (s,3H), 2.92 (s,3H).

Example 99

N-methyl-(2-methoxymethoxyethyl)aminocarbonyl-(0-methyl)tyrosine.

Using the procedure of Example 35 with the resultant compound from Example 98 gave the desired product. $^1$H-NMR ($CDCl_3$, TMS) $\delta$ 7.13 (m,2H), 6.84 (m,2H), 4.53 (d,1H), 4.51 (d,1H), 3.80 (s,3H), 3.60 (m,2H), 3.29 (s,3H), 2.89 (s,3H).

## Example 100

(N-butyl, 4-$OCH_3$)-phenylalanine.

To a stirred 0°C suspension of (4-$OCH_3$)-phenylalanine (1.00 g, 5.12 mmol) and butyraldehyde (0.406 g, 110M%) in methanol (10 ml) was added sodium cyanoborohydride (241 mg, 75 M%). The mixture was warmed to room temperature for 23 h and filtered. The solid was washed with methanol and suction dried to give 1.07 g (83%) of the desired product. Mass spectrum: $M^+$ = 251.

Anal. Calcd. for $C_{14}H_{21}NO_3 \bullet 1/3\ H_2O$:
C, 65.3; H, 8.5; n, 5.4.
Found:
C, 65.1; H, 8.3; N, 5.6.

## Example 101

Ethyl hydrogen (α,α-dimethylbenzyl)malonate.

Diethyl (α,α-dimethylbenzyl)malonate was prepared by the conjugate addition of phenyl magnesium bromide to diethyl isopropylidenemalonate as described by C. Holmberg [Liebigs Ann. Chem., 748 (1981)]. A solution of this diester (42.1 g, 0.15 mmol) in ethanol (100 ml) was treated by dropwise addition with a solution of potassium hydroxide (8.48 g, 0.13 mmol) in 100 ml of ethanol. After heating at 90°C for 1 h and at 50°C for 20 h, the reaction mixture was evaporated on the rotary evaporator to a residue. The residue was diluted with water and extracted with ether to remove unreacted starting material. The aqueous phase was cooled to 5°C, acidified to pH 3 with 6N HCl, and extracted with methylene chloride. The organic layer was washed with brine solution and dried over magnesium sulfate. Evaporation of the solvent gave 27.3 g (84%) of liquid product. 1HMR ($CDC1_3$): 1.05 (t,3H), 1.6 (s,6H), 3.78 (s,1H), 3.96 (m,2H), 7.2-7.4 (m,5H).

## Example 102

Ethyl 2(R,S)-[[(4-morpholinyl)carbonyl]amino]-3, 3-dimethyl-3-phenylpropionate.

To a solution of ethyl hydrogen (α,α-dimethylbenzyl)malonate (4 g, 0.016 mmol) in toluene was added triethylamine (2.33 ml, 0.016 mmol) and diphenyl-phosphoryl azide (4.4 g, 0.016 mmol). The reaction mixture was heated at 100°C for 2.5 h, cooled to 5°C, and treated with 1.4 ml (0.016 mmol) of orpholine. After stirring overnight at room temperature, the toluene solution was washed successively with 1N HC1 and aqueous sodium bicarbonate solution. The dried organic solution was evaporated to a residue which was purified by column chromatography on silica gel. There was obtained 3.7 g (69%) of product after trituration with hexane, m.p. 93-94°C.

Anal. Calcd. for $C_{18}H_{26}N_2O_4$:
C, 64.55; H, 7.84; N, 8.38.
Found:
C, 64.72; H, 7.95; N, 8.33.

Example 103

2(R,S)-[[4-Morpholinyl)carbonyl]amino]-3,3-dimethyl-3-phenylpropionic Acid.

A solution of the product from Example 102 (2 g, 5.99 mmol) in dioxane (10 ml) was treated with 0.26 g (6.5 mmol) of sodium hydroxide in 5 ml of water. After stirring for 16 h at 35° C, the reaction was worked up as described in Example 35 to give a 93% yield of product.

Example 104

(2′S,1′R,5S)-3-Ethyl-5-(1′-methoxyethoxymethoxy-2′-tert-butyloxycarbonylamino-3′-cyclohexylpropyl)-oxazolidin-2-thione.

Using the procedure of Example 7 but replacing the phosgene in toluene with thiophosgene provided the desired product.

Example 105

Boc-Phe-His Amide of (2′S,1′R,5S)-3-benzyloxycarbonylmethylamino-5-(1′-hydroxy-2′-amino-3′-cyclohexylpropyl)-oxazolidin-2-one.

Using the procedure of Example 30 with the resultant compound from Example 9 and Boc-Phe-His-OH in place of Boc-His-OH provided the desired compound.

Example 106

Boc-Phe-His Amide of (2′S,1′R,5S)-3-Methylamino-5-(1′-hydroxy-2′-amino-3′-cyclohexylpropyl)-oxazolidin-2-one Acetic Acid Salt.

The resultant compound from Example 105 and an equal weight of 10% palladium on carbon in acetic acid were stirred under a hydrogen atmosphere for 6 h. The mixture was filtered and evaporated to provide the desired product.

Example 107

Boc-Phe-His Amide of (2′S,1′R,5S)-3-Ethyl-5-(1′-hydroxy-2′-amino-3′-cyclohexylpropyl) imidazolidin-2-one.

Using the procedure of Example 30 with the resultant compound from Example 14 and using Boc-Phe-His-OH in place of Boc-His-OH provided the desired product.

Example 108

Boc-Phe-His Amide of (4S,1′R,2′S)-3-Aza-4-(1′-hydroxy-2′-amino-3′-cyclohexylpropyl)piperidin-2-one.

Using the procedure of Example 30 with the resultant compound from Example 21 and using Boc-Phe-His-OH in place of Boc-His-OH provided the desired compound.

Example 109

Boc-Phe-His Amide of (6S,1′R,2′S)-3,4-Diaza-2-oxo-4-methyl-6-(1′-hydroxy-2′-amino-3′-cyclohexylpropyl)-tetrahydropyran.

Using the procedure of Example 30 with the resultant compound from Example 24 and using Boc-Phe-His-OH in place of Boc-His-OH gave the desired product.

Example 110

Boc-Phe-His Amide of (2′S,1′R,5S)-3-Ethyl-5-(1′-hydroxy-2′-amino-3′-cyclohexylpropyl)oxazolidin-2-thione.

Using the procedure of Example 30 with the resultant compound from Example 104 and using Boc-Phe-His-OH in place of Boc-His-OH gave the desired product.

Example 111

Cbz-[(β,β-di-Me)-β-Ala]-Phe-(Me)His Amide of (2′S,1′R,5S)-3-Ethyl-5-(1′-hydroxy-2′-amino-3′-cyclohexylpropyl)oxazolidin-2-one.

The resultant compound from Example 32 was deprotected according to the procedure of Example 29 and coupled to the resultant compound of Example 35 using the procedure of Example 32 to give the desired compound.

Example 112

[(β,β-di-Me)-β-Ala]-Phe-(Me)His Amide of (2′S,1′R,5S)-3-Ethyl-5-(1′-hydroxy-2′-amino-3′-cyclohexylpropyl)-oxazolidin-2-one Acetic Acid Salt.

Using the procedure of Example 106 with the resultant compound of Example 111 gave the desired product 70.

## Example 113

Boc-Phe-His Amide of (2′S,1′R,5S)-3-Methoxy-5-(1′-hydroxy-2′-amino-3′-cyclohexylpropyl) oxazolidin-2-one.

Using the procedure of Example 30 with the resultant compound from Example 8 and using Boc-Phe-His-OH in place of Boc-His-OH gave the desired product, melting point 183-187° C.
Anal. Calcd. for $C_{33}H_{48}N_6O_8 \bullet 1.5\ H_2O$:
C, 57.97; H, 7.35; N, 12.29.
Found:
C, 58.37; H, 7.25; N, 12.05.

## Example 114

Boc-Phe-Leu Amide of (2′S,1′R,5S)-2-Oxo-4-(1′-hydroxy-2′-amino-3′-cyclohexylpropyl)dioxolane.

Using the procedure of Example 31 with the resultant compound from Example 11 and using Boc-Phe-Leu-OH in place of Boc-Leu-OH gave the desired product, melting point 155-157° C. $^1$H-NMR ($CDCl_3$, TMS) δ 7.34 (m,3H),7.20 (m,2H), 6.90 (d,1H), 6.20 (d,1H), 4.88 (d,1H), 3.93 (m,1H), 4.65 (m,1H), 3.15 (dd,1H), 3.03 (dd,1H), 1.43 (s,9H).

## Example 115

Boc-Phe-His Amide of (6S,1′R,2′S)-3-Aza-2-oxo-6-(1′-hydroxy-2′-amino-3′-cyclohoxylpropyl)tetrahydropyran.

Using the procedure of Example 30 with the resultant compound from Example 17 and using Boc-Phe-His-OH in place of Boc-His-OH gave the desired product, melting point 146-148° C.
Anal. Calcd. for $C_{33}$-$H_{48}$-$N_6O_7 \bullet 1/3\ H_2O$:
C, 61.28; H, 7.58; N, 12.99.
Found:
C, 61.27; H, 7.83; N, 12.89.

## Example 116

Boc-Phe-His Amide of (2′S,1′R,5S)-3-(2-hydroxyethyl)-5-(1′-hydroxy-2′-amino-3′-cyclohexylpropyl) oxazolidin-2-one.

Using the procedure of Example 30 with the resultant compound from Example 28 and using Boc-Phe-His-OH in place of Boc-His-OH gave the desired product, melting point 135-138 ° C.
Anal. Calcd. for $C_{34}H_{50}N_6O_8 \bullet 1.75\ H_2O$:
C, 58.15; H, 7.68; N, 11.97.
Found:
C, 58.39; H, 7.59; N, 11.59.

## Example 117

Boc-His-Amide of (2′S,1′R,5S)-3-(2-hydroxyethyl)-5-(1′-hydroxy-2′-amino-3′-cyclohexylpropyl) oxazolidin-2-one.

Using the procedure of Example 30 with the resultant compound of Example 28 provided the desired product.

## Example 118

(2R)-2-Benzyl-3[(2-methoxyethoxymethoxyethyl) methylaminocarbonyl]propionyl-His Amide of (2 S,1 R,5S)-3-(2-hydroxyethyl)-5-(1 -hydroxy-2 -amino-3 -cyclohexylpropyl)oxazolidin-2-one.

Using the procedure of Example 30 with the resultant compound from Example 117 and using the resultant compound from Example 97 in place of Boc-His-OH provided the desired product, melting point 52-56 ° C.
Anal. Calcd. for $C_{38}H_{58}N_6O_{10} \bullet 0.55\ H_2O$:
C, 59.37; H, 7.75; N, 10.93.
Found:
C, 59.76; H, 7.94; N, 10.46.

## Example 119

Boc-His Amide of (2′S,1′R,5S)-3-Methoxy-5(1′-hydroxy-2′-amino-3′-cyclohexylpropyl)oxazolidin-2-one.

Using the procedure of Example 30 with the resultant from Example 8 provided the desired product.
Anal. Calcd. for $C_{24}H_{39}N_5O_7$:
C, 56.57; H, 7.71; N, 13.74,
Found:
C, 56.48; H, 7.63; N, 13.77.

## Example 120

(2R)-2-Benzyl-3-(morpholinocarbamoyl)propionyl-His Amide of (2'S,1'R,5S)-3-Methoxy-5-(1'-hydroxy-2'-amino-3'-cyclohexylpropyl)oxazolidin-2-one.

Using the procedure of Example 30 with the resultant compound from Example 119 and using the resultant compound from Example 46 in place of Boc-His-OH gave the desired product, melting point 147-151 °C.
Anal. Calcd. for $C_{34}H_{49}N_7O_8 \bullet 0.5\ H_2O$:
C, 58.94; H, 7.27; N, 14.15.
Found:
C, 58.96; H, 7.28; N, 14.00.

## Example 121

(2R)-2-Benzyl-3-ethoxycarbamoylpropionyl-His Amide of (2'S,1'R,5S)-3-Methoxy-5-(1'-hydroxy-2'-amino-3'-cyclohexylpropyl)oxazolidin-2-one.

Using the procedure of Example 30 with the resultant compound from Example 119 and using the resultant compound of Example 47 in place of Boc-His-OH provided the desired compound, melting point 118-123 °C.
Anal. Calcd. for $C_{32}H_{46}N_6O_8 \bullet 0.5\ H_2O$:
C, 58.97; H, 7.27; N, 12.89.
Found:
C, 59.30; H, 7.41; N, 12.49.

## Example 122

Boc-6-aminohexanoyl-(Me)Tyr-His Amide of (2'S,1'R,5S)-3-Ethyl-5-(1'-hydroxy-2'-amino-3'-cyclohexylpropyl)oxazolidin-2-one.

Using the procedure of Example 30 with the resultant compound from Example 30 and replacing Boc-His-OH with the resultant compound from Example 67 provided the desired compound.

## Example 123

Cbz-[(β,β-di-Me)-β-Ala]-(Me)Tyr-His Amide of (2'S,1'R,5S)-3-Ethyl-5-(1'-hydroxy-2'-amino-3'-cyclohexylpropyl)oxazolidin-2-one.

Using the procedure of Example 30 with the resultant compound from Example 30 and replacing Boc-His-OH with the resultant compound from Example 37 provided the desired compound, melting point 109-111 °C.
Anal. Calcd. for $C_{43}H_{59}N_7O_9$:
C, 62.79; H, 7.29; N, 11.92
Found:
C, 62.69; H, 7.24; N, 11.88.

Example 124

6-Aminohexanoyl-(Me)Tyr-His Amide of (2′S,1′R,5S)-3-Ethyl-5-(1′-hydroxy-2′-amino-3′-cyclohexylpropyl)-oxazolidin-2-one.

The resultant compound from Example 122 was deprotected according to the procedure of Example 29 to provide the desired product, melting point 80-82° C.
Anal. Calcd. for $C_{35}H_{55}N_7O_7 \bullet 1.65\ H_2O$:
C, 59.43; H, 8.08; N, 13.48.
Found:
C, 59.06; H, 7.68; N, 13.20.

Example 125

[(β,β-di-Me)-β-Ala]-His Amide of (2′S,1′R,5S)-3-Ethyl-5-(1′-hydroxy-2′-amino-3′-cyclohexylpropyl) oxazolidin-2-one Acetic Acid Salt.

The resultant compound from Example 123 was deprotected according to the procedure of Example 106 to give the desired product, melting point 73-75° C.
Anal. Calcd. for $C_{39}H_{61}N_7O_{11} \bullet 1.39\ H_2O$:
C, 56.50; H, 7.75; N, 11.83.
Found:
C, 56.10; H, 7.35; N, 12.23.

Example 126

(2S)-[(4-morpholino)carbonyl]oxy-3-phenylpropionyl His Amide of (2′S,1′R,5S)-3-Ethyl-5-(1′-hydroxy-2′-amino-3′-cyclohexylpropyl)oxazolidin-2-one.

Using the procedure of Example 30 with the resultant compound from Example 30 and replacing Boc-His-OH with the resultant compound from Example 39 provided the desired compound.

Example 127

(2R)-2-Benzyl-3-morpholinocarbonylpropionyl-His Amide of (2′S,1′R,5S)-3-Ethyl-5-(1′-hydroxy-2′-amino-3′-cyclohexylpropyl)oxazolidin-2-one.

Using the procedure of Example 30 with the resultant compound from Example 30 and replacing Boc-His-OH with the resultant compound from Example 49 provided the desired compound, melting point 112-114° C.

Anal. Calcd. for $C_{35}H_{50}N_6O_7 \bullet 1.5\ H_2O$:
C, 60.59; H, 7.70; N, 12.11.
Found:
C, 60.57; H, 7.54; N, 11.94.

## Example 128

### Morpholinocarbonyl(Me)Tyr-Leu Amide of (2′S,1′R,5S)-3-Ethyl-5-(1′-hydroxy-2′-amino-3′-cyclohexylpropyl) oxazolidin-2-one.

Using the procedure of Example 31 with the resultant compound from Example 31 and replacing Boc-Leu-OH with the resultant compound from Example 74 provided the desired compound, melting point 120-123° C.
Exact Mass Calcd. for $C_{35}H_{56}N_5O_8$ M + H):
674.4129.
Found:
674.4116.

## Example 129

### Boc-Phe-(dl)-(3-pyrazolyl)Ala Amide of (2′S,1′R,5S)-3-Ethyl-5-(1′-hydroxy-2′-amino-3′-cyclohexylpropyl)-oxazolidin-2-one.

Using the procedure of Example 30 with the resultant compound from Example 30 and replacing Boc-His-OH with the resultant compound from Example 52 provided the desired compound.

## Example 130

### (2R)-3-t-Butyloxycarbonyl-2-benzylpropionyl-His Amide of (2′S,1′R,5S)-3-Ethyl-5-(1′-hydroxy-2′-amino-3′-cyclohexylpropyl)oxazolidin-2-one.

Using the procedure of Example 30 with the resultant compound from Example 30 and replacing Boc-His-OH with the resultant compound from Example 50 provided the desired compound, melting point 116-119° C. $^1$H-NMR ($CD_3OD$, TMS) δ 7.62 (d,1H), 7.22 (m,5H), 6.92 (d,1H), 1.40 (s,9H), 1.15 (t,3H).
Anal. Calcd. for $C_{31}H_{51}N_5O_7 \bullet 0.5\ H_2O$:
C, 63.42; H, 7.91; N, 10.57.
Found:
C, 63.17; H, 7.71; N, 10.34.

## Example 131

(4-t-Butyloxycarbonyl-3,3-dimethyl)butanoyl-Phe-His Amide of(2′S,1′R,5S)-3-Ethyl-5-(1′-hydroxy-2′-amino-3′-cyclohexylpropyl)oxazolidin-2-one.

Using the procedure of Example 30 with the resultant compound from Example 30 and replacing Boc-His-OH with the resultant compound from Example 60 provided the desired compound.

Example 132

(2R)-3-Carboxy-2-benzylpropionyl-His Amide of (2′S,1′R,5S)-3-Ethyl-5-(1′-hydroxy-2′-amino-3′-cyclohexylpropyl)oxazolidin-2-oneTrifluoroacetic Acid Salt.

The resultant compound from Example 130 was stirred for 2 h in trifluoroacetic acid. The solvent was evaporated with methanol chasers to afford the desired product, melting point 112-115° C. $^1$H-NMR ($CD_3OD$, TMS) $\delta$ 8.73 (d,1H), 7.23 (m,6H), 4.62 (dd,1H), 4.30 (m,1H), 4.17 (m,1H), 2.28 (dd,1H), 1.16 (t,3H).

Example 133

(4-Carboxy-3,3-dimethyl)butanoyl-Phe-His Amide of (2′S,1′R,5S)-3-Ethyl-5-(1′-hydroxy-2′-amino-3′-cyclohexylpropyl)oxazolidin-2-oneTrifluoroacetic Acid Salt.

The resultant compound from Example 131 was treated according to the procedure of Example 132 to provide the desired product.

Example 134

(N-Methyl-2,3-dihydroxypropylamino)carbonyl-(Me)Tyr-His Amide of (2′S,1′R,5S)-3-Ethyl-5-(1′-hydroxy-2′-amino-3′-cyclohexylpropyl)oxazolidin-2-one.

Using the procedure of Example 30 with the resultant compound from Example 30 and replacing Boc-His-OH with the resultant compound from Example 57 provided the desired compound, melting point 107-110° C.

Anal. Calcd. for $C_{35}H_{53}N_7O_9 \cdot 2\ H_2O$:
C, 55.91; H, 7.64; N, 13.07.
Found:
C, 55.74; H, 7.31; N, 13.13.

Example 135

(2RS)-(4-morpolinylcarbonylmethyl)-3-(1′-napthyl)propionyl-His Amide of (2′S,1′R,5S)-3-Ethyl-5-(1′-hydroxy-2′-amino-3′-cyclohexylpropyl)oxazolidin-2-one.

Using the procedure of Example 30 with the resultant compound from Example 30 and replacing Boc-His-OH with the resultant compound from Example 61 provided the desired compound.

Example 136

[(4-Sulfonylmorpholinyl)carbonyl]-Phe-His Amide of (2′S,1′R,5S)-3-Ethyl-5-(1′-hydroxy-2′-amino-3′-cyclohexylpropyl)oxazolidin-2-one.

Using the procedure of Example 30 with the resultant compound from Example 30 and replacing Boc-His-OH with the resultant compound from Example 64 provided the desired compound.

Example 137

N-Methyl-N-(2-(N,N-dimethylamino)ethyl)carbamoyl(Me)Tyr-His Amide of (2′S,1′R,5S)-3-Ethyl-5-(1′-hydroxy-2′-amino-3′-cyclohexylpropyl)oxazolidin-2-one.

Using the procedure of Example 30 with the resultant compound from Example 30 and replacing Boc-His-OH with the resultant compound from Example 69 provided the desired compound.

Example 138

Isobutyryl-(Me)Tyr-His Amide of (2′S,1′R,5S)-3-Ethyl-5-(1′-hydroxy-2′-amino-3′-cyclohexylpropyl) oxazolidin-2-one.

Using the procedure of Example 30 with the resultant compound from Example 30 and replacing Boc-His-OH with the resultant compound from Example 70 provided the desired compound, melting point 155-160° C.
Anal. Calcd. for $C_{34}H_{50}N_6O_7 \bullet 2/3\ H_2O$:
C, 61.24; H, 7.76; N, 12.60.
Found:
C, 61.61; H, 7.78; N, 12.27.

Example 139

Isobutyryl-(phenylmethyl)Ala-His Amide of (2′S,1′R,5S)-3-Ethyl-5-(1′-hydroxy-2′-amino-3′-cyclohexylpropyl)oxazolidin-2-one.

Using the procedure of Example 30 with the resultant compound from Example 30 and replacing Boc-His-OH with the resultant compound from Example 71 provided the desired compound.

Example 140

Isobutyryl-(0-benzyl)Thr-His Amide of (2′S,1′R,5S)-3-Ethyl-5-(1′-hydroxy-2′-amino-3′-cyclohexylpropyl)oxazolidin-2-one.

Using the procedure of Example 30 with the resultant compound from Example 30 and replacing Boc-His-OH with the resultant compound from Example 72 provided the desired compound.

Example 141

Phenethylmethylaminocarbonyl-(Me)Tyr-His Amide of (2′S,1′R,5S)-3-Ethyl-5-(1′-hydroxy-2′-amino-3′-cyclohexylpropyl)oxazolidin-2-one.

Using the procedure of Example 30 with the resultant compound from Example 30 and replacing Boc-His-OH with the resultant compound from Example 76 provided the desired compound, melting point 120-125°C.

Anal. Calcd. for $C_{40}H_{55}N_7O_7 \bullet 0.5\ H_2O$:
C, 63.64; H, 7.48; N, 12.99.
Found:
C, 64.02; H, 7.54; N, 12.66.

Example 142

Diisopropylaminocarbonyl-Phe-His Amide of (2′S,1′R,5S)-3-Ethyl-5-(1′-hydroxy-2′-amino-3′-cyclohexylpropyl)oxazolidin-2-one.

Using the procedure of Example 30 with the resultant compound from Example 30 and replacing Boc-His-OH with the resultant compound from Example 78 provided the desired compound, melting point 131-134°C.

Anal. Calcd. for $C_{36}H_{55}N_7O_6 \bullet 0.9\ H_2O$:
C, 61.94; H, 8.20; N, 14.04.
Found:
C, 62.22; H, 8.11; N, 13.66.

Example 143

2-Ethyl-2-methylbutanoyl-Phe-His Amide of (2′S,1′R,5S)-3-Ethyl-5-(1′-hydroxy-2′-amino-3′-cyclohexylpropyl)oxazolidin-2-one.

Using the procedure of Example 30 with the resultant compound from Example 30 and replacing Boc-His-OH with the resultant compound from Example 79 provided the desired compound, melting point 135-139 °C. Mass Spectrum: $(M+H)^+$ = 667.

## Example 144

(2R)-2-Benzyl-3-[N-(2-hydroxyethyl)-N-methyl]aminocarbonylpropionyl-His Amide of (2′S,1′R,5S)-3-Ethyl-5-(1′-hydroxy-2′-amino-3′-cyclohexylpropyl)oxazolidin-2-one.

Using the procedure of Example 30 with the resultant compound from Example 30 and replacing Boc-His-OH with the resultant compound from Example 81 provided the desired compound, melting point 120-125 °C. Mass Spectrum: $(M+H)^+$ = 655.

## Example 145

(2R)-2-Benzyl-3-methylaminocarbonylpropionyl-His Amide of (2′S,1′R,5S)-3-Ethyl-5-(1′-hydroxy-2′-amino-3′-cyclohexylpropyl)oxazolidin-2-one.

Using the procedure of Example 30 with the resultant compound from Example 30 and replacing Boc-His-OH with the resultant compound from Example 83 provided the desired compound, melting point 125-130 °C.
Anal. Calcd. for $C_{32}H_{46}N_6O_6 \bullet 0.75\ H_2O$:
C, 61.57; H, 7.67; N, 13.46.
Found:
C, 61.96; H, 7.65; N, 12.93.

## Example 146

(3,4-cis-Dihydroxypyrrolidinylcarbonyl)-(Me)Tyr-His Amide of (2′S,1′R,5S)-3-Ethyl-5-(1′-hydroxy-2′-amino-3′-cyclohexylpropyl)oxazolidin-2-one.

Using the procedure of Example 30 with the resultant compound from Example 30 and replacing Boc-His-OH with the resultant compound from Example 88 provided the desired compound, melting point 148-151 °C.
Anal. Calcd. for $C_{37}H_{55}N_7O_{11} \bullet H_2O \bullet HOAc$:
C, 56.12; H, 7.26; N, 12.38.
Found:
C, 55.98; H, 7.07; N, 12.43.

## Example 147

(2R)-2-Benzyl-3-(3,4-cis-dimethoxymethoxypyrrolidinocarbonyl)propionyl-His Amide of (2′S,1′R,5S)-3-Ethyl-5-(1′-hydroxy-2′-amino-3′-cyclohexylpropyl)oxazolidin-2-one.

Using the procedure of Example 30 with the resultant compound from Example 30 and replacing Boc-His-OH with the resultant compound from Example 96 provided the desired compound, melting point 88-91 °C.
Anal. Calcd. for $C_{39}H_{58}N_6O_{10}\bullet 0.3\ H_2O$:
C, 60.34: H, 7.61; N, 10.82.
Found:
C, 60.74; H, 7.78; N, 10.39.

## Example 148

(2R)-2-Benzyl-3-[(2-methoxyethoxymethoxyethyl)methylaminocarbonyl]propionyl-His Amide of (2′S, 1′R,5S)-3-Ethyl-5-(1′-hydroxy-2′-amino-3′-cyclohexylpropyl)oxazolidin-2-one.

Using the procedure of Example 30 with the resultant compound from Example 30 and replacing Boc-His-OH with the resultant compound from Example 97 provided the desired compound, melting point 57-60 °C.
Anal. Calcd. for $C_{38}H_{58}N_6O_9\bullet H_2O$:
C, 59.98; H, 7.95; N, 11.04.
Found:
C, 60.17; H, 7.94; N, 10.79.

## Example 149

N-Methyl-(2-methoxymethoxyethyl)aminocarbonyl-(Me)Tyr-His of (2′S,1′R,5S)-3-Ethyl-5-(1′-hydroxy-2′-amino-3′-cyclohexylpropyl)oxazolidin-2-one.

Using the procedure of Example 30 with the resultant compound from Example 30 and replacing Boc-His-OH with the resultant compound from Example 99 provided the desired compound, melting point 93-96 °C.
Anal. Calcd. for $C_{36}H_{55}N_7O_9\bullet 0.5\ H_2O$:
C, 58.52; H, 7.64; N, 13.27.
Found:
C, 58.45; H, 7.50; N, 13.11.

## Example 150

N-Butyl-(Me)Tyr-His Amide of (2′S,1′R,5S)-3-Ethyl-5-(1′-hydroxy-2′-amino-3′-cyclohexylpropyl) oxazolidin-2-one.

Using the procedure of Example 30 with the resultant compound from Example 30 and replacing Boc-His-OH with the resultant compound from Example 100 provided the desired compound.

Example 151

(2RS)-[[(4-morpholinyl)carbonyl]amino]-3,3-dimethyl-3-phenylpropionyl-His Amide of (2′S,1′R,5S)-3-Ethyl-5-(1′-hydroxy-2′-amino-3′-cyclohexylpropyl)oxazolidin-2-one.

Using the procedure of Example 30 with the resultant compound from Example 30 and replacing Boc-His-OH with the resultant compound from Example 103 provided the desired compound.

Example 152

Boc-Phe-His Amide of ((2′S,1′R,5S)-5-(1′-hydroxy-2′-amino-3′-cyclohexylpropyl)oxazolidin-2-one.

Using the procedure of Example 30 but replacing Boc-His-OH with Boc-Phe-His-OH gave the desired product, melting point 137-140° C.
Anal. Calcd. for $C_{34}H_{50}N_6O_7 \bullet 0.75\ H_2O$:
C, 61.11, H, 7.77; N, 12.57.
Found:
C, 61.11; H, 7.63; N, 12.21.

Example 153

Cbz-(α-Me)Pro-Phe-His Amide of (2′S,1′R,5S)-3-Ethyl-5-(1′-hydroxy-2′-amino-3′-cyclohexylpropyl)oxazolidin-2-one.

Using the procedure of Example 30 with the resultant compound from Example 152 and using Cbz-(α-Me)Pro-OH in place of Boc-His-OH provided the desired compound, melting point 125-130° C.
Anal. Calcd. for $C_{43}H_{57}N_7O_8 \bullet 2\ H_2O$:
C, 61.78; H, 7.35; N, 11.72.
Found:
C, 62.11; H, 7.21; N, 11.31.

Example 154

H-(α-Me)Pro-Phe-His Amide of (2′S,1′R,5S)-3-Ethyl-5-(1′-hydroxy-2′-amino3′-cyclopropyl)oxazolidin-2-one Acetic Acid Salt.

Using the procedure of Example 106 with the resultant compound from Example 153 gave the desired compound, melting point 88-91° C.
Anal. Calcd. for $C_{37}H_{55}N_7O_8 \bullet H_2O$:
C, 59.74; H, 7.72; N, 13.18.
Found:
C, 59.67; H,7.54; N, 13.21.

Example 155

Boc(1′-Napthyl)Ala-His Amide of (2′S,1′R,5S)-3-Ethyl-5-(1′-hydroxy-2′-amino-3′-cyclohexylpropyl) oxazolidin-2-one.

Using the procedure of Example 30 with the resultant compound from Example 30 and replacing Boc-His-OH with Boc-(1′-Napthyl)Ala-OH provided the desired compound.

Example 156

Dibenzylacetyl-His Amide of (2′S,1′R,5S)-3-Ethyl-5-(1′-hydroxy-2′-amino-3′-cyclohexylpropyl) oxazolidin-2-one.

Using the.procedure of Example 30 with the resultant compound from Example 30 and replacing BoC-His-OH with dibenzylacetic acid provided the desired compound.

Example 157

3-Phenylpropionyl-His Amide of (2′S,1′R,5S)-3-Ethyl-5-(1′-hydroxy-2′-amino-3′-cyclohexylpropyl) oxazolidin-2-one.

Using the procedure of Example 30 with the resultant compound from Example 30 and replacing Boc-His-OH with hydrocinnamic acid provided the desired compound, melting point 105-110 °C.
Anal. Calcd. for $C_{29}H_{41}N_5O_5 \bullet 0.25\ H_2O$:
C, 64.01; H, 7.69; N, 12.87.
Found:
C, 63.81; H, 7.70; N, 12.59.

Example 158

Boc-(Bn)Glu Amide of (2′S,1′R,5S)-3-Ethyl-5-(1′-hydroxy-2′-amino-3′-cyclohexylpropyl)oxazolidin-2-one.

Using the procedure of Example 31 and replacing Boc-Leu-OH with Boc-(Bn)Glu-OH afforded the desired compound.

Example 159

Morpholinocarbonyl-(Me)Tyr-(Bn)Glu Amide of (2'S,1'R,5S)-3-Ethyl-5-(1'-hydroxy-2'-amino-3'-cyclohexylpropyl)oxazolidin-2-one.

Using the procedure of Example 31 with the resultant compound from Example 158 and replacing Boc-Leu-OH with the resultant compound from Example 74 provided the desired product.

Example 160

Morpholinocarbonyl-(Me)Tyr-Glu Amide of (2'S,1'R,5S)-3-Ethyl-5-(1'-hydroxy-2'-amino-3'-cyclohexylpropyl)oxazolidin-2-one.

Using the procedure of Example 106 with the resultant compound from Example 159 gave the desired product.

Example 161

Boc-(Me)Cys Amide of (2'S,1'R,5S)-3-Ethyl-5-(1'-hydroxy-2'-amino-3'-cyclohexylpropyl)oxazolidin-2-one.

Using the procedure of Example 31 and replacing the Boc-Leu-OH with Boc-(Me)Cys-OH afforded the desired compound.

Example 162

Morpholinocarbonyl-(Me)Tyr-(Me)Cys Amide of (2'S,1'R,5S)-3-Ethyl-5-(1'-hydroxy-2'-amino-3'-cyclohexylpropyl)oxazolidin-2-one.

Using the procedure of Example 31 with the resultant compound from Example 161 and replacing Boc-Leu-OH with the resultant compound from Example 74 gave the desired product.

Example 163

(2R,4R,5S)-2-Isobutyl-4-hydroxy-5-tertbutyloxycarbonylamino-6-phenylhexanoic Acid Amide of (2'S,1'R,5S)-3-Ethyl-5-(1'-hydroxy-2'-amino-3'-cyclohexylpropyl)oxazolidin-2-one.

Using the procedure of Evans, et al. (J. Org. Chem. 1985, 50, 4615) with the resultant compound of Example 29 and (3R,5S,1'S)-5-(t-butyloxycarbonylamino)-2-phenylethyl)-3-isobutyldihydrofuran-2-(3H)-one (D.J. Kempf, J. Org. Chem. 1986, 51, 3921) gave the desired compound after purification by column chromatography using 3:2 ethyl acetate/hexane.

Example 164

(2R,4R,5S)-2-(4-Pentenyl)-4-hydroxy-5-tertbutyloxycarbonylamino-6-phenylhexanoic Acid Amide of (2′S,1′R,5S)-3-Ethyl-5-(1′-hydroxy-2′-amino-3′-cyclohexylpropyl)oxazolidin-2-one.

Using the procedure of Evans, et al. (J. Org. Chem. 1985, 50, 4615) with the resultant compound of Example 29 and (3R,5R,1′S)-5-(1-(t-butyloxycarbonylamino)-2-phenylethyl)-3-(4-pentenyl)dihydrofuran-2-(3H)-one (D.J. Kempf, J. Org. Chem. 1986, 51, 3921) gave the desired compound after purification by MPLC using 3:2 ethyl acetate/hexane.

Example 165

(Imidazol-4-yl)acetyl-Phe-His Amide of (2′S,1′R,5S)-3-Ethyl-5-(1′-hydroxy-2′-amino-3′-cyclohexylpropyl)-oxazolidin-

Using the procedure of Example 30 with the resultant compound from Example 152 and replacing Boc-His-OH with the (imidazol-4-yl)acetic acid provided the desired compound.

Example 166

H-Phe-His Amide of (2′S,1′R,5S)-3-Ethyl-5-(1′-hydroxy-2′-amino-3′-cyclohexylpropyl)oxazolidin-2-one.

Using the procedure of Example 29 with the resultant compound from Example 152 provided the desired product.

Example 167

(Imidazol-1-yl)acetyl-Phe-His Amide of (2′S,1′R,5S)-3-Ethyl-5-(1′-hydroxy-2′-amino-3′-cyclohexylpropyl)-oxazolidin-2-one.

The resultant compound of Example 166 (250 mg) in dry THF at 0°C was treated sequentially with 2 eq. N-methyl-morpholine and 1 equivalent bromoacetyl bromide. After 1 h, imidazole (5 eq) was added. The mixture was warmed to room temperature for 6 h and then evaporated. Chromatography of the residue on silica gel (dichloromethane/isopropyl amine/methanol, 89:9:2) provided the desired product.

Example 168

N-(2,3-dihydroxypropyl)Gly-Phe-His Amide of (2′S,1′R,5S)-3-Ethyl-5-(1′-hydroxy-2′-amino-3′-cyclohexylpropyl)oxazolidin-2-one.

Following the procedure of Example 167, but replacing imidazole with 1-amino-2,3-dihydroxypropane provided the desired product.

## Example 169

L-(4-Thiazolyl)alanine Amide of (2′S,1′R,5S)-3-ethyl-5-(1′-hydroxy-2′-amino-3′-cyclohexylpropyl) oxazolidin-2-one.

Using the procedure of Example 30 and replacing Boc-His-OH with Boc-DL-(4-thiazdyl)ala-OH afforded the intermediate Boc-protected compound. This material was stirred for 2 h in 4 M HCl/dioxane, the solvent was evaporated and the residue was dissolved in water which was basified with $K_2CO_3$. The product was extracted into chloroform which was dried and evaporated. Chromatography on silica gel with methanol/chloroform mixtures afforded the desired L-isomer (followed by the D-isomer). $^1H$ NMR ($CDCl_3$) δ 8.77 (D,1H), 8.10 (d,1H), 7.12 (d,1H), 3.92 (m,1H), 3.77 (m, 1H).

## Example 170

L-(3-Pyrazolyl)alanine Amide of (2′S,1′R,5S)-3-Ethyl-5-(1′-hydroxy-2′-amino-3′-cyclohexylpropyl) oxazolidin-2-one.

Using the procedure of Example 169 but replacing Boc-DL-(4-thiazolyl)ala-OH with Boc-DL-(3-pyrazolyl)ala-OH provided the desired product. $^1H$ NMR ($CDCl_3$, TMS) δ 8.00 (d,1H), 7.49 (d,1H), 6.17 (d,1H), 4.11 (m,1H).

## Example 171

Boc-L-(2-Thienyl)alanine Amide of (2′S,1′R,5S)-3-ethyl-5-(1′-hydroxy-2′-amino-3′-cyclohexylpropyl) oxazolidin-2-one.

Using the procedure of Example 30 but replacing Boc-His-OH with Boc-L-(2-thienyl)ala-OH afforded the desired product. $^1H$ NMR ($CDCl_3$) δ 7.20 (dd,1H), 6.96 (dd,1H), 6.88 (dd,1H), 6.63 (d,1H), 4.98 (d,1H), 4.58 (m,1H), 4.38 (m,1H), 4.18 (m,1H), 1.44 (s,9H), 1.17 (t,3H).

## Example 172

Boc-L-(1-Pyrazolyl)alanine.

Pyrazole (700 mg, 10.3 mmol) and N-(tert-Butyloxycarbonyl)-L-serine-β-lactone (1.707 g, 9.117 mmol, Arnold et al., J. Am. Chem. Soc. 1985, 107, 7105) in $CH_3CN$ (75 ml) were heated at 52° C for 72 h. The solvent was evaporated and the residue was dissolved in hot methanol (8 ml) and then water (24 ml) was added with heating until the mixture became turbid. The mixture was cooled to room temperature with rapid stirring, and after stirring overnight 745 mg (32%) of the desired product was collected as a white solid. $^1H$ NMR ($CDCl_3$) δ 7.65 (d,1H), 7.41 (d,1H), 6.30 (dd,1H), 5.48 (br,1H), 4.82 (dd,1H), 4.67 (dd,1H), 4.48 (m,1H), 1.47 (s,9H).

Example 173

Boc-L-(1-Imidazolyl)alanine Methyl Ester.

Imidazole (250 mg, 3.67 mmol) and N-(tert-Butyloxycarbonyl)-L-serine-β-lactone (350.0 mg, 1.87 mmol, Arnold et al., J. Am. Chem. Soc. 1985, 107, 7105) in $CH_3CN$ (9 ml) were stirred at room temperature for 24 h. The mixture was cooled to 0° C, treated with diazomethane in ether, evaporated, and chromatographed on silica gel with 3% methanol in chloroform to afford 305 mg (61%) of the desired product as an oil. $^1H$ NMR ($CDCl_3$) δ 7.39 (s,1H), 7.05 (s,1H), 6.82 (s,1H), 5.18 (br,1H), 4.58 (m,1H), 4.42 (m,2H), 3.79 (s,3H), 1.47 (s,9H).

Example 174

Boc-L-(1-Imidazolyl)alanine.

The resultant compound from Example 173 (301.0 mg, 1.12 mmol) in dioxane (6 ml) at 0° C was treated with $LiOH•H_2O$ (64.0 mg, 1.53 mmol) in water (4 ml). After 1 h the reaction was quenched with 2.0 M HCl (0.75 ml, 1.50 mmol) and evaporated to a white foam which was used without further purification.

Example 175

Boc-L-β-Azidoalanine Methyl Ester (A) and Boc-Dehydroalanine Methyl Ester (B).

To sodium azide (1.62 g, 24.9 mmol) in water (2.0 ml) was added benzene (25 ml) and the mixture was cooled to 0° C. Concentrated sulfuric acid (0.70 ml, 12.6 mmol) was added dropwise with rapid stirring. The solution of $HN_3$ was transferred via cannula to a flask charged with anhydrous $Na_2SO_4$ at 0° C and allowed to stand for 1 h. To triphenylphosphine (5.75 g, 21.9 mmol) in tetrahydrofuran (20 ml, THF) at -78° C was added diethylazodicarboxylate (3.40 ml, 21.6 mmol) in THF (10 ml) followed by the $HN_3$ solution and Boc-serine methyl ester (4.00 g, 18.2 mmol) in THF (16 ml). After stirring at -78° C 1 h and at room temperature overnight the solvent was evaporated and the residue was chromatographed on silica gel with 2:1 hexane/ethane to provide 0.33 g (9%) of product B followed by 3.25 g (73%) of product A, both as mobile oils.

A: $^1H$ NMR ($CDCl_3$) δ 5.38 (br,1H), 4.48 (m,1H), 3.80 (s,3H), 3.72 (d,2H), 1.47 (s,9H).

B: $^1H$ NMR ($CDCl_3$) δ 7.02 (br,1H), 6.17 (s,1H), 5.73 (d,1H), 3.83 (s,3H), 1.49 (s,9H).

Example 176

Boc-L-β-Azidoalanine.

The resultant compound from Example 175 A (350.0 mg, 1.433 mmol) in dioxane (6 ml) at 0°C was treated with $LiOH \cdot H_2O$ (84.0 mg, 2.00 mmol) in water (4 ml). After 1 h the mixture was concentrated, diluted with water, washed with ether, acidified with 0.5 M $H_3PO_4$, and extracted into chloroform which was dried over $Na_2SO_4$ and evaporated to provide 250.4 mg (76%) of a colorless oil. $^1H$ NMR ($CDCl_3$) δ 5.37 (br,1H), 4.52 (m,1H), 3.80 (m,2H), 1.47 (s,9H).

Example 177

Boc-DL-β-(4-Morpholinyl)alanine Methyl Ester.

Morpholine (0.12 ml, 1.38 mmol) and the resultant compound from Example 175 B (258.1 mg, 1.28 mmol) in $CH_3CN$ (6 ml) were heated at 60°C for 18 h. The solvent was evaporated and the residue was chromatographed on silica gel with 1:1 ethyl acetate/hexane to provide 288.6 mg (78%) of the product as a colorless oil. $^1H$ NMR ($CDCl_3$) δ 5.32 (br,1H), 4.34 (m,1H), 3.75 (s,3H), 3.68 (m,4H), 2.68 (m,2H), 2.55-2.38 (m,4H), 1.46 (s,9H).

Example 178

Boc-DL-β-(4-Morpholinyl)alanine.

Using the procedure of Example 174 with the resultant compound from Example 177 provided the desired product as a foam.

Example 179

Boc-DL-β-(N-Methyl-N-methoxylamino)alanine Methyl Ester.

N,O-Dimethylhydroxylamine hydrochloride (600 mg, 6.16 mmol), $NaHCO_3$ (560 mg, 6.6 mmol), and the resultant product from Example 175 B (570.5 mg, 2.84 mmol) in $CH_3CN$ (14 ml) were heated at 100°C for 65 h. The mixture was filtered and evaporated and the residue was chromatographed on silica gel with 20% ethyl acetate in hexane to afford 112.1 mg (15%) of the desired product as an oil. $^1H$ NMR ($CDCl_3$) δ 5.50 (br,1H), 4.38 (m,1H), 3.72 (s,3H), 3.39 (s,3H), 3.11 (dd,1H), 2.88 (dd,1H), 2.57 (s,3H), 1.46 (s,9H).

Example 180

Boc-DL-β-(N-Methyl-N-methoxylamino)alanine.

Using the procedure of Example 174 with the resultant compound from Example 179 provided the desired product as a foam. $^1$H NMR ($CD_3OD$) δ 4.28 (m,1H), 3.44 (s,3H), 2.95 (m,2H), 2.58 (s,3H), 1.45 (s,9H).

Example 181

Boc-DL-N-Methyl-(4-thiazolyl)alanine.

To Boc-DL-(4-thiazolyl)alanine (275.0 mg, 1.01 mmol) in tetrahydrofuran (3 ml) at 0 °C was added $CH_3I$ (0.50 ml, 8.03 mmol) and NaH (130.0 mg, 3.25 mmol, 60% in oil). After stirring at room temperature for 24 h the mixture was diluted with ethyl acetate (5 ml), quenched with water (1 ml), and evaporated. The residue was partitioned between ether and water, and the ether was washed with saturated $NaHCO_3$ solution. The combined aqueous phases were acidified to pH 3 with 0.5 M $H_3PO_4$ and extracted with ethyl acetate which was dried over $Na_2SO_4$ and evaporated to afford 290.0 mg (100%) of a brittle oil. $^1$H NMR ($CDCl_3$) δ 8.82 (s,1H), 7.12, 7.08 (2s,total 1H), 4.95,4.74 (2m,total 1H), 3.60-3.20 (m,2H), 2.81 (s,3H), 1.43, 1.40 (2s,total 9H).

Example 182

L-N-Methyl-(4-thiazolyl)alanine Amide of (2′S,1′R,5S)-3-ethyl-5-(1′-hydroxy-2′-amino-3′-cyclohexylpropyl)-oxazolidin-2-one.

Using the procedure of Example 169 but replacing Boc-DL-(4-thiazolyl)ala-OH with the resultant compound from Example 181 gave the desired product.

Example 183

Boc-l-(1-Imidazolyl)alanine Amide of (2′S,1′R,5S)-3-ethyl-5-(1′-hydroxy-2′-amino-3′-cyclohexylpropyl)oxazolidin-2-one.

Using the procedure of Example 30 but replacing Boc-His-OH with the resultant compound from Example 174 gave the desired product.

Example 184

Boc-L-(1-Pyrazolyl)alanine Amide of (2′S,1′R,5S)-3-ethyl-5-(1′-hydroxy-2′-amino-3′-cyclohexylpropyl) oxazolidin-2-one.

Using the procedure of Example 30 but replacing Boc-His-OH with the resultant compound from Example 172 gave the desired product.

Example 185

Boc-L-β-Azidoalanine Amide of (2′S,1′R,5S)-3-ethyl-5-(1′-hydroxy-2′-amino-3′-cyclohexylpropyl) oxazolidin-2-one.

Using the procedure of Example 30 but replacing Boc-His-OH with the resultant compound from Example 176 gave the desired product.

Example 186

L-β-(4-Morpholinyl)alanine Amide of (2′S,1′R,5S)-3-ethyl-5-(1′-hydroxy-2′-amino-3′-cyclohexylpropyl) oxazolidin-2-one.

Using the procedure of Example 169 but replacing Boc-DL-(4-thiazolyl)ala-OH with the resultant compound from Example 178 and using 1:1 trifluoracetic acid/$CH_2Cl_2$ at 0° C for amine deprotection instead of 4 M HCl/dioxane afforded the desired product.

Example 187

L-β-(N-Methyl-N-methoxylamino)alanine Amide of (2′S,1′R,5S)-3-ethyl-5-(1′-hydroxy-2′-amino-3′-cyclohexylpropyl)oxazolidin-2-one.

Using the procedure of Example 186 but replacing the resultant compound from Example 178 with the resultant compound from Example 180 gave the desired product.

Example 188

Benzyl (2R)-2-Benzyl-3-(4-trifluoroethylpiperazin-1-ylcarbonyl)propionate.

The resultant acid from Example 43 (0.500 g, 1.68 mmol) in $CH_2Cl_2$ (7 ml) at -10° C was treated with N-methylmorpholine (0.20 ml, 1.82 mmol) and then isobutyl chloroformate (0.22 ml, 1.68 mmol). After 5 min 1-trifluoroethylpiperazine (0.30 g, 1.78 mmol) was added and the mixture was stirred at -10° C for 15 min and then at room temperature for 2 h. The solvent was evaporated and the residue was taken up in ethyl acetate

which was washed with saturated $NaHCO_3$ solution, water and brine, and then dried over $Na_2SO_4$ and evaporated. Chromatography of the residue on silica gel with 20-33% ethyl acetate in hexane provided 0.61 g (81%) of an oil. $^1H$ NMR ($CDCl_3$) $\delta$ 7.10-7.40 (m,10H), 5.15 (d,1H), 5.05 (d,1H), 3.25-3.70 (m,5H), 3.04 (dd,1H), 2.97 (q,2H), 2.81 (dd,1H), 2.72 (dd,1H), 2.60 (m,4H), 2.32 (dd,1H).

## Example 189

### (2R)-2-Benzyl-3-(4-trifluoroethylpiperazin-1-ylcarbonyl)propionic Acid.

The resultant compound from Example 188 (610 mg), and 10% palladium on carbon (300 mg) in methanol were stirred under an $H_2$ atmosphere for 2 h. Filtration and solvent evaporation afforded 470 mg (96%) of a solid, m.p. 96-98° C.

## Example 190

### Benzyl (2R)-2-Benzyl-3-(4-methylpiperazin-1-ylcarbonyl)propionate.

Using the procedure of Example 188 but replacing 1-trifluoroethylpiperazine with 1-methylpiperazine provided the desired product as an oil. $^1H$ NMR ($CDCl_3$) $\delta$ 7.10-7.40 (m,10H), 5.16 (d,1H), 5.05 (d,1H), 3.25-3.70 (m,5H), 3.03 (dd,1H), 2.82 (dd,1H), 2.72 (dd,1H), 2.35 (m,5H), 2.28 (s,3H).

## Example 191

### (2R)-2-Benzyl-3-(4-methylpiperazin-1-ylcarbonyl)propionic Acid.

Prepared from the resultant compound of Example 190 according to the procedure of Example 189. $^1H$ NMR ($CDCl_3$) $\delta$ 7.15-7.35 (m,6H), 2-50-2.80 (m,4H), 2.47 (s,3H), 2.30 (dd,1H).

## Example 192

### Benzyl (2R)-2-Benzyl-3-((2-pyridin-2-ylethyl)methylaminocarbonyl)propionate.

Using the procedure of Example 188 but replacing 1-trifluoroethylpiperazine with 2-(2-methylaminoethyl)pyridine provided the desired product as an oil. $^1H$ NMR ($CDCl_3$) $\delta$ 8.48 (m,1H), 7.57 (m,1H), 6.95-7.40 (m,12H), 5.00-5.20 (m,2H), 2.87, 2.82 (2s,total 3H), 2.31, 2.18 (2dd,total 1H).

## Example 193

(2R)-2-Benzyl-3-((2-pyridin-2-ylethyl)methylaminocarbonyl)propionic Acid.

Prepared from the resultant compound of Example 192 according to the procedure of Example 189. $^1$H NMR ($CDCl_3$) δ 8.49 (m,1H), 7.58 (m,1H), 6.95-7.32 (m,7H), 2.87, 2.72 (2s,total 3H).

Example 194

Benzyl (2R)-2-Benzyl-3-((N-pyridin-4-yl)methylaminocarbonyl)propionate.

Using the procedure of Example 188 but replacing 1-trifluoroethylpiperazine with 4-methylaminopyridine provided the desired product. $^1$H NMR ($CDCl_3$) δ 8.6 (m,2H), 7.4-7.0 (m,12H), 5.1 (q,2H), 3.3 (m,1H), 3.2 (s,3H), 3.0 (dd,1H), 2.7 (dd,1H), 2.6 (dd,1H), 2.25 (dd,1H).

Example 195

(2R)-2-Benzyl-3-((N-pyridin-4-yl)methylaminocarbonyl)propionic Acid.

Prepared from the resultant compound of Example 194 according to the procedure of Example 189, m.p. 88-92°C.

Example 196

Benzyl (2S)-2-(4-morpolinyl)-3-phenylpropionate.

2,5-Dihydrofuran (0.78 ml, 10.3 mmol) in methanol (4 ml) and $CH_2Cl_2$ (16 ml) at -60°C was treated with ozone until a blue color persisted, and the excess ozone was removed under a stream of $N_2$. To this solution was added $NaCNBH_3$ (456 mg, 7.26 mmol). After 15 min at -60°C, H-Phe-OBn p-toluene-sulfonic acid salt (2.22 g, 5.19 mmol) in methanol (20 ml) was added over 5 min, and the mixture was stirred at -60°C for 15 min and at 0°C for 20 h. The mixture was quenched with acetic acid (0.30 ml, 5.2 mmol), stirred at 0°C for 30 min and the solvent was evaporated. The residue was taken up in saturated $NaHCO_3$ solution and extracted into $CH_2Cl_2$ which was dried over $Na_2SO_4$ and evaporated. Chromatography of the residue on silica gel with 20% ethyl acetate in hexane afforded 1.374 g (81%) of an oil. $^1$H NMR ($CDCl_3$) δ 7.10-7.35 (m,10H), 5.03 (s,2H), 3.62-3.75 (m,4H), 3.48 (dd,1H), 3.08 (dd,1H), 2.97 (dd,1H), 2.58-2.80 (m, 4H).

Example 197

(2S)-2-(4-Morpholinyl)-3-phenylpropionic Acid.

Prepared from the resultant compound of Example 196 according to the procedure of Example 189. $^1$H NMR ($CDCl_3$) δ 7.15-7.35 (m,5H), 3.78 (m,4H), 3.57 (m,1H), 3.12 (m,2H), 3.03 (m,4H).

Example 198

Benzyl (2R)-2-Benzyl-3-chloromethylcarbonylpropionate.

The resultant acid from Example 43 (500 mg, 1.68 mmol) in $CH_2Cl_2$ (8 ml) at 0° C was treated with oxalyl chloride (0.160 ml, 1.83 mmol) and dimethylformamide (0.0065 ml). After 2 h at 0° C, the solvent was evaporated and the residue was dissolved in ether (6 ml), cooled to 0° C and treated with an ether solution of $CH_2N_2$. After 2 h at 0° C the solvent was evaporated and the residue was dissolved in ether (6 ml), cooled to -10° C, and treated with 4.0 M HCl/dioxane (0.6 ml, 2.4 mmol). After 1 h the solvent was evaporated and the residue was chromatographed on silica gel with 10% ethyl acetate in hexane to afford 476.8 mg (83%) of a colorless oil. $^1H$ NMR ($CDCl_3$) δ 7.08-7.40 (m,10H), 5.12 (d,1H), 5.08 (d,1H), 4.02 (s,2H), 3.30 (m,1H), 3.10 (dd,1H), 2.97 (dd,1H), 2.78 (dd,1H), 2.55 (dd,1H).

Example 199

Benzyl (2R)-2-Benzyl-3-thiazol-4-ylpropionate.

The resultant compound from Example 198 (476.8 mg, 1.44 mmol) and thioformamide (176 mg, 2.88 mmol) in acetone (6 ml) were stirred at room temperature for 108 h. N-methylmorpholine (0.16 ml, 1.40 mmol) was added and after 20 min the mixture was diluted with ether, filtered, evaporated, and chromatographed on silica gel with 20% ethyl acetate in hexane to afford 369 mg (76%) of an oil. $^1H$ NMR ($CDCl_3$) δ 8.70 (d,1H), 7.05-7.35 (m,10H), 6.90 (d,1H), 5.00 (d,1H), 4.95 (d, 1H), 3.28-3.35 (m,1H), 3.19 (dd,1H), 2.95-3.10 (m,2H), 2.88 (dd,1H).

Example 200

(2R)-2-Benzyl-3-thiazol-4-ylpropionic Acid.

The resultant compound from Example 199 (364 mg) was stirred for ¼ h in 30% HBr in acetic acid (5 ml). The solvent was evaporated and the residue was dissolved in 1 M HCl and washed with water. The aqueous phase was adjusted to pH 4 with solid $NaHCO_3$ and extracted with chloroform which was dried over $Na_2SO_4$ and evaporated to afford 186.5 mg (70%) of an oil. $^1H$ NMR ($CDCl_3$) δ 8.78 (d,1H), 7.15-7.35 (m,5H), 6.99 (d,1H), 3.00-3.30 (m,4H), 2.81 (dd,1H).

Example 201

Benzyl (2R)-2-Benzyl-5-tert-butylmercapto-4-oxopentanoate.

To tert-butylmercaptan (0.11 ml) in dimethylformamide (5 ml) at 0° C was added potassium bis-(trimethylsilyl)amide in toluene (1.80 ml, 0.90 mmol, 0.5 M) followed by the resultant compound from Example 198 (259.6 mg, 0.785 mmol) in dimethylformamide (3 ml). After 16 h at room temperature the

mixture was diluted with ethyl acetate, washed with water and brine, then dried over $Na_2SO_4$ and evaporated. Chromatography of the residue on silica gel with 10% ethyl acetate in hexane afforded 219.6 mg (73%) of a colorless oil. $^1H$ NMR ($CDCl_3$) δ 7.10-7.40 (m,10H), 5.07 (s,2H), 3.25 (s,2H), 3.18-3.29 (m,1H), 2.97-3.07 (m,2H), 2.78 (dd,1H), 2.71 (dd,1H), 1.25 (s,9H).

## Example 202

### Benzyl (2R)-2-Benzyl-5-tert-butylsulfinyl-4-oxopentanoate.

The resultant compound from Example 201 (44.4 mg, 0.115 mmol) in $CH_2Cl_2$ (2 ml) at -10°C was treated with metachloroperbenzoic acid (25.0 mg, 0.116 mmol, 80% pure). After 2 h at -10°-0°C the solvent was evaporated and the residue was dissolved in ethyl acetate which was washed with 1:1 10% $Na_2SO_3$ solution/saturated $NaHCO_3$ solution, saturated $NaHCO_3$ solution and brine, and then dried over $Na_2SO_4$ and evaporated to afford 46.0 mg (99%) of a colorless oil. $^1H$ NMR ($CDCl_3$) δ 7.05-7.40 (m,10H), 5.08 (m,2H), 3.36-3.53 (m,2H), 3.30 (m,1H), 2.95-3.18 (m,2H), 2.80 (2dd,total 1H), 2.69 (2dd,total 1H), 1.24 (s,9H).

## Example 203

### Benzyl (2R)-2-Benzyl-5-tert-butylsulfonyl-4-oxopentanoate.

The resultant compound from Example 201 (171.9 mg, 0.447 mmol) in $CH_2Cl_2$ (5 ml) was treated with meta-chloroperbenzoic acid (290 mg, 1.34 mmol, 80% pure). After 75 min at room temperature the product was isolated as described in Example 202 to afford 184.0 mg (59%) of a colorless oil. $^1H$ NMR ($CDCl_3$) δ 7.08-7.35 (m,10H), 5.07 (s,2H), 3.98 (d,1H), 3.88 (d,1H), 3.23-3.33 (m,1H), 3.18 (dd,1H), 3.03 (dd,1H), 2.88 (dd,1H), 2.82 (dd,1H), 1.38 (s,9H).

## Example 204

### (2R)-2-Benzyl-5-tert-butylsulfinyl-4-oxopentanoic Acid.

Prepared from the resultant compound from Example 202 according to the procedure of Example 189. $^1H$ NMR ($CDCl_3$) δ 7.15-7.35 (m,5H), 1.23 (s,9H).

## Example 205

### (2R)-2-Benzyl-5-tert-butylsulfonyl-4-oxopentanoic Acid.

Prepared from the resultant compound from Example 203 according to the procedure of Example 189. $^1H$ NMR ($CDCl_3$) δ 7.15-7.35 (m,5H), 3.94 (d,1H), 3.88 (d,1H), 2.90-3.30 (m,3H), 2.70-2.85 (m,2H), 1.39 (s,9H).

Example 206

Benzyl (2R)-2-Benzyl-5-morpholin-4-yl-4-oxopentanoate.

The resultant compound from Example 198 (610 mg, 1.84 mmol) in dimethylformamide (10 ml) was treated with NaI (33 mg, 0.33 mmol) and morpholine (0.60 ml, 6.88 mmol). After 2 h the mixture was diluted with ethyl acetate, washed with water and brine, and then dried over $Na_2SO_4$, and evaporated. Chromatography of the residue on silica gel with 60% ethyl acetate/40% hexane afforded 460 mg (65%) of an oil. $^1H$ NMR ($CDCl_3$) $\delta$ 7.05-7.40 (m,10H), 5.11 (d,1H), 5.06 (d,1H), 3.68 (m,4H), 3.22-3.32 (m,1H), 3.15 (d,1H), 3.08 (d,1H), 3.05 (m,1H), 2.87 (dd,1H), 2.77 (dd,1H), 2.35-2.50 (m,5H).

Example 207

(2R)-2-Benzyl-5-morpholin-4-yl-4-oxopentanoic Acid.

Prepared from the resultant compound from Example 206 according to the procedure of Example 189. $^1H$ NMR ($CDCl_3$) $\delta$ 7.15-7.35 (m,5H), 3.60-3.75 (m,4H).

Example 208

Methyl $\alpha$-Benzylacrylate.

$\alpha$-Benzylacrylic acid (1.00 g, 6.17 mmol) in methanol (20 ml) was treated with $BF_3 \bullet Et_2O$ (2 ml). The mixture was heated to reflux for 14 h, cooled, and poured into saturated $NaHCO_3$ solution. Extraction with ether followed by drying over $Na_2SO_4$ and evaporation afforded 1.03 g (95%) of a mobile oil. $^1H$ NMR ($CDCl_3$) $\delta$ 7.17-7.35 (m,5H), 6.23 (m,1H), 5.47 (m,1H), 3.74 (s,3H), 3.63 (s,2H).

Example 209

Methyl (2RS)-2-benzyl-3-(N-methoxyl-N-methylamino)propionate.

The resultant compound from Example 208 (800 mg, 4.54 mmol), N-methyl,O-methylhydroxylamine hydrochloride (0.57 g, 5.4 mmol), and $NaHCO_3$ (0.46 g, 5.48 mmol) in dimethylsulfoxide (5 ml) were heated at 130 °C for 20 h. The mixture was diluted with ethyl acetate, washed with water, saturated $NaHCO_3$ solution and brine, and then was dried over $Na_2SO_3$ and evaporated. Chromatography of the residue on silica gel with 10% ethyl acetate in hexane afforded 226 mg (21%) of a mobile oil. $^1H$ NMR ($CDCl_3$) $\delta$ 7.10-7.30 (m,5H), 3.60 (s,3H), 3.47 (s,3H), 2.80-3.10 (m,4H), 2.60 (dd,1H), 2.55 (s,3H).

Example 210

Methyl (2RS)-2-benzyl-pyrazol-1-ylpropionate.

Using the procedure of Example 209 but replacing N-methyl,O-methylhydroxylamine hydrochloride and $NaHCO_3$ with pyrazole provided the desired product as an oil. $^1H$ NMR ($CDCl_3$) δ 7.52 (d,1H), 7.10-7.35 (m,6H), 6.10 (dd,1H), 4.38 (dd,1H), 4.24 (dd,1H), 3.57 (s,3H), 3.37 (m,1H), 2.98 (dd,1H), 2.82 (dd,1H).

Example 211

(2RS)-2-Benzyl-3-pyrazol-1-ylpropionic Acid.

The resultant compound from Example 210 (100.0 mg, 0.409 mmol) in dioxane (2 ml) at 0°C was treated with $LiOH \bullet H_2O$ (22.0 mg, 0.524 mmol) in water (1 ml). After 1 h at 0°C and 30 min at room temperature the solvent was evaporated and the residue was taken up in water, the pH was adjusted to pH 3-4, and the mixture was extracted with $CHCl_3$ which was dried over $Na_2SO_4$ and evaporated to afford 96 mg (100%) of a solid. $^1H$ NMR ($CDCl_3$) δ 7.56 (d,1H), 7.10-7.35 (m,6H), 6.26 (dd,1H), 4.30 (m,2H), 3.34 (m,1H), 3.12 (dd,1H), 2.72 (dd,1H).

Example 212

(2RS)-2-Benzyl-3-(N-methoxyl-N-methylamino)propionic Acid.

Using the procedure of Example 211 with the resultant compound from Example 209 gave the desired product. $^1H$ NMR ($CDCl_3$) δ 7.10-7.35 (m,5H), 3.58 (s,3H), 2.62 (s,3H).

Example 213

Methyl (2RS)-2-benzyl-3-tert-butylmercaptopropionate.

To sodium (3.05 g, 133 mmol) in methanol (135 ml) was added tert-butylmercaptan (17.0 ml, 151 mmol). After 20 min methyl α-benzylacrylate (17.05 g, 96.8 mmol) in methanol (100 ml) was added and after 1 h at room temperature the mixture was heated at reflux for 17 h. After cooling, the mixture was acidified with 2 M HCl (70 ml), concentrated, taken up in ether, washed with water and brine, then dried over $MgSO_4$ and evaporated to 23.59 g (92%) of an oil. $^1H$ NMR ($CDCl_3$) δ 7.15-7.35 (m,5H), 3.63 (s,3H), 2.60-3.05 (m,5H), 1.28 (s,9H).

Example 214

Methyl (2RS)-2-benzyl-3-tert-butylsulfonylpropionate.

To the resultant compound from Example 213 (270 mg, 1.01 mmol) in methanol (6 ml) and water (5 ml) at 0 ° C was added potassium peroxymonosulfate (1.845 g, 6 mmol) in portions. After 15 min at 0 ° C and 24 h at room temperature the mixture was filtered, diluted with water, and extracted with $CH_2Cl_2$ which was washed with brine, dried over $MgSO_4$, and evaporated to 300 mg (99%) of an oil. $^1H$ NMR δ 7.15-7.35 (m,5H), 3.68 (s,3H), 3.45 (m,2H), 3.12 (dd,1H), 2.98 (m,2H), 1.37 (s,9H).

## Example 215

(2RS)-2-Benzyl-3-tert-butylsulfonylpropionic Acid.

The resultant compound from Example 214 (282 mg, 0.95 mmol) in 6 M HCl (2 ml) and acetic acid (0.4 ml) was heated at reflux for 16 h. The mixture was cooled and filtered and the resulting solid was recrystallized from methycyclohexane/ethyl acetate to afford 152 mg (56%) of the desired product, m.p. 147-148 ° C.

## Example 216

(2S)-2-Benzyl-3-tert-butylsulfonylpropionic Acid Amide of Histidine Benzyl Ester.

Using the carbodiimide coupling procedure of Example 30 with the resultant acid from Example 215 and histidine benzyl ester di-p-toluene sulfonic acid salt gave after workup the crude (2RS) amides. Chromatography on silica gel with 1.5-2% $CH_3OH$ in $CHCl_3$ afforded 704 mg of the (2S)-isomer followed by 1.07 g of the (2R)-isomer.
(2S)-isomer: $^1H$ NMR ($CDCl_3$) δ 7.48 (d,1H), 7.10-7.45 (m,11H), 6.52 (s,1H), 6.40 (br,1H), 5.12 (d,1H), 5.07 (d,1H), 4.70 (m,1H), 3.78 (dd,1H), 3.10-3.25 (m,3H), 3.02 (dd,1H), 2.91 (dd,1H), 2.82 (dd,1H), 1.40 (s,9H).
(2R)-isomer: $^1H$ NMR ($CDCl_3$) δ 7.37 (s,1H), 7.20-7.40 (m,11H), 7.04 (br,1H), 6.43 (s,1H), 5.15 (d,1H), 5.07 (d.1H), 4.59 (m,1H), 3.48 (dd,1H), 3.23 (m,1H), 2.98-3.10 (m,4H), 2.83 (dd,1H), 1.38 (s,9H).

## Example 217

(2S)-2-Benzyl-3-tert-butylsulfonylpropionic Acid Amide of (imidazole-Boc)Histidine Benzyl Ester.

To the resultant (2S)-isomer from Example 216 (700 mg, 1.37 mmol) in $CH_2Cl_2$ (4.6 ml) was added di-tert-butyl-dicarbonate (314 mg, 1.44 mmol). After 14 h the solvent was evaporated to afford the desired product. $^1H$ NMR ($CDCl_3$) δ 7.92 (d,1H), 7.15-7.40 (m,10H), 7.10 (d,1H), 7.06 (br,1H), 5.12 (d,1H), 5.08 (d,1H), 4.83 (m,1H), 3.58 (dd,1H), 3.22 (m,1H), 2.85-3.15 (m,5H), 1.60 (s,9H), 1.34 (s,9H).

## Example 218

(2S)-2-Benzyl-3-tert-butylsulfonylpropionic Acid Amide of (imidazole-Boc)Histidine.

Using the procedure of Example 189 with the resultant product from Example 217 provided the desired product. $^1H$ NMR ($CDCl_3$) $\delta$ 8.13 (d,1H), 7.58 (s,1H), 7.15-7.35 (m,5H), 6.47 (br,1H), 4.62 (m,1H), 3.69 (dd,1H), 3.00-3.27 (m,4H), 2.28-2.42 (m,2H), 1.60 (s,9H), 1.37 (s,9H).

Example 219

Isonicitonyl-(O-methyl)tyrosine Benzyl Ester.

To (O-methyl)tyrosine benzyl ester hydrochloride salt (1.07 mmol) in $CH_2Cl_2$ (10 ml) was added isonicitonyl chloride hydrochloride (200 mg, 1.12 mmol) and N-methylmorpholine (0.42 ml, 3.82 mmol). After 3 h the mixture was concentrated, taken up in ethyl acetate, washed with saturated $NaHCO_3$ solution, water and brine, and then dried over $Na_2SO_4$ and evaporated. Chromatography of the residue on silica gel with 1:1 ethyl acetate/hexane afforded 132.5 mg (32%) of a solid. $^1H$ NMR ($CDCl_3$) $\delta$ 8.73 (m,2H), 7.54 (m,2H), 7.33-7.43 (m,5H), 6.89 (m,2H), 6.74 (m,2H), 6.61 (d,1H), 5.26 (d,1H), 5.17 (d,1H), 5.07 (m,1H), 3.77 (s,3H), 3.20 (m,2H).

Example 220

Isonicitonyl-(O-methyl)tyrosine.

Using the procedure of Example 189 with the resultant compound from Example 219 gave the desired product. $^1H$ NMR ($CD_3OD$) $\delta$ 8.65 (d,2H), 7.68 (dd,2H), 7.18 (m,2H), 6.82 (m,2H), 4.77 (dd,1H), 3.73 (s,3H), 3.32 (m,2H).

Example 221

1-Methylisonipicotonyl-(O-methyl)tyrosine Benzyl Ester.

Using the carbodiimide coupling procedure of Example 30 with 1-methylisonipicotic acid and (O-methyl)tyrosine benzyl ester hydrochloride salt gave the desired product after chromatography on silica gel with 4-6% methanol in chloroform. $^1H$ NMR ($CDCl_3$) $\delta$ 7.30-7.43 (m,5H), 6.87 (m,2H), 6.73 (m,2H), 5.89 (d,1H), 5.20 (d,1H), 5.12 (d,1H), 4.89 (m,1H), 3.77 (s,3H), 3.07 (m,2H), 2.87 (m,2H), 2.28 (s,3H), 1.65-2.15 (m,7H).

Example 222

1-Methylisonipicotonyl-(O-methyl)tyrosine.

Using the procedure of Example 189 with the resultant compound from Example 221 provided the desired product as a waxy solid. $^1$H NMR ($CD_3OD$) δ 7.12 (m,2H), 6.81 (m,2H), 4.55 (dd,1H), 3.74 (s,3H), 2.82 (s,3H).

Example 223

1-Methylpiperazin-4-ylcarbonyl-(O-methyl)tyrosine Methyl Ester.

To α-isocyanato-L-(O-methyl)tyrosine methyl ester (0.250 g) in $CH_2Cl_2$ (5 ml) was added 1-methylpiperazine (0.12 ml, 1.08 mmol). After 2 h the solvent was evaporated and the residue was chromatographed on silica gel with 2% methanol in chloroform to afford the desired product as a solid. $^1$H NMR ($CDCl_3$) δ 7.02 (m,2H), 6.83 (m,2H), 4.84 (d,1H), 4.76 (m,1H), 3.79 (s,3H), 3.73 (s,3H), 3.37 (m,4H), 3.07 (m,2H), 2.37 (m,4H), 2.31 (s,3H).

Example 224

1-Methylpiperazin-4-ylcarbonyl-(O-methyl)tyrosine.

To the resultant compound from Example 223 (375.0 mg, 1.12 mmol) in dioxane (6 ml) at 0°C was added $LiOH \bullet H_2O$ (56.3 mg, 1.34 mmol) in $H_2O$ (3 ml). After 70 min the reaction was quenched with 2 M HCl (0.67 ml, 1.34 mmol), concentrated, diluted with brine, and extracted into chloroform which was dried over $Na_2SO_4$ and evaporated. The residue was suspended in ethanol, filtered, and the filtrate was evaporated to afford 344.8 mg (96%) of a white solid. $^1$H NMR ($CD_3OD$) δ 7.13 (m,2H), 6.81 (m,2H), 4.42 (m,1H), 3.75 (s,3H), 3.50 (m,4H), 3.33 (m,1H), 2.93 (m,1H), 2.82 (m,4H), 2.61 (s,3H).

Example 225

Methyl (2S)-(4-methylpiperazin-1-yl)carbonyloxy-3-phenylpropionate.

Using the procedure of Example 38 but replacing morpholine with 1-methylpiperazine and modifying the isolate procedure to omit the HCl washes afforded, after chromatog raphy on silica gel with 2% methanol in chloroform, the desired product as a colorless oil. $^1$H NMR ($CDCl_3$) δ 7.17-7.33 (m,5H), 5.18 (dd,1H), 3.73 (s,3H), 3.46 (m,4H), 3.18 (dd,1H), 3.09 (dd,1H), 2.34 (m,4H), 2.28 (s,3H).

Example 226

(2S)-(4-Methylpiperazin-1-yl)carbonyloxy-3-phenylpropionic Acid.

Using the procedure of Example 174 with the resultant compound from Example 225 afforded the desired product. $^1H$ NMR ($CD_3OD$) δ 7.15-7.30 (m,5H), 4.98 (m,1H), 2.78 (s,3H).

Example 227

Benzyl (2R)-2-benzyl-3-((2-morpholin-4-ylethyl)methylaminocarbonyl)propionate.

To benzyl (2R)-2-benzyl-3-(N-(2-hydroxyethyl)-N-methyl)-aminocarbonylpropionate (110 mg, 0.31 mmol) in $CH_2Cl_2$ (2 ml) at -78°C was added triethylamine (0.070 ml, 0.50 mmol) and methanesulfonyl chloride (0.036 ml, 0.047 mmol). After 1 h morpholine (0.085 ml, 0.97 mmol) was added and the mixture was stirred at room temperature for 5 h. The solvent was evaporated and the residue was suspended in ethyl acetate, washed with saturated $NaHCO_3$ solution, water and brine, then dried over $Na_2SO_4$ and evaporated. Chromatography of the residue on silica gel with 2:1 ethyl acetate in hexane afforded 90.0 mg (68%) of the desired product. $^1H$ NMR ($CDCl_3$) δ 7.10-7.37 (m,10H), 5.00-5.20 (m,2H), 3.60-3.73 (m,4H), 2.94, 2.90 (2s,total 3H).

Example 228

(2R)-2-Benzyl-3-((2-morpholin-4-ylethyl)methylaminocarbonyl)propionic Acid.

Using the procedure of Example 189 with the resultant compound from Example 227 gave the desired product. $^1H$ NMR ($CDCl_3$) δ 7.17-7.33 (m,5H), 3.60-3.70 (m,4H), 2.92, 2.86 (2s,total 3H).

Example 229

Benzyl (2R)-2-benzyl-3-((2-imidazol-1-ylethyl)methylaminocarbonyl)propionate.

Using the procedure of Example 227 and replacing morpholine with imidazole and changing stirring at room temperature for 5 h to heating at reflux for 4 h gave, after chromatography on silica gel with 0.5% methanol in chloroform, the desired product. $^1H$ NMR ($CDCl_3$) δ 7.42 (s,1H), 7.00-7.40 (m,11H), 6.89 (s,1H), 5.18 (d,1H), 5.08 (s,1H), 4.05 (m,2H), 3.61 (m,1H), 3.50 (m,1H), 3.32 (m,1H), 3.08 (m,1H), 2.60-2.85 (m,2H), 2.59 (s,3H), 2.27 (dd,1H).

Example 230

(2R)-2-Benzyl-3-((2-imidazol-1-ylethyl)methylaminocarbonyl)propionic Acid.

Using the procedure of Example 189 with the resultant compound from Example 229 gave the desired product. $^{1}H$ NMR ($CDCl_3$) δ 7.83 (s,1H), 7.15-7.32 (m,5H), 7.14 (s,1H), 6.93 (s,1H), 3.30 (m,2H), 3.09 (m,1H), 2.60-2.78 (m,2H), 2.60 (s,3H).

Example 231

Benzyl (2R)-2-benzyl-3-(2-(4-methylpiperazin-1-ylethyl)methylaminocarbonyl)propionate.

Using the procedure of Example 227 and replacing morpholine with 1-methylpiperazine and changing stirring at room temperature for 5 h to heating at reflux for 4 h gave, after chromatography on silica gel with 1-3% methanol in chloroform, the desired product. $^{1}H$ NMR ($CDCl_3$) δ 7.10-7.35 (m,10H), 5.00-5.20 (m,2H), 2.93, 2.89 (2s,total 3H), 2.28 (2s,total 3H).

Example 232

(2R)-2-Benzyl-3-(2-(4-methylpiperazin-1-ylethyl)methylaminocarbonyl)propionic Acid.

Using the procedure of Example 189 with the resultant compound from Example 231 gave the desired product. $^{1}H$ NMR ($CDCl_3$) δ 7.13-7.32 (m,5H), 2.92, 2.88 (2s,total 3H), 2.31, 2.33 (2s,total 3H).

Example 233

Benzyl (2R)-2-benzyl-3-((2-diethylaminoethyl)methylaminocarbonyl)propionate.

Using the procedure of Example 188 but replacing 1-trifluoroethylpiperazine with N,N-diethyl-N′-methylethylene diamine gave, after chromatography on silica gel with 3% methanol in chloroform, the desired product. $^{1}H$ NMR ($CDCl_3$) δ 7.09-7.37 (m,10H), 5.02-5.20 (m,2H), 2.94, 2.88 (2s,total 3H), 0.91-1.08 (m,6H).

Example 234

(2R)-2-Benzyl-3-((2-diethylaminoethyl)methylaminocarbonyl)propionic Acid.

Using the procedure of Example 189 with the resultant product from Example 233 gave the desired product. $^{1}H$ NMR ($CDCl_3$) δ 7.12-7.30 (m,5H), 3.00 (s,3H), 1.17 (t,6H).

Example 235

(2R)-2-Benzyl-3-morpholin-4-ylcarbonylpropionyl-L-(4-thiazolyl)ala Amide of (2′S,1′R,5S)-3-ethyl-5-(1′-hydroxy-2′-amino-3′-cyclohexylpropyl)oxazolidin-2-one.

Using the procedure of Example 30 with the resultant compound from Example 169 and replacing Boc-His-OH with the resultant compound from Example 49 provided the desired compound, m.p. 99-108° C.
Anal. Calcd. for $C_{35}H_{49}N_5O_7S \bullet 0.5\ H_2O$:
C, 60.67; H, 7.27; N, 10.11.
Found:
C, 60.71; H, 6.91; N, 10.03.

## Example 236

(2R)-2-Benzyl-3-morpholin-4-ylcarbonylpropionyl-L-(3-pyrazolyl)ala Amide of (2′S,1′R,5S)-3-ethyl-5-(1′-hydroxy-2′-amino-3′-cyclohexylpropyl)oxazolidin-2-one.

Using the procedure of Example 30 with the resultant compound from Example 170 and replacing Boc-His-OH with the resultant compound from Example 49 provided the desired compound, m.p. 140-145° C.
Anal. Calcd. for $C_{35}H_{50}N_6O_7$:
C, 63.04; H, 7.56; N, 12.60.
Found:
C, 63.09; H, 7.76; N, 12.37.

## Example 237

(2R)-2-Benzyl-3-morpholin-4-ylcarbonylpropionyl-L-(2-thienyl)ala Amide of (2′S,1′R,5S)-3-ethyl-5-(1′-hydroxy-2′-amino-3′-cyclohexylpropyl)oxazolidin-2-one.

Using the procedure of Example 30 with the resultant compound from Example 171 and replacing Boc-His-OH with the resultant compound from Example 49 gave the desired product, m.p. 105-110° C.
Anal. Calcd. for $C_{36}H_{50}N_4O_7S$:
C, 63.32; H, 7.38; N, 8.20.
Found:
C, 63.50; H, 7.48; N, 8.11.

## Example 238

(2R)-2-Benzyl-3-(4-trifluoroethylpiperazin-1-ylcarbonyl)propionyl-L-(4-thiazolyl)ala Amide of (2′S,1′R,5S)-3-ethyl-5-(1′-hydroxy-2′-amino-3′-cyclohexylpropyl)oxazolidin-2-one.

Using the procedure of Example 30 with the resultant compound from Example 169 and replacing Boc-His-OH with the resultant compound from Example 189 gave the desired product, m.p. 100-108° C.
Anal. Calcd. for $C_{37}H_{51}N_6O_6F_3S$:
C, 58.10; H, 6.72; N, 10.99.
Found:
C, 58.24; H, 6.92; N, 10.91.

Example 239

(2R)-2-Benzyl-3-(4-methylpiperazin-1-ylcarbonyl)propionyl-L-(4-thiazolyl)ala Amide of (2′S,1′R,5S)-3-ethyl-5-(1′-hydroxy-2′-amino-3′-cyclohexylpropyl)oxazolidin-2-one.

Using the procedure of Example 30 with the resultant compound from Example 169 and replacing Boc-His-OH with the resultant compound from Example 191 gave the desired product, m.p. 96-103° C.
Anal. Calcd. for $C_{36}H_{52}N_6O_6S \bullet 0.75\ H_2O$:
C, 60.86; H, 7.59; N, 11.83.
Found:
C, 60.85; H, 7.53; N, 11.74.

Example 240

(4-Sulfonylmorpholinyl)carbonyl-Phe-L-(N-methyl-4-thiazolyl)ala Amide of (2′S,1′R,5S)-3-ethyl-5-(1′-hydroxy-2′-amino-3′-cyclohexylpropyl)oxazolidin-2-one.

Using the procedure of Example 30 with the resultant compound from Example 182 and replacing Boc-His-OH with the resultant compound from Example 64 gave the desired product.

Example 241

(2R)-2-Benzyl-3-((2-pyridin-2-ylethyl)methylaminocarbonyl)propyl-L-(1-imidazolyl)ala Amide of (2′S,1′R,5S)-3-ethyl-5-(1′-hydroxy-2′-amino-3′-cyclohexylpropyl)oxazolidin-2-one.

The resultant compound from Example 183 was deprotected and converted to the free amino compound as described in Example 186 and 169, and was coupled to the resultant compound from Example 193 according to the procedure of Example 30 to give the desired product.

Example 242

(2R)-2-Benzyl-3-((N-pyridin-4-yl)methylaminocarbonyl)propionyl-L-(1-pyrazolyl)ala Amide of (2′S,1′R,5S)-3-ethyl-5-(1′-hydroxy-2′-amino-3′-cyclohexylpropyl)oxazolidin-2-one.

The resulting compound from Example 184 was deprotected and converted to the free amino compound as described in Examples 186 and 169, and was coupled to the resultant compound from Example 195 according to the procedure of Example 30 to give the desired product.

Example 243

1-Methylisonipicotonyl-(0-methyl)Tyr-L-(β-azido)ala Amide of (2′S,1′R,5S)-3-ethyl-5-(1′-hydroxy-2′-amino-3′-cyclohexylpropyl)oxazolidin-2-one.

Using the procedure of Example 30 with the resultant compound of Example 185 and replacing Boc-His-OH with the resultant compound from Example 222 gave the desired product.

Example 244

1-Methylisonipicotonyl-(0-methyl)Tyr-L-(dimethylamino)ala Amide of (2′S,1′R,5S)-3-ethyl-5-(1′-hydroxy-2′-amino-3′-cyclohexylpropyl)oxazolidin-2-one.

The resultant compound from Example 243, formaldahyde in water solution, and 10% palladium on carbon were stirred under a hydrogen atmosphere in methanol. The reaction was filtered and evaporated to afford the desired product.

Example 245

Isonicitonyl-(0-methyl)Tyr-L-(4-morpholinyl)ala Amide of (2′S,1′R,5S)-3-ethyl-5-(1′-hydroxy-2′-amino-3′-cyclohexylpropyl)oxazolidin-2-one.

Using the procedure of Example 30 with the resultant compound from Example 186 and replacing Boc-His-OH with the resultant compound from Example 220 gave the desired product.

Example 246

1-Methylpiperazin-4-ylcarbonyl-(0-methyl)Tyr-L-(β-N-methyl-N-methoxylamino)alanine Amide of (2′S,1′R,5S)-3-ethyl-5-(1′-hydroxy-2′-amino-3′-cyclohexylpropyl)oxazolidin-2-one.

Using the procedure of Example 30 with the resultant compound from Example 187 and using the resultant compound from Example 224 in place of Box-His-OH gave the desired product.

Example 247

(2S)-2-(4-Morpholinyl)-3-phenylpropionyl-L-(4-thiazolyl)ala Amide of (2′S,1′R,5S)-3-ethyl-5-(1′-hydroxy-2′-amino-3′-cyclohexylpropyl)oxazolidin-2-one.

Using the procedure of Example 30 with the resultant compound from Example 169 and replacing Boc-His-OH with the resultant compound from Example 197 gave the desired product.

Example 248

(2R)-2-Benzyl-3-thiazol-4-ylpropionyl-L-(4-thiazolyl)ala Amide of (2'S,1'R,5S)-3-ethyl-5-(1'-hydroxy-2'-amino-3'-cyclohexylpropyl)oxazolidin-2-one.

Using the procedure of Example 30 with the resultant compound from Example 169 and replacing Boc-His-OH with the resultant compound from Example 200 gave the desired compound.

Example 249

(2R)-2-Benzyl-5-tert-butylsulfinyl-4-oxopentanoyl-L-(4-thiazolyl)ala Amide of (2'S,1'R,5S)-3-ethyl-5-(1'-hydroxy-2'-amino-3'-cyclohexylpropyl)oxazolidin-2-one.

Using the procedure of Example 30 with the resultant compound from Example 169 and replacing Boc-His-OH with the resultant compound from Example 204 provided the desired compound.

Example 250

(2R)-2-Benzyl-5-tert-butylsulfonyl-4-oxopentanoyl-L-(4-thiazolyl)ala Amide of (2'S,1'R,5S)-3-ethyl-5-(1'-hydroxy-2'-amino-3'-cyclohexylpropyl)oxazolidin-2-one.

Using the procedure of Example 30 with the resultant compound from Example 169 and replacing Boc-His-OH with the resultant compound from Example 205 provided the desired product.

Example 251

(2R)-2-Benzyl-5-morpholin-4-yl-4-oxopentanoyl-L-(4-thiazolyl)ala Amide of (2'S,1'R,5S)-3-ethyl-5-(1'-hydroxy-2'-amino-3'-cyclohexylpropyl)oxazolidin-2-one.

Using the procedure of Example 30 with the resultant compound from Example 169 and replacing Boc-His-OH with the resultant compound from Example 207 gave the desired compound.

Example 252

(2R,4R) and (2R,4S)-2-Benzyl-5-morpholin-4-yl-4-hydroxypentanoyl-L-(4-thiazolyl)ala Amide of (2'S,1'R,5S)-3-ethyl-5-(1'-hydroxy-2'-amino-3'-cyclohexylpropyl)oxazolidin-2-one.

The resultant compound from Example 251 in methanol was treated with $NaBH_4$. After 3 h the mixture was quenched with 2 M HCl, poured into saturated $NaHCO_3$ solution, and extracted into ethyl acetate which was dried and evaporated. Chromatography of the residue on silica gel with methanol/chloroform mixtures provided the desired products.

Example 253

(2R)-2-Benzyl-3-pyrazol-1-ylpropionyl-L-(4-thiazolyl)ala Amide of (2'S,1'R,5S)-3-ethyl-5-(1'-hydroxy-2'-amino-3'-cyclohexylpropyl)oxazolidin-2-one.

Using the procedure of Example 30 with the resultant compound from Example 169 and replacing Boc-His-OH with the resultant compound from Example 211 gave, after isomer separation on silica gel, the desired product.

Example 254

(2R)-2-Benzyl-3-(N-methoxyl-N-methylamino)propionyl-L-(4-thiazolyl)ala Amide of (2'S,1'R,5S)-3-ethyl-5-(1'-hydroxy-2'-amino-3'-cyclohexylpropyl)oxazolidin-2-one.

Using the procedure of Example 30 with the resultant compound from Example 169 and replacing Boc-His-OH with the resultant compound from Example 212 gave, after isomer separation on silica gel, the desired product.

Example 255

(2S)-2-Benzyl-3-tert-butylsulfonylpropionyl-HisAmide of (2'S,1'R,5S)-3-ethyl-5-(1'-hydroxy-2'-amino-3'-cyclohexylpropyl)oxazolidin-2-one

Using the procedure of Example 30 with the resultant compound from Example 29 and replacing the Boc-His-OH with the resultant compound from Example 218 gave an intermediate imidazole-Boc product which was deprotected in 3:1:1 acetic acid/tetrahydrofuran/water at 50° C for 19 h. Solvent evaporation and chromatography on silica gel with chloroform/methanol mixtures afforded the desired product, m.p. 200-205° C.

Anal. Calcd. for $C_{34}H_{51}N_5O_7S \bullet 0.5\ H_2O$:
C, 59.80; H, 7.67; N, 10.26.
Found:
C, 59.80; H, 7.54; N, 9.93.

Example 256

(2S)-(4-Methylpiperazin-1-yl)carbonyloxy-3-phenylpropionyl-L-(4-thiazolyl)ala Amide of(2′S,1′R,5S)-3-ethyl-5-(1′-hydroxy-2′-amino-3′-cyclohexylpropyl)oxazolidin-2-one.

Using the procedure of Example 30 with the resultant compound from Example 169 and replacing Boc-His-OH with the resultant compound from Example 226 gave the desired product.

Example 257

(2R)-2-Benzyl-((2-morpholin-2-ylethyl)methylaminocarbonyl)propionyl-L-(4-thiazolyl)ala Amide of (2′S,1′R,5S)-3-ethyl-5-(1′-hydroxy-2′-amino-3′-cyclohexylpropyl)oxazolidin-2-one.

Using the procedure of Example 30 with the resultant compound from Example 169 and replacing Boc-His-OH with the resultant compound from Example 228 gave the desired product.

Example 258

(2R)-2-Benzyl-3-((2-imidazol-1-yethyl)methylaminocarbonyl)propionyl-L-(4-thiazolyl)ala Amide of (2′S,1′R,5S)-3-ethyl-5-(1′-hydroxy-2′-amino-3′-cyclohexylpropyl)oxazolidin-2-one.

Using the procedure of Example 30 with the resultant compound from Example 169 and replacing Boc-His-OH with the resultant compound from Example 230 gave the desired product.

Example 259

(2R)-2-Benzyl-3-(2-(4-methylpiperazin-1-ylethyl)methylaminocarbonyl)propionylL-(4-thiazolyl)ala Amide of (2′S,1′R,5S)-3-ethyl-5-(1′-hydroxy-2′-amino-3′-cyclohexylpropyl)oxazolidin-2-one.

Using the procedure of Example 30 with the resultant compound from Example 169 and replacing Boc-His-OH with the resultant compound from Example 232 gave the desired product.

Example 260

(2R)-2-Benzyl-3-((2-L-(4-thiazolyl)ala Amide of (2′S,1′R,5S)-3-ethyl-5-(1′-hydroxy-2′-amino-3′-cyclohexylpropyl)oxazolidin-2-one.

Using the procedure of Example 30 with the resultant compound from Example 169 and replacing Boc-His-OH with the resultant compound from Example 234 gave the desired product.

Example 261

(2S,3R,4S)-1-Benzyloxycarbonylethylamino-2-amino-3-methoxyethoxymethoxy-4-tert-butyloxycarbonylamino-5-cyclohexylpentane.

The resultant compound from Example 13 (3.5616 g, 6.019 mmol) in methanol (40 ml) was treated with triethylamine (2.60 ml, 18.6 mmol) and propane 1,3-dithiol (1.85 ml, 18.4 mmol). After 72 h the mixture was filtered, evaporated, and chromatographed on silica gel with 3% methanol in chloroform to afford 3.1498 g (93%) of an oil. $^1$H NMR ($CDCl_3$) $\delta$ 7.27-7.40 (m,5H), 5.13 (s,2H), 4.24 (m,2H), 4.05 (m,1H), 3.38 (s,3H), 3.02 (m,1H), 1.43 (s,9h), 1.14 (m,3H).

## Example 262

(2S,3R,4S)-1-Ethylamino-2-amino-3-methoxyethoxymethoxy-4-tert-butyloxycarbonylamino-5-cyclohexylpentane.

The resutlant compound from Example 261 and 10% palladium on carbon in acetic acid were stirred under a hydrogen atmosphere. After filtration and solvent evaporation, the residue was partitioned between chloroform and aqueous $K_2CO_3$ solution and extracted into chloroform which was dried over $Na_2SO_4$ and evaporated to give the desired product. $^1$H NMR ($CDCl_3$) $\delta$ 4.86 (d,1H), 4.77 (s,2H), 4.04 (m,1H), 3.73 (m,2H), 3.55 (m,2H), 3.38 (s,3H), 3.27 (m,1H), 2.88 (m,2H), 2.50-2.76 (m,3H), 1.42 (s,9H), 1.12 (t,3H).

## Example 263

(2′S,3′R,4S)-2-Ethyl-4-(1′-methoxyethoxymethoxy-2′-tert-butyloxycarbonylamino-3′-cyclohexylpropyl)-1,2,5-thiadiazolidine-1,1-dioxide.

The resultant compound from Example 262 (130 mg, 0.30 mmol) in $CH_2Cl_2$ (5 ml) was treated with diisopropylethylamine (0.185 ml, 1.06 mmol), cooled to -23°C and treated with $SO_2Cl_2$ (0.031 ml, 0.31 mmol). After 90 min at -23°C and 2 h at room temperature the mixture was evaporated and taken up in ethyl acetate which was washed with 0.5 M $H_3PO_4$, aqueous $NaHCO_3$ solution and brine, and then dried over $Na_2SO_4$ and evaporated. Chromatography of the residue on silica gel with 40% ethyl acetate in hexane afforded 55 mg (37%) of the desired product. $^1$H NMR ($CDCl_3$) $\delta$ 5.82 (d,1H), 4.77 (d,1H), 4.71 (d,1H), 4.59 (d,1H), 3.93 (m,1H), 3.80 (m,1H), 3.60-3.70 (m,2H), 3.50-3.60 (m,3H), 3.45 (m,1H), 3.38 (s,3H), 3.09-3.28 (m,2H), 2.88-3.01 (m,1H), 1.43 (s,9H), 1.26 (t,3H).

## Example 264

Boc-Phe-His Amide of (2′S,1′R,4S)-2′-Ethyl-4-(1′-hydroxy-2′-amino-3′-cyclohexylpropyl)-1,2,5-thiadiazolidine-1,1-dioxide.

Using the procedure of Example 30 with the resultant compound from Example 263 and replacing Boc-His-OH with Boc-Phe-His-OH gave the desired product, m.p. 159-164°C
Anal. Calcd. for $C_{33}H_{51}N_7O_7S \bullet 0.5\ H_2O$:
C, 56.71; H, 7.50; N, 14.03.
Found:
C, 56.72; H, 7.42; N, 13.67.

Example 265

(2′S,1′R,3S)-5-Ethyl-3-(1′-methoxyethoxymethoxy-2′-tert-butyloxycarbonylamino-3′-cyclohexylpropyl)-1,2,5-oxothiazolidine-1,1-dioxide.

Using the procedure of Example 7 but replacing phosgene in toluene with $SO_2Cl_2$ gave the desired product.

Example 266

(2′S,1′R,3S)-5-Ethyl-3-(1′-methoxyethoxymethoxy-2′-tert-butyloxycarbonylamino-3′-cyclohexylpropyl)-1,2,5-oxothiazolidine-1-oxide.

Using the procedure of Example 7 but replacing phosgene in toluene with thionyl chloride gave the desired product.

Example 267

Boc-Phe-His Amide of (2′S,1′R,3S)-5-ethyl-3-(1′-hydroxy-2′-amino-3′-cyclohexylpropyl)-1,2,5-oxothiazolidine-1,1-dioxide.

Using the procedure of Example 30 with the resultant compound from Example 265 and using Boc-Phe-His-OH in place of Boc-His-OH gave the desired product.

Example 268

Boc-Phe-His Amide of (2′S,1′R,3S)-5-ethyl-3-(1′-hydroxy-2′-amino-3′-cyclohexylpropyl)-1,2,5-oxothiazolidine-1-oxide.

Using the procedure of Example 30 with the resultant compound from Example 266 and using Boc-Phe-His-OH in place of Boc-His-OH gave the desired product.

Example 269

3-(t-Butyloxycarbonyl)-4-(cyclohexylmethyl)-2,2-dimethyl-5-vinyloxazolidine.

The procedure of S. Thaisrivong (J. Med. Chem. 1987, 30, 976) was employed. A solution of 40 g of the resultant compound of Example 1 and 102 g of 2-methoxypropene in 250 ml of dichloromethane was stirred at room temperature. Solid pyridinium p-toluenesulfonate (PPTS) (177 g) was added slowly to the reaction

mixture. After addition was complete, the reaction was stirred for 1 h and neutralized by addition of solid sodium bicarbonate. The solids were filtered and the filtrate was concentrated. Flash chromatography on silica gel gave 57 g of the desired compound. IR ($CDCl_3$) 1690 (C=O carbamate) $cm^{-1}$; $^1H$ NMR ($CDCl_3$) δ 5.95 (m,1H), 5.32 (m,1H), 5.20 (dt,1H), 4.27 (dd,1H), 1.47 (s,9H).

Anal. Calcd. for $C_{19}H_{33}NO_3$:
C, 70.55; H, 10.28; N, 4.33.
Found:
C, 70.47; H, 10.27; N, 4.09.

## Example 270

### 3-(t-Butyloxycarbonyl)-4-(cyclohexylmethyl)-2,2-dimethyloxazolidine-5-carboxaldehyde.

A solution of 10 g of the resultant compound of Example 269 in 150 ml of 2:1 dichloromethane:methanol was cooled in an dry-ice acetone bath. Ozone was bubbled through the solution until a blue color persisted (1 h). Dry nitrogen was then bubbled through the reaction mixture to remove excess dissolved ozone. The reaction mixture was cannulated into a suspension of 8 g zinc dust, 8 ml glacial acetic acid, 200 ml water, and 200 ml of methanol cooled to -45°C. After 5 min the bath was removed and the mixture allowed to warm to room temperature overnight. 100 ml of saturated sodium chloride was added and the entire reaction mixture extracted with two 300 ml portions of dichloromethane. The combined dichloromethane extracts were decanted, dried ($MgSO_4$), filtered, and evaporated. The crude aldehyde was purified by flash chromatography (1:4) ethyl acetate:hexane to give 9.7 g of the desired compound as a mixture of diastereomers (3:1 trans:cis) as judged by the integrated resonances of the two aldehyde protons. IR ($CDCl_3$) 1735 (C=O aldehyde), 1690 (C=O carbamate) $cm^{-1}$; $^1H$ NMR ($CDCl_3$) δ 9.83 (s,1H, CHO), 9,73 (d,1H,CHO cis diastereomer), 4.14 (m,1H), 1.46 (s,9H).

Anal. Calcd. for $C_{18}H_{31}NO_4$:
C, 66.43; H, 9.60; N, 4.30.
Found:
C, 65.27; H, 9.79; N, 4.20.

### Equilibration of Aldehyde Isomers

A suspension of 25 g of the above aldehyde in 300 ml of methanol and powdered potassium carbonate (10.7 g) was stirred at room temperature for 6 h. The reaction mixture was cooled in an ice-water bath and treated with 9.3 g of glacial acetic acid for 5 min. A solution of 0.5 M sodium dihydrogen phosphate (300 ml) was added to the mixture. After 30 min, the solution was concentrated to one-half the volume under reduced pressure and extracted with ether (600 ml). The combined ether extracts were dried ($MgSO_4$), filtered, and concentrated. The aldehyde was purified by flash chromatography using (1:4) ethyl acetate:hexane to give 19.5 g of the desired compound as an 8:1 mixture of trans:cis diastereomers.

## Example 271

### (5S,4'S,5'R)-5-(3-(t-Butyloxycarbonyl)-4-(cyclohexylmethyl)-2,2-dimethyloxazolidin-5-yl)-3-methylenedihydrofuran-2(4H)-one.

A solution of 16.52 g (51 mmol) of the resultant compound of Example 270 in 15 ml of anhydrous tetrahydrofuran was treated with 3.98 g (61 mmol) of freshly activated zinc dust. With vigorous stirring, the mixture was treated with 10 g (56 mmol) of methyl 2-(bromomethyl)acrylate at a rate which maintained the

temperature at 50-60° C. Upon completion of the addition, the mixture was stirred at 50° C for 1 h. After being allowed to cool, the mixture was poured into 100 ml of cold 1 M HCl and extracted with dichloromethane (3 X 100 ml). The combined organic layers were washed successively with saturated aqueous $NaHCO_3$ and $H_2O$, dried over $Na_2SO_4$, and concentrated. Silica gel chromatography using 9:1 hexane:ethyl acetate provided 10.83 g (61%) of the desired compound. $^1H$ NMR ($CDCl_3$) δ 0.8-2.0 (br envelope), 1.49 (s,9H), 1.54 (s,3H), 1.57 (s,3H), 2.93 (ddt,J=18,6,3Hz,1H), 3.05 (m,1H), 3.70 (m,1H), 4.07 (m,1H), 4.47 (ddd,J=13,9,6Hz,1H), 5.70 (br t,J=3Hz,1H), 6.28 (t,J=3Hz,1H). Mass spectrum: $(M+H)^+$ = 394.

Anal. Calcd. for $C_{22}H_{35}NO_5$:
C, 67.15; H, 8.96; N, 3.56.
Found:
C, 67.66; H, 9.11; N, 3.60.

## Example 272

### (3S,5S,4'S,5'R)-5-(3-(t-Butyloxycarbonyl)-4-(cyclohexylmethyl)-2,2-dimethyloxazolidin-5-yl)-3-methyldihydrofuran-2(3H)-one.

A mixture of 8.03 g (20 mmol) of the resultant compound of Example 271 and 0.81 g of 10% palladium on carbon in 200 ml of ethyl acetate was shaken under 4 atmospheres of $H_2$. After filtration, concentration of the filtrate gave 7.58 g (94%) of the desired compound. $^1H$ NMR ($CDCl_3$) δ 0.8-2.0 (br envelope), 1.31 (s,3H), 1.48 (s,9H), 1.54 (s,3H), 1.58 (s,3H), 2.57 (m,1H), 2.68 (m,1H), 3.74 (m,1H), 4.04 (m,1H), 4.31 (ddd,J=13,9,6Hz,1H). Mass spectrum: $(M+H)^+$ = 396.

## Example 273

### (4S,5R,1'S,3'S)-3-(t-Butyloxycarbonyl)-4-(cyclohexylmethyl)-5-(1,4-dihydroxy-3-methylbutyl)-2,2-dimethyloxazolidine.

A mixture of 0.50 g (1.26 mmol) of the resultant compound of Example 272 and 0.15 g (4 mmol) of sodium borohydride in 50 ml of tetrahydrofuran was heated at reflux under $N_2$ atmosphere for 48 h. After being allowed to cool, the mixture was treated cautiously with aqueous $NH_4Cl$, extracted with ether, washed with saturated brine, dried over $MgSO_4$, and concentrated in vacuo. Silica gel chromatography using 2:1 chloroform/ethyl acetate gave 0.37 g (73%) of the desired compound. $^1H$ NMR ($CDCl_3$) δ 0.7-2.0 (br envelope), 0.94 (d,J=7Hz,3H), 1.49 (s,9H), 1.52 (s,3H), 1.55 (s,3H), 3.43 (dd,J=11,8Hz,1H), 3.55-3.7 (m,3H), 4.09 (br d,1H). Mass spectrum: $(M+H)^+$ = 400.

## Example 274

### (4S,5R,2'S,4'S)-3-(t-Butyloxycarbonyl)-4-(cyclohexylmethyl)-2,2-dimethyl-5-(4-methyltetrahydrofuran-2-yl)oxazolidine.

A solution of 51 mg (0.13 mmol) of the resultant compound of Example 273 and 0.037 ml (0.27 mmol) of triethylamine in 2 ml of dichloromethane was cooled to 0° C under $N_2$ atmosphere and treated with 0.012 mol (0.15 mmol) of methanesulfonyl chloride. After 1 h, the solution was diluted with dihloromethane, washed successively with 10% citric acid, water and saturated aqueous $NaHCO_3$, dried over $Na_2SO_4$, and

concentrated in vacuo. The crude mesylate thus produced (59 mg) was taken up in 8 ml of dry tetrahydrofuran, treated with 20 mg (0.50 mmol) of sodium hydride (60% dispersion in oil), and heated at reflux for 2 h. After being allowed to cool, the solution was treated cautiously with saturated aqueous $NH_4Cl$, extracted with ether, dried over $MgSO_4$ and concentrated. Silica gel chromatography using 9:1 hexane/ethyl acetate gave 30 mg (75%) of the desired compound. $^1H$ NMR ($CDCl_3$) $\delta$ 0.7-2.4 (br envelope), 1.06 (d,J=7Hz,3H), 1.48 (s,9H), 1.52 (s,3H), 1.56 (s,3H), 3.30 (t,J=9Hz,1H), 3.66 (m,1H), 3.9-4.0 (m,3H). Mass spectrum: $(M+H)^+$ = 382.

## Example 275

### (5S,4′S,5′R)-5-(3-(t-Butyloxycarbonyl)-4-cyclohexylmethyl)-2,2-dimethyloxazolidin-5-yl)-3,3-dimethyldihydrofuran-2(3H)-one.

A solution of 0.21 ml (1.5 mmol) of dry diisopropylamine in 15 ml of anhydrous tetrahydrofuran was cooler under nitrogen atmosphere to -78°C and treated with 0.56 ml (1.4 mmol) of n-butyllithium. The resulting solution was allowed to warm for 5 min, recooled to -78°C, treated with a solution of 500 mg (1.26 mmol) of the resultant compound of Example 272 in 5 ml of tetrahydrofuran, stirred for 15 min, and treated with 0.08 ml (1.3 mmol) of iodomethane. After being allowed to warm to ambient temperature, the solution was partitioned between ether and aqueous $NH_4Cl$, washed sequentially with 10% $Na_2S_2O_3$ and saturated brine, dried over $MgSO_4$, and concentrated. Silica gel chromatography using 5.5:1 hexane/ethyl acetate afforded 456 mg (88%) of the desired compound as a white solid. $^1H$ NMR ($CDCl_3$) $\delta$ 0.8-1.7 (br envelope), 1.29 (s,3H), 1.31 (s,3H), 1.49 (s,9H), 1.53 (s,3H), 1.58 (s,3H), 1.89 (m,1H), 1.98 (dd,J=13,9Hz,1H), 2.21 (m,1H), 3.72 (m,1H), 4.04 (m,1H), 4.36 (td,J=8,6Hz,1H). Mass spectrum: $(M+H)^+$ = 410.
Anal. Calcd. for $C_{23}H_{39}NO_5$:
C, 67.45; H, 9.60; N, 3.42.
Found:
C, 67.69; H, 9.64; N, 3.42.

## Example 276

### (4S,5R,1′S)-3-(t-Butyloxycarbonyl)-4-(cyclohexylmethyl)-5-(1,4-dihydroxy-3,3-dimethylbutyl)-2,2-dimethyloxazolidine.

Using the procedure of Example 273 with the resultant compound of Example 275 gave, after silica gel chromatography using 7:3 hexane/ethyl acetate, a 77% yield of the desired compound as a white foam. $^1H$ NMR ($CDCl_3$) $\delta$ 0.9-2.0 (br envelope), 0.95 (s,3H), 0.97 (s,3H), 1.49 (s,9H), 1.52 (s,3H), 1.56 (s,3H), 3.41 (d,J=11Hz,1H), 3.49 (d,J=11Hz,1H), 3.56 (m,1H), 3.69 (m,1H), 4.09 (m,1H). Mass spectrum: $(M+H)^+$ = 414.
Anal. Calcd. for $C_{23}H_{43}NO_5 \bullet 0.25\ H_2O$:
C, 66.07; H, 10.49; N, 3.35.
Found:
C, 66.05; H, 10.16; N, 3.40.

## Example 277

Boc-Phe-His Amide of (5S,1′R,2′S)-5-(2-Amino-3-cyclohexyl-1-hydroxy)-3-methylenedihydrofuran-2(4H)-one.

Using the procedure of Example 30 with the resultant compound of Example 271 and using Boc-Phe-His-OH in place of Boc-His-OH gave the desired compound, mp 171-173 °C. Mass spectrum: $(M+H)^+$ = 638.
Anal. Calcd. for $C_{34}H_{47}N_5O_7 \bullet 1.5\ H_2O$:
C, 61.43; H, 7.58; N, 10.53.
Found:
C, 61.14; H, 7.28; N, 10.40.

## Example 278

Boc-Phe-His Amide of (3S,5S,1′R,2′S)-5-(2-Amino-3-cyclohexyl-1-hydroxy)-3-methyldihydrofuran-2(3H)-one.

Using the procedure of Example 30 with the resultant compound of Example 272 and using Boc-Phe-His-OH in place of Boc-His-OH gave the desired compound, mp 129-130 °C. Mass spectrum: $(M+H)^+$ = 640.
Anal. Calcd. for $C_{34}H_{49}N_5O_7 \bullet 1.75\ H_2O$:
C, 60.83; H, 7.88; N, 10.43.
Found:
C, 60.69; H, 7.42; N, 10.27.

## Example 279

Boc-Phe-His Amide of (4S,5R,6S)-6-Amino-7-cyclohexyl-4,5-dihydroxy-1-hepten-2-oicAcid Sodium Salt.

A solution of 32 mg (0.50 mmol) of the resultant compound of Example 277 in 1 ml of tetrahydrofuran and 0.5 ml of $H_2O$ was cooled to 0 °C and treated with 0.05 ml (0.15 mmol) of 3 N NaOH. The resulting solution was stirred at 0 °C for 3 h, and concentrated to dryness. The residue was washed with ethyl acetate and dried *in vacuo* to give the desired compound, mp 198-200 °C. Mass spectrum: $(M+H)^+$ = 678; $(M\text{-}Na+2H)^+$ = 656.

## Example 280

Boc-Phe-His Amide of (2S,4S,1′R,2′S)-2-(2-Amino-7-cyclohexyl-4,5-dihydroxyheptan-2-oicAcid Sodium Salt.

Using the procedure of Example 279 with the resultant compound of Example 278 gave the desired compound, mp 154-155 °C. Mass spectrum: $(M\text{-}Na+2H)^+$ = 658.

## Example 281

Boc-Phe-His Amide of (2S,3R,4S,6S)-2-Amino-1-cyclohexyl-3,4,7-trihydroxy-6-methylheptane.

Using the procedure of Example 30 with the resultant compound of Example 273 and using Boc-Phe-His-OH in place of Boc-His-OH gave the desired compound. Mass spectrum: $(M+H)^+$ = 644.

Example 282

Boc-Phe-His Amide of (2S,4S,1′R,2′S)-2-(2-Amino-3-cyclohexyl-1-hydroxy)-4-methyltetrahydrofuran.

Using the procedure of Example 30 with the resultant compound of Example 274 and using Boc-Phe-His-OH in place of Boc-His-OH gave the desired compound. Mass spectrum: $(M+H)^+$ = 626.

Example 283

Boc-Phe-His Amide of (5S,1′R,2′S)-5-(2-Amino-3-cyclohexyl-1-hydroxy)-3,3-dimethyldihydrofura-2(3H)-one.

Using the procedure of Example 30 with the resultant compound of Example 275 and using Boc-Phe-His-OH in place of Boc-His-OH gave the desired compound. Mass spectrum: $(M+H)^+$ = 640.
Anal. Calcd. for $C_{35}H_{51}N_5O_7 \bullet H_2O$:
C, 62.57; H, 7.95; N, 10.42.
Found:
C, 62.79; H, 7.88; N, 10.21.

Example 284

Boc-Phe-His Amide of (2S,3R,4S)-2-Amino-1-cyclohexyl-3,4,7-trihydroxy-6,6-dimethylheptane.

Using the procedure of Example 30 with the resultant compound of Example 276 and using Boc-Phe-His-OH in place of Boc-Phe-OH gave the desired compound. Mass spectrum: $(M+H)^+$ = 658.

Example 285

Boc-L-(4-thiazolyl)-Ala Amide of (2S,4S,1′R,2′S)-2-(2-Amino-3-cyclohexyl-1-hydroxy)-4-methyltetrahydrofuran.

Using the procedure of Example 30 with the resultant compound of Example 274 and using Boc-L-(4-thiazolyl)-Ala-OH in place of Boc-His-OH gave the desired compound. $^1H$ NMR ($CDCl_3$) δ 0.7-2.1 (br envelope), 1.02 (d,J=7Hz,3H), 1.47 (s,9H), 2.28 (m,1H), 3.20 (m,2H), 3.34 (m,1H), 3.47 (m,3H), 3.88 (m,2H), 4.52 (m,1H), 6.33 (br d,1H), 6.59 (br d,1H), 7.13 (d,J=2Hz,1H), 8.77 (d,J=2Hz,1H).

Anal. Calcd. for $C_{25}H_{41}N_3O_5S$:
C, 60.58; H, 8.34; N, 8.48.
Found:
C, 61.08; H, 8.33; N, 8.46.

## Example 286

### (2R)-2-Benzyl-3-(morpholinocarbonyl)propionyl-L-(4-thiazolyl)-Ala Amide of (2S,4S,1′R,2′S)-2-(2-Amino-3-cyclohexyl-1-hydroxy)-4-methyltetrahydrofuran.

Using the procedure of Example 30 with the resultant compound of Example 285 and using the resultant compound of Example 49 in place of Boc-His-OH gave the desired compound, mp 61-62. Mass spectrum: $(M+H)^+ = 655$.
Anal. Calcd for $C_{35}H_{50}N_4O_6S \bullet 0.5\ H_2O$:
C, 63.32; H, 7.74; N, 8.44.
Found:
C, 63.45; H, 7.69; N, 8.30.

## Example 287

### tert-Butyl (2(S)-2-(tert-butyloxycarbamoyl)-3-phenylpropyl)sulfide.

Tert-butyl mercaptan (0.52 ml, 416 mg, 4.61 mmol) was added to a suspension of sodium hydride (111 mg, 4.62 mmol) in 8 ml of THF, cooled to 0 °C under a nitrogen atmosphere. The resulting suspension was stirred at 0 °C for 1 h, and for an additional 3 h at room temperature. To the resulting thick white suspension, cooled to 0 °C, was added dropwise via cannula, a solution of 1-phenyl-3-trifluorosulfonyloxy-2-t-butyloxyamidopropane (1.63 g, 4.02 mmol) in 10 ml of THF. The resultant mixture was allowed to stir and slowly warm to room temperature over 18 h. The mixture was partitioned between 75 ml $Et_2O$ and 50 ml water. The organic phase was extracted with 50 ml saturated $NaHCO_3$, then the combined aqueous phases were extracted with 2 x 50 ml $Et_2O$. All organic phases were combined, washed with 50 ml brine, dried ($MgSO_4$), and the filtrate concentrated under reduced pressured to afford 1.40 g of orangish solid. Purification by recrystallization (hexanes, three crops) gave 1.16 g (89%) of white crystals; m.p. 67-69 °C. $^1H$ NMR ($CDCl_3$) $\delta$ 1.31 (s,9H), 1.43 (s,9H), 2.57 (dd,1H), 2.66 (dd,1H), 2.84 (m,2H), 4.04 (bm,1H), 4.79 (bm,1H), 7.16-7.4 (m,5H).

## Example 288

### tert-Butyl (2(S)-2-(tert-butyloxycarbamoyl)-3-phenylpropyl)sulfone.

The resultant compound from Example 72 (863 mg, 2.67 mmol) was dissolved in 5 ml absolute ethanol and 5 ml THF, and 2.5 ml water and 5 ml pH 4.5 aqueous phosphate buffer was added. The mixture was cooled in ice and treated with OXONE (2.45 g, 8.00 mmol $KHSO_5$). The mixture was stirred and allowed to warm to room temperature. After 60 h, the mixture was partitioned between 50 ml water and 50 ml $CH_2Cl_2$. The aqueous phase was further extracted with 3 x 50 ml $CH_2Cl_2$, and the combined organic extracts were washed with brine, dried ($MgSO_4$), filtered, and the filtrate concentrated under reduced pressure to give 942

mg of white solid. Recrystallization from $CH_2Cl_2/Et_2O$ (three crops) afforded 839 mg (88%) of white crystals; m.p. 169-170.5°C. $^1H$ NMR ($CDCl_3$) δ 1.38 (s,9H), 1.42 (s,9H), 3.1-3.3 (m,4H), 4.30 (bm,1H), 5.26 (bs,1H), 7.21-7.42 (m,5H). High resolution mass spectrum; calcd. for $C_{13}H_{30}NO_4S$ $(M+H)^+$: 356.1895. Found: 356.1894.

Example 289

tert-Butyl (2(S)-2-amino-3-phenylpropyl)sulfone hydrochloride.

The resultant compound from Example 73 (741 mg, 2.09 mmol) was treated with 2.5 ml of 4.5 M HCl in dioxane at room temperature. After 24 h the volatiles were removed under reduced pressure, and the residue placed under high vacuum overnight, to afford 614 mg (100%) of the desired compound; m.p. >220°C. $^1H$ NMR ($CDCl_3$) δ 1.34 (s,9H), 3.20 (dd,1H), 3.26 (dd,1H), 3.70 (dd,1H), 3.89 (dd,1H), 4.25 (bm,1H), 7.25-7.35 (m,5H), 8.7 (bs,1H + $H_2O$).

Example 290

Diethyl 4-Thiazolylmethyl malonate.

Sodium metal (4.14 g, 180 mmol) was added to 150 ml of absolute ethanol and cooled in an ice-water bath under a nitrogen atmosphere. After the sodium dissolved diethyl malonate (28.83 g, 180 mmol) was added and the solution warmed to room temperature and stirred for 2 h. The reaction mixture was recooled in an ice-water bath and 4-chloromethylthiazole (2.4 g, 18.0 mmol) in 5 ml of ethanol was added dropwise over 10 min and stirred for 20 h at room temperature. The crude reaction mixture was concentrated then partitioned between 50 ml $H_2O$ and 50 ml $CH_2Cl_2$ the organic layer was filtered through $MgSO_4$ and concentrated. The crude product was chromatographed on silica gel with 10% EtOAc/hex to give 3.1 g (67%) of a clear oil. $^1H$ NMR ($CDCl_3$, TMS) δ (t,6H), 3.4 (d,2H), 3.9 (t,1H), 4.15 (qd,4H), 7.0 (d,1H), 8.7 (d,1H); IR ($CDCl_3$) 2900, 2250, 1740, 1290, 1050 $cm^{-1}$. Mass spectrum: $(M+H)^+$ = 258.
Anal. Calcd for $C_{11}H_{15}NO_4S$:
C, 51.34; H, 5.88; N, 5.44.
Found:
C, 50.84; H, 5.82; N, 5.39.

Example 291

Ethyl 4-Aza-5(S)-benzyl-6-t-butylsulfonyl-3-oxo-2(R,S)-(4-thiazolylmethyl)hexanoate.

To a solution of 0.5 g (1.9 mmol) of the diethyl malonate (Example 290) in 25 ml of absolute ethanol cooled in an ice-water bath and stirred under a nitrogen atmosphere was added a solution of potassium hydroxide (0.11 g, 1.9 mmol) in 25 ml of absolute ethanol. Upon complete addition, the reaction was stirred for 72 h at room temperature and concentrated to afford the crude acid salt. The acid salt was redissolved in 30 ml of DMF and cooled in an ice-water bath. 1-Hydroxybenzotriazole (HOBT) (0.38 g, 2.88 mmol), 4-methyl morpholine (0.28 g, 2.8 mmol), tert-butyl-(2(S)-2-amino-3-phenylpropyl)sulfone hydrochloride (0.55 g, 1.9 mmol), and (1-(3-dimethyl-aminopropyl)-3-ethylcarbodiimide hydrochloride (EDAC) (0.36 g, 1.9 mmol) were added. After 18 h, the solution was diluted with 50 ml saturated aqueous $NaHCO_3$ and extracted with 30 ml EtOAc (3X). The combined ethyl acetate extracts were washed with 25 ml 0.5 N HCl (2X), filtered

through $MgSO_4$, and evaporated to dryness. The crude product was chromatographed on silica gel with 1:1 EtOAc/hex to give 0.78 g (88%) of a white solid. $^1H$ NMR ($CDCl_3$, TMS) δ 1.2 (td,6H), 1.4 (d,9H), 1.7 (s,2H-($H_2O$)), 3.0-3.3 (m,4H), 3.35 (dd,2H), 3.74 (2dd,1H), 4.12 (m,4H), 4.54 (m,1H), 7.0 (dd,1H), &.1 (bd,1H), 7.25 (m,5H), 8.65 (dd,1H); IR ($CDCl_3$) 3420, 2900, 2250, 1740, 1670, 1520, 1290, 1140 $cm^{-1}$. Mass spectrum: $(M+H)^+$ = 467.

Anal. Calcd. for $C_{22}H_{30}N_2O_5S_2$:
C, 56.63; H, 6.48; N, 6.01.
Found:
C, 56.79; H, 6.56; N, 6.01.

## Example 292

### 4-Aza-5(S)-benzyl-6-t-butylsulfonyl-3-oxo-2(R,S)-(4-thiazolylmethyl)hexane carboxamide of (2 S,1 R,5S)-3-ethyl-5-(1 -hydroxy-2 -amino-3 -(cyclohexylpropyl)oxazolidine-2-one.

The ethyl ester (0.20 g, 0.4 mmol) obtained from Example 291 and potassium hydroxide (0.024 g, 0.4 mmol) in 2 ml of absolute ethanol is stirred at room temperature under nitrogen atmosphere for 18 h and concentrated. The resulting acid salt, 1-HOBT (0.043 g, 0.4 mmol), 4-methylmorpholine (0.12 g, 0.40 mmol), and the resultant compound from Example 7 which was deprotected as described in Example 30 were dissolved in 5 ml DMF and coupled according to the procedure of Example 30. The title compound is isolated after silica gel column chromatography.

## Example 293

### Ethyl 4-Aza-5(R)-benzyl-6-t-butylsulfonyl-3-oxo-2(R,S)-(4-thiazdylmethyl)hexanoate.

Using the procedure for the synthesis of the ester in Example 291, but replacing tert-butyl-(2(S)-2-amino-3-phenylpropyl)sulfone hydrochloride with tert-butyl (2(R)-2-amino-3-phenylpropyl)sulfone hydrochloride gave 0.74 g (83%) of a white solid. $^1H$ NMR ($CDCl_3$, TMS) δ 1.2 (td,3H), 1.35 (d,9H), 1.8 (bs,2H($H_2O$)), 3.0-3.3 (m,4H), 3.35 (dd,2H), 3.75 (t,1H), 4.15 (m,4H), 4.55 (bm,1H), 7.0 (dd,1H), 7.25 (m,5H), 8.7 (dd,1H); IR ($CDCl_3$) 3420, 2900, 2250, 1740, 1670, 1520, 1290, 1140 $cm^{-1}$. Mass spectrum: $(M+H)^+$ = 467.

Anal. Calcd. for $C_{22}H_{30}N_2O_5S_2$:
C, 56.63; H, 6.48; N, 6.01.
Found:
C, 56.64; H, 6.54; N, 5.99.

## Example 294

### 4-Aza-5(R)-benzyl-6-t-butylsulfonyl-3-oxo-2(R,S)-(4-thiazolylmethyl)hexane carboxamide of (2 S,1 R,5S)-3-ethyl-5-(1 -hydroxy-2 -amino-3 -(cyclohexylpropyl)oxazolidine-2-one.

Using the procedure for the synthesis of the title compound in Example 292, but substituting the ethyl ester in Example 293 for the ethyl ester in Example 291 gives the product after chromatography purification.

Example 295

Diethyl 2-Flouro-2-(4-thiazolylmethyl)malonate.

Sodium metal (0.15 g, 6.34 mmol) was added to 25 ml of absolute ethanol, cooled in an ice-water bath, and stirred under nitrogen until the metal had completely dissolved. Diethyl 2-fluoromalonate (1.13 g, 6.34 mmol) was added, and the solution was warmed to room temperature and stirred for 2 h. The ice bath was reapplied, and 4-chloromethylthiazole (0.85 g, 6.34 mmol) in 5 ml of ethanol was added. The solution was stirred for 18 h at room temperature, concentrated, and partitioned between 25 ml $H_2O$ and 25 ml $CH_2Cl_2$. The organic layer was filtered through $MgSO_4$ and taken to dryness. The crude product was chromatographed on silica gel with 10% EtOAc/hex to give 1.52 g (87%) of a colorless oil. $^1$H NMR ($CDCl_3$, TMS) $\delta$ 1.3 (t,6H), 3.75 (d,2H), 4.3 (q,4H), 7.22 (t,1H), 8.72 (d,1H); IR ($CDCl_3$) 1750(c=O,ester) $cm^{-1}$. Mass spectrum: $(M+H)^+$ = 276.
Anal. Calcd. for $C_{11}H_{14}NO_4SF$:
C, 47.99; H, 5.13; N, 5.09.
Found:
C, 47.84; H, 5.19; N, 4.80.

Example 296

Ethyl 4-Aza-5(S)-benzyl-6-t-butylsulfonyl-2-fluoro-3-oxo-2(R,S)-(4-thiazolylmethyl)hexanoate.

Using the procedure for the synthesis of the ethyl ester in Example 291 and substituting the product from Example 295, the title compound was obtained in 92% yield. $^1$H NMR ($CDCl_3$, TMS) $\delta$ 1.25 (m,3H), 1.35 (d,9H), 3.0-3.3 (m,5H), 3.75 (dt,2H), 4.25 (m,2H), 4.65 (m,1H), 7.1-7.35 (m,6HO, 8.65 (t,1H); IR ($CDCl_3$) 3420, 2900, 2250, 1740, 1670, 1520, 1120, 1060 $cm^{-1}$. Mass spectrum: $(M+H)^+$ = 485.
Anal. Calcd. for $C_{22}H_{29}N_2O_5S_2F$:
C, 54.52; H, 6.03; N, 5.78.
Found:
C, 54.72; H, 6.10; N, 5.76.

Example 297

4-Aza-5(S)-benzyl-6-t-butylsulfonyl-2-fluoro-3-oxo-2(R,S)-(4-thiazolylmethyl)hexane carboxamide of (2 S,1 R,5S)-3-ethyl-5-(1 -hydroxy-2 -amino-3 -cyclohexylpropyl) oxazolidine-2-one.

Using the procedure for the synthesis of the title compound in Example 292 but substituting the ethyl ester in Example 296 gives the product.

Example 298

Ethyl 4-Aza-5(R)-benzyl-6-t-butylsulfonyl-2-fluoro-3-oxo-2(R,S)-(4-thiazolylmethyl)hexanoate.

Using the procedure for the synthesis of the ester in Example 296 but substituting tert-butyl (2(S)-2-amino-3-phenylpropyl)sulfone hydrochloride with tert-butyl (2(R)-2-amino-3-phenylpropyl)sulfone hydrochloride gave 90% yield of the title compound. $^1$H NMR ($CDCl_3$, TMS) $\delta$ 1.25 (dt,3H), 1.4 (d,9H), 2.9-3.3 (m,5H), 3.75 (dt,2H), 4.25 (m,2H), 4.65 (m,1H), 7.1-7.35 (m,6H), 8.68 (t,1H); IR ($CDCl_3$) 3420, 2900, 2250, 1740, 1670, 1520, 1120, 1060 $cm^{-1}$. Mass spectrum: $(M+H)^+$ = 485.

Anal. Calcd. for $C_{22}H_{29}N_2O_5S_2F$:
C, 54.52; H, 6.03; N, 5.78.
Found:
C, 54.68; H, 6.10; N, 5.79.

## Example 299

4-Aza-5(R)-benzyl-6-t-butylsulfonyl-2-fluoro-3-oxo-2(R,S)-(4-thiazolylmethyl)hexane carboxamide of (2 S,1 R,5S)-3-ethyl-5-(1 -hydroxy-2 -amino-3 -cyclohexylpropyl)oxazolidine-2-one.

Using the procedure for the synthesis of the compound in Example 292 but substituting the ethyl ester in Example 298 gives the product.

## Example 300

3(S)-Acetoxy-2(R,S)-benzyl-3-morpholinocarbonylpropionic Acid.

To a solution of 0.55 g (1.9 mmol) of diethyl (2S,3R,S)-3-benzyl-2-hydroxysuccinate, prepared according to the procedure of D. Seebach, Org. Syn, 63, 109, in 8 ml of tetrahydrofuran, cooled in an ice-water bath was added 220 mg (5.2 mmol) of lithium hydroxide meno hydrate in 8 ml of water. The bath was removed and the reaction stirred for 18 h. The reaction mixture was acidified with concentrated HCl until pH 4 and the solvents removed under high vacuum to give a white solid. The crude diacid was warmed to 50° C in 4 ml of 1:1 acetic anhydride:acetyl chloride for 3 h. The excess acetic anhydride-acetyl chloride was removed under high vacuum. The residue was dissolved in dichloromethane, cooled in an ice-water bath, and 2 ml of morpholine was added. The reaction was stirred for 1 h, diluted with 1 N HCl, and the crude product extracted with chloroform. The combined chloroform extracts were dried ($MgSO_4$), filtered, and concentrated to give a brown oil. The product was purified by silica gel chromatography using 5:95:0.02 methanol:dichloromethane:acetic acid as eluant. Mass spectrum: $(M+H)^+$ = 336.

## Example 301

3(S)-Acetoxy-2(R,S)-benzyl-3-morpholinocarbonylpropionyl-L-(4-thiazolyl)ala Amide of (2′S,1′R,5S)-3-ethyl-5-(1 -hydroxy-2 -amino-3 -cyclohexylpropyl)oxazolidin-2-one.

Using the procedure of Example 30 with the resultant compound from Example 169 and replacing Boc-His-OH with the resultant compound from Example 300 gave the desired product.

Example 302

3(S)-Hydroxy-2(R,S)-benzyl-3-morpholinocarbonylpropionyl-L-(4-thiazolyl)ala Amide of (2′S,1′R,5S)-3-ethyl-5-(1′-hydroxy-2′-amino-3′-cyclohexylpropyl)oxazolidin-2-one.

Using the procedure of Example 211 with the resultant compound from Example 301 gave the desired product.

Example 303

Boc-L-(4-thiazolyl)ala-L-(4-thiazolyl)ala Amide of (2′S,1′R,5S)-3-ethyl-5-(1′-hydroxy-2′-amino-3′-cyclohexylpropyl)oxazolidin-2-one.

Using the procedure of Example 169 with the resultant compound from Example 169 and replacing Boc-His-OH with Boc-DL-(4-thiazolyl)ala-OH gave, after isomer separation on silica gel, the desired product.

Example 304

(4S,5R,6S)-1N-Methyl-6-(t-butyloxycarbonylamino)-7-cyclohexyl-4,5-dihydroxyhept-1-ene-2-carboxamide.

Using the procedure of Kempf (J. Org. Chem. 1986, 51, 3921), the dilithium derivative of N-methylmethacrylamide was condensed with the resultant compound of Example 270 to give, after silica gel chromatography using 2:1 hexane/ethyl acetate, the desired compound in 30% yield. $^1$H NMR($CDCl_3$) δ 0.8-1.7(br envelope), 1.48(s,9H), 1.53(s,3H), 1.59(s,3H), 1.97(br d,1H), 2.38(dd,J = 14,8Hz,1H), 2.79-(dd,J = 14,2Hz,1H), 2.90(d,J = 5Hz,3H), 3.58(m,2H), 4.16(m,1H), 5.47(s,1H), 5.63(s,1H), 6.12(br,1H). Mass spectrum: $(M+H)^+$ = 425.

Example 305

Boc-Phe-His Amide of (4S,5R,6S)-(2N)-Methyl-6-amino-7-cyclohexyl-4,5-dihydroxyhept-1-ene-2-carboxamide.

Using the procedure of Example 30 with the resultant compound of Example 304 and using Boc-Phe-His-OH in place of Boc-His-OH gave the desired compound. Mass spectrum: $(M+H)^+$ = 669.

Anal. Calcd for $C_{35}H_{52}N_6O_7 \bullet 0.75H_2O$:
C,61.61;H,7.90;N,12.32.
Found:
C,61.52;H,7.58;N,12.13.

The compounds of the present invention can be used in the form of salts derived from inorganic or organic acids. These salts include but are not limited to the following: acetate, adipate, alginate, citrate, aspartate, benzoate, benzenesulfonate, bisulfate, butyrate, camphorate, camphorsulfonate, digluconate, cyclopentanepropionate, dodecylsulfate, ethanesulfonate, glucoheptonate, glycerophosphate, hemisulfate, heptonate, hexanoate, fumarate, hydrochloride, hydrobromide, hydroiodide, 2-hydroxy-ethanesulfonate, lactate, maleate, methanesulfonate, nicotinate, 2-naphthalenesulfonate, oxalate, pamoate, pectinate, persulfate, 3-phenylpropionate, picrate, pivalate, propionate, succinate, tartrate, thiocyanate, tosylate, and undecanoate. Also, the basic nitrogen-containing groups can be quaternized with such agents as loweralkyl halides, such as methyl, ethyl, propyl, and butyl chloride, bromides, and iodides; dialkyl sulfates like dimethyl, diethyl, dibutyl, and diamyl sulfates, long chain halides such as decyl, lauryl, myristyl and stearyl chlorides, bromides and iodides, aralkyl halides like benzyl and phenethyl bromides, and others. Water or oil-soluble or dispersible products are thereby obtained.

Examples of acids which may be employed to form pharmaceutically acceptable acid addition salts include such inorganic acids as hydrochloric acid, sulphuric acid and phosphoric acid and such organic acids as oxalic acid, maleic acid, succinic acid and citric acid. Other salts include salts with alkali metals or alkaline earth metals, such as sodium, potassium, calcium or magnesium or with organic bases.

The compounds of the present invention can also be used in the form of esters. Examples of such esters include a hydroxyl-substituted compound of formula I which has been acylated with a blocked or unblocked amino acid residue, a phosphate function, or a hemisuccinate residue. The amino acid esters of particular interest are glycine and lysine; however, other amino acid residues can also be used. These esters serve as pro-drugs of the compound of the present invention and serve to increase the solubility of these substances in the gastrointestinal tract. The preparation of the pro-drug esters is carried out by reacting a hydroxyl-substituted compound of formula I with an activated amino acyl, phosphoryl or hemisuccinyl derivative. The resulting product is then deprotected to provide the desired pro-drug ester.

The novel compounds of the present invention possess an excellent degree of activity and specificity in treating hypertension in a host. The ability of the compounds of the invention to inhibit human renal renin can be demonstrated in vitro by reacting a selected compound at varied concentrations with human renal renin, free from acid proteolytic activity, and with renin substrate (human angiotensinogen) at 37 degrees C and pH of 6.0. At the end of the incubation, the amount of angiotensin I formed is measured by radioimmunoassay and the molar concentration required to cause 50% inhibition, expressed as the $IC_{50}$, is calculated. When tested in accordance with the foregoing procedure, the compounds of the invention demonstrated $IC_{50}$'s in the range of $10^{-6}$ to $10^{-10}$ M as seen in Table I.

Table I

| Example | $IC_{50}$(nM) |
|---|---|
| 107 | 1.5 |
| 113 | 0.9 |
| 114 | 33 |
| 115 | 3.1 |
| 116 | 0.7 |
| 118 | 0.63 |
| 120 | 35 |
| 121 | 12 |
| 123 | 0.50 |
| 124 | 6.3 |
| 125 | 1.8 |
| 127 | 0.60 |
| 128 | 29 |
| 130 | 2.5 |
| 132 | 54 |
| 134 | 7.9 |
| 138 | 1.7 |
| 141 | 0.67 |
| 142 | 0.73 |
| 143 | 0.50 |
| 144 | 0.44 |
| 145 | 7.9 |
| 146 | 6.9 |
| 147 | 2.1 |
| 148 | 0.48 |
| 149 | 4.6 |
| 152 | 0.63 |
| 153 | 0.87 |
| 154 | 1.1 |
| 157 | 240 |

| | |
|---|---|
| 235 | 0.6 |
| 236 | 1.1 |
| 237 | 1.5 |
| 238 | 0.74 |
| 239 | 1.2 |
| 255 | 0.33 |
| 264 | 1.4 |
| 277 | 0.71 |
| 278 | 0.32 |
| 279 | 2.4 |
| 280 | 1.9 |
| 281 | 1.1 |
| 282 | 1.0 |
| 283 | 1.2 |
| 284 | 1.5 |
| 286 | 0.5 |

The ability of the compounds of the invention to inhibit renin in vivo can be demonstrated in salt depleted monkeys using the following protocol:

Protocol

1. Dose monkeys with furosemide (@ 30 mg/kg ± 2 mg, PO) via banana, 8 days prior to experiment.
2. Start low sodium and fruit diet 7 days prior to experiment.
3. Dose monkeys again, (@ 30 mg/kg ± mg, PO) via banana, 1 day prior to experiment.
4. Starve monkeys the night before, and morning of the experiment.
5. Pre-anesthetize monkeys with ketamine (10 mg/kg, IM).
6. Place IV catheter in rear of one leg via saphenous vein.
7. Anesthetize monkeys with nembutal (15 mg/kg, IV) with bolus injection via IV catheter.
8. Maintenance infusion of 0.09 mg/kg/min of nembutal IV. 0.9 x body weight (kg) = ml of 50mg/ml nembutal QS to 50 ml with heparanized (20 units/ml) 5% dextrose Ing, USP (D5W).
9. Femoral artery canulated and advanced into aorta for direct measurement of pulsatile blood pressure via a statham transducer connected to a grass polygraph. All data was monitored digitally via output from the polygraph to an on-line computer system.
10. Animals were monitored for a stable blood pressure tracing before control blood samples were drawn.
11. Compound is given (@ 0.1 mg/kg, IV) after control blood samples are drawn.
12. Animals were monitored for 180 minutes, or until animal recovered to initial blood pressure level, post drug administration.
13. Animals were given captopril (0.1 mg/kg IV), as a verification of a hypotensive effect in each animal.

When tested in accordance with the foregoing procedure, the compounds of the invention demonstrated the effects shown in Table II.

Table II
Effects of Renin Inhibitor (0.1 mg/kg, i.v.)
in Salt-Depleted Monkeys

| | | Time following drug administration (minutes) | | | | | | |
|---|---|---|---|---|---|---|---|---|
| | Example | 0 | 5 | 15 | 30 | 60 | 120 | 180 |
| Mean Arterial Pressure (mmHg) | 148 | 73.76 | 58.3 | 49.92 | 44.86 | 45.12 | 63.14 | 72.26 |
| | 146 | 83.2 | 74.0 | 73.6 | 72.4 | 78.3 | 84.1 | 84.0 |
| | 125 | 64.8 | 58.9 | [illegible] | [illegible] | 57.4 | 69.6 | 71.1 |
| | 149 | 66.5 | 60.0 | 59.1 | 62.8 | 66.8 | 70.1 | 73.8 |
| Heart Rate (beats/min.) | 148 | 143.7 | 150.02 | 152.04 | 153.04 | 155.8 | 162.68 | 165.94 |
| | 146 | 165.4 | 172.0 | 167.3 | 168.8 | 163.3 | 171.0 | 172.3 |
| | 125 | 140.2 | 144.3 | 145.2 | 144.1 | 148.3 | 159.7 | 163.9 |
| | 149 | 135.7 | 136.3 | 138.4 | [illegible] | 141.0 | 148.4 | 154.8 |
| Plasma Renin Activity (ng/mL plasma/hr.) | 148 | 54.66 | 2.16 | 2.88 | 5.22 | 14.94 | 39.96 | 51.16 |
| | 146 | 144.3 | 5.9 | 24.0 | 58.7 | 114.5 | 170.6 | 197.0 |
| | 125 | 94.8 | 2.7 | 11.7 | 31.4 | 58.5 | 102.8 | 117.2 |
| | 149 | 6.15 | 1.35 | 5.4 | 7.8 | 8.55 | 10.2 | 11.1 |

The values for example 148 represents the average of the results for five different monkeys tested. The values for each of examples 146, 125 and 149 represent the average of the results for two different monkeys tested. These results demonstrate hypotension and suppression of plasma renin activity, without reflex tachycardia, induced by intravenous administration of the compounds.

The compounds of the invention may also be used with one or more antihypertensive agents selected from the group consisting of diuretics, and/or β-adrenergic blocking agents, central nervous system-acting agents, adrenergic neuron blocking agents, vasodilators, angiotensin I converting enzyme inhibitors, and other antihypertensive agents.

Total daily dose administered to a host in single or divided doses may be in amounts, for example, from 0.001 to 10 mg/kg body weight daily and more usually 0.01 to 10 mg. Dosage unit compositions may contain such amounts of submultiples thereof to make up the daily dose.

The amount of active ingredient that may be combined with the carrier materials to produce a single dosage form will vary depending upon the host treated and the particular mode of administration.

It will be understood, however, that the specific dose level for any particular patient will depend upon a variety of factors including the activity of the specific compound employed, the age, body weight, general health, sex, diet, time of administration, route of administration, rate of excretion, drug combination, and the severity of the particular disease undergoing therapy.

The compounds of the present invention may be administered orally, parenterally, by inhalation spray, rectally, or topically in dosage unit formulations containing conventional nontoxic pharmaceutically acceptable carriers, adjuvants, and vehicles as desired. Topical administration may also involve the use of transdermal administration such as transdermal patches or iontophoresis devices. The term parenteral as used herein includes subcutaneous injections, intravenous, intramuscular, intrasternal injection, or infusion techniques.

Injectable preparations, for example, sterile injectable aqueous or oleagenous suspensions may be formulated according to the known art using suitable dispersing or wetting agents and suspending agents. The sterile injectable preparation may also be a sterile injectable solution or suspension in a nontoxic parenterally acceptable diluent or solvent, for example, as a solution in 1,3-butanediol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

Suppositories for rectal administration of the drug can be prepared by mixing the drug with a suitable nonirritating excipient such as cocoa butter and polyethylene glycols which are solid at ordinary temperatures but liquid at the rectal temperature and will therefore melt in the rectum and release the drug.

Solid dosage forms for oral administration may include capsules, tablets, pills, powders, and granules. In such solid dosage forms, the active compound may be admixed with at least one inert diluent such as sucrose lactose or starch. Such dosage forms may also comprise, as is normal practice, additional substances other than inert diluents, e.g., lubricating agents such as magnesium stearate. In the case of capsules, tablets, and pills, the dosage forms may also comprise buffering agents. Tablets and pills can additionally be prepared with enteric coatings.

Liquid dosage forms for oral administration may include pharmaceutically acceptable emulsions, solutions, suspensions, syrups, and elixirs containing inert diluents commonly used in the art, such as

water. Such compositions may also comprise adjuvants, such as wetting agents, emulsifying and suspending agents, and sweetening, flavoring, and perfuming agents.

The foregoing is merely illustrative of the invention and is not intended to limit the invention to the disclosed compounds. Variations and changes which are obvious to one skilled in the art are intended to be within the scope and nature of the invention which are defined in the appended claims.

## Claims

1. A compound of the formula:

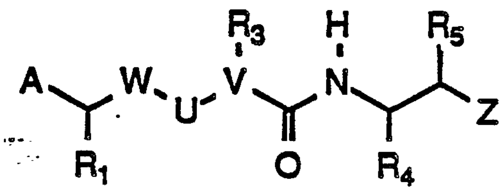

wherein A is hydrogen, loweralkyl, aminoalkyl, (alkyl)aminoalkyl, dialkylaminoalkyl, (alkoxy)aminoalkyl, (alkoxy)(alkyl)aminoalkyl, phenylalkyl, (substituted phenyl)alkyl wherein the phenyl ring is substituted with one, two or three substituents independently selected from loweralkoxy, loweralkyl, amino, alkylamino, dialkylamino, hydroxy, halo, mercapto, nitro, thioalkoxy, carboxaldehyde, carboxy, alkoxycarbonyl and carboxamide, naphthylalkyl, (substituted naphthyl)alkyl wherein the naphthyl ring is substituted with one, two or three substituents independently selected from loweralkoxy, loweralkyl, amino, alkylamino, dialkylamino, hydroxy, halo, mercapto, nitro thioalkoxy, carboxaldehyde, carboxy, alkoxycarbonyl and carboxamide, substituted or unsubstituted heterocyclic, where saturated heterocyclics may be unsubstituted, monosubsituted or disubstituted with hydroxy, oxo, amino, alkylamino, dialkylamino, alkoxy, polyalkoxy, loweralkyl, haloalkyl or polyhaloalkyl; unsaturated heterocyclics may be unsubstituted or monosubstituted with hydroxy, amino, alkylamino, dialkylamino, alkoxy, polyalkoxy, loweraklyl, haloalkyl or polyhaloalkyl, or A is (unsubstituted heterocyclic)alkyl or (substituted heterocyclic)alkyl wherein unsubstituted or substituted heterocyclic is as defined above, or A is $-OR_7$ or $-SR_7$ wherein $R_7$ is hydrogen, loweralkyl, aminoalkyl, (alkyl)aminoalkyl, dialkylaminoalkyl, (alkoxy)aminoalkyl, (alkoxy)(alkyl)aminoalkyl, phenylalkyl, (substituted phenyl)alkyl wherein substituted phenyl is as defined above, naphthylalkyl, (substituted naphthyl)alkyl wherein the substituted naphthyl is as defined above, substituted or unsubstituted heterocyclic as defined above, (unsubstituted heterocyclic)alkyl or (substituted heterocyclic)alkyl wherein unsubstituted or substituted heterocyclic is as defined above, (unsubstituted heterocyclic)C(O)- or (substituted heterocyclic)C(O)- wherein unsubstituted or substituted heterocyclic is as defined above; or A is $-NR_8R_9$ wherein $R_8$ and $R_9$ are independently selected from hydrogen, hydroxy, alkoxy, loweralkyl, aminoalkyl, cyanoalkyl and hydroxyalkyl; or A is

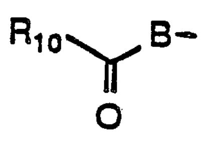

or

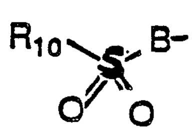

wherein B is NH, alkylamino, S, O, $CH_2$, $NHCH_2$ or $CH(OR_{52})$ wherein $R_{52}$ is hydrogen, loweralkyl or loweralkylcarbonyl, and $R_{10}$ is hydrogen, loweralkyl, cycloalkyl, phenyl, substituted phenyl as defined above, naphthyl, substituted naphthyl as defined above, alkoxy, alkenyloxy, hydroxyalkoxy, dihydroxyalkoxy, phenylalkoxy, (substituted phenyl)alkoxy wherein substituted phenyl is as defined above, naphthylalkoxy, (substituted naphthyl)alkoxy wherein substituted naphthyl is as defined above, phenylalkoxyalkyl, (substituted phenyl)alkoxyalkyl wherein substituted phenyl is as defined above, naphthylalkoxyalkyl, (substituted naphthyl)alkoxyalkyl wherein substituted naphthyl is as defined above, thioalkoxyalkyl, loweralkylsulfinylalkyl, loweralkylsulfonylalkyl, phenylthioalkyl, (substituted phenyl)thioalkyl wherein substituted phenyl is as defined above, naphthylthioalkyl, (substituted naphthyl)thioalkyl wherein substituted naphthyl is as defined above, phenylsulfonylalkyl, (substituted phenyl)sulfonylalkyl wherein substituted phenyl is as defined above, naphthylsulfonylalkyl, (substituted naphthyl)sulfonylalkyl wherein substituted naphthyl is as

defined above, amino, alkylamino, dialkylamino, (hydroxyalkyl)(alkyl)amino, (dihydroxyalkyl)(alkyl)amino, aminoalkyl, alkoxycarbonylalkyl, carboxyalkyl, (N-protected)-aminoalkyl, alkylaminoalkyl, (N-protected)-(alkyl)aminoalkyl, dialkylaminoalkyl, (heterocyclic)alkyl, a substituted or unsubstituted heterocyclic as defined above, aminocycloalkyl, aminoalkylamino, (dialkylaminoalkyl)(alkyl)amino, phenylalkylamino, (substituted phenyl)alkylamino wherein substituted phenyl is as defined above, naphthylalkylamino, (substituted naphthyl)alkylamino wherein substituted naphthyl is as defined above, (phenylalkyl)(alkyl)amino, ((substituted phenyl)alkyl)(alkyl)amino wherein substituted phenyl is as defined above, (naphthylalkyl)(alkyl)-amino, ((substituted naphthyl)alkyl)(alkyl)amino wherein substituted naphthyl is as defined above, alkoxyalkyl(alkyl)amino, (polyalkoxy)alkyl(alkyl)amino, di-(alkoxyalkyl)amino, di-(hydroxyalkyl)amino, di-((polyalkoxy)alkyl)amino, ((heterocyclic)alkyl)(alkyl)amino, ((heterocyclic)alkyl)amino, (heterocyclic)(alkyl)-amino, (alkylaminoalkyl)(alkyl)amino, (dialkylaminoalkyl)(alkyl)amino, ((alkoxy)(alkyl)aminoalkyl)(alkyl)amino, ((alkoxy)aminoalkyl)(alkyl)amino, polyalkoxy or (polyalkoxy)alkyl; or A is $R_{41}CH(OH)CH_2$- or $R_{41}CH(OH)CH(OH)$- wherein $R_{41}$ is loweralkyl, cycloalkyl, phenyl, substituted phenyl as defined above, naphthyl, substituted naphthyl as defined above, phenylalkyl, (substituted phenyl)alkyl wherein substituted phenyl is as defined above, naphthylalkyl, (substituted naphthyl)alkyl wherein substituted naphthyl is as defined above, phenylalkoxyalkyl, (substituted phenyl)alkoxyalkyl wherein substituted phenyl is as defined above, naphthylalkoxyalkyl, (substituted naphthyl)alkoxyalkyl wherein substituted naphthyl is as defined above, thioalkoxyalkyl, loweralkylsulfinylalkyl, loweralkylsulfonylalkyl, phenylthioalkyl, (substituted phenyl)thioalkyl wherein substituted phenyl is as defined above, naphthylthioalkyl, (substituted naphthyl)thioalkyl wherein substituted naphthyl is as defined above, phenylsulfonylalkyl, (substituted phenyl)sulfonylalkyl wherein substituted phenyl is as defined above, naphthylsulfonylalkyl, (substituted naphthyl)sulfonylalkyl wherein substituted naphthyl is as defined above, aminoalkyl, alkoxycarbonylalkyl, carboxyalkyl, (N-protected)-aminoalkyl, alkylaminoalkyl, (N-protected)(alkyl)aminoalkyl, dialkylaminoalkyl, heterocyclicalkyl, a substituted or unsubstituted heterocyclic as defined above, aminocycloalkyl or (polyalkoxy)alkyl;

W is C=O, CHOH or $NR_2$ wherein $R_2$ is hydrogen or loweralkyl;

U is C=O, $CH_2$ or $NR_2$ wherein $R_2$ is hydrogen or loweralkyl, with the proviso that when W is CHOH then U is $CH_2$ and with the proviso that U is C=O or $CH_2$ when W is $NR_2$;

V is CH, C(OH) or C(halogen) with the proviso that V is CH when U is $NR_2$;

$R_1$ is loweralkyl, cycloalkylalkyl, benzyl, (alpha, alpha)-dimethylbenzyl, 4-methoxybenzyl, halobenzyl, 4-hydroxybenzyl, (1-naphthyl)methyl, (2-naphthyl)methyl, (unsubstituted heterocyclic)methyl, (substituted heterocyclic)methyl wherein unsubstituted or substituted heterocyclic is as defined above, phenethyl, 1-benzyloxyethyl, phenoxy, thiophenoxy or anilino, provided that B is $CH_2$ or CHOH or A is hydrogen when $R_1$ is phenoxy, thiophenoxy or anilino;

$R_3$ is loweralkyl, loweralkenyl, ((alkoxy)alkoxy)alkyl, carboxyalkyl, (thioalkoxy)alkyl, azidoalkyl, aminoalkyl, (alkyl)aminoalkyl, dialkylaminoalkyl, (alkoxy)(alkyl)aminoalkyl, (alkoxy)aminoalkyl, benzyl or heterocyclic ring substituted methyl;

$R_4$ is loweralkyl, cycloalkylmethyl or benzyl;

$R_5$ is OH or $NH_2$; and

Z is

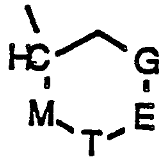 or 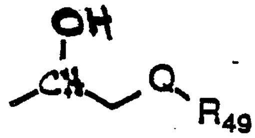

wherein M is O, S or NH, T is C=O, C=S, S, S(O), $S(O)_2$ or $CH_2$, E is O, S, $NR_6$ wherein $R_6$ is hydrogen, loweralkyl, hydroxyalkyl, hydroxy, alkoxy, amino, or alkylamino, or E is $CR_6R_{42}$ wherein $R_6$ is as defined above and $R_{42}$ is hydrogen or loweralkyl or E is $C=CR_{43}R_{44}$ wherein $R_{43}$ and $R_{44}$ are independently

selected from hydrogen and loweralkyl, G is absent, $CH_2$, or $NR_{11}$ wherein $R_{11}$ is hydrogen or loweralkyl, with the proviso that when G is $NR_{11}$ then $R_6$ is loweralkyl or hydroxyalkyl, Q is $CR_{45}R_{46}$ wherein $R_{45}$ and $R_{46}$ are independently selected from hydrogen and loweralkyl or Q is $C=CR_{47}R_{48}$ wherein $R_{47}$ and $R_{48}$ are independently selected from hydrogen and loweralkyl, and $R_{49}$ is $-CH_2OH$, carboxy, alkoxycarbonyl or $-CONR_{50}R_{51}$ wherein $R_{50}$ is hydrogen or loweralkyl and $R_{51}$ is hydrogen, loweralkyl, aminoalkyl, alkylaminoalkyl, dialkylaminoalkyl or alkoxyalkyl; or pharmaceutically acceptable salts or esters thereof.

2. The compounds of claim 1 wherein the heterocyclic is:

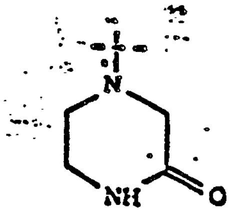 , 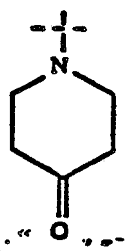 , 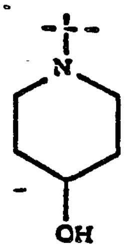 , 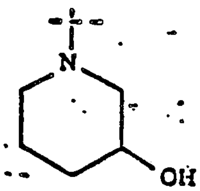 ,

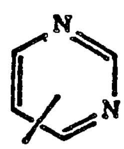 , 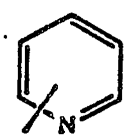 , 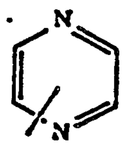 , 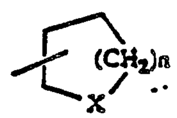 , 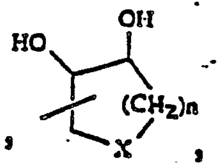 ,

wherein n is 1 or 2 and X is N, NH, O, S, provided that X is the point of connection only when X is N,

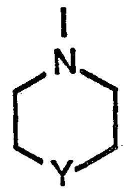

wherein Y is NH, N-loweralkyl, O, S, or $SO_2$, or

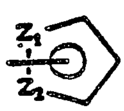 , 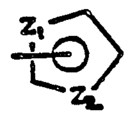 , 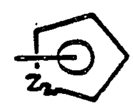 ,

wherein $Z_1$ is N, O, or S and not the point of connection and $Z_2$ is N when it is the point of connection and NH, O or S when it is not the point of connection.

3. The compounds of Claim 1 wherein $R_1$ is benzyl, $R_3$ is (4-imidazolyl)methyl, $R_4$ is cyclohexylmethyl, $R_5$ is OH, Z is

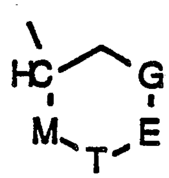

wherein M is O, T is C=O and E in $NR_6$ wherein $R_6$ is hydrogen, loweralkyl, hydroxyalkyl, hydroxy, alkoxy, amino or alkylamino, or wherein M is O, T is $CH_2$, G is absent and E is $CH(CH_3)$, or Z is

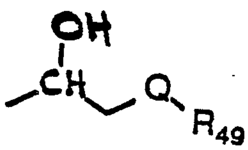

wherein Q is $CR_{45}R_{46}$ wherein $R_{45}$ and $R_{46}$ are independently selected from hydrogen and loweralkyl or Q is $C=CR_{47}R_{48}$ wherein $R_{47}$ and $R_{48}$ are independently selected from hydrogen and loweralkyl, and $R_{49}$ is $-CH_2OH$, carboxy, alkoxycarbonyl or $-CONR_{50}R_{51}$ wherein $R_{50}$ is hydrogen or loweralkyl and $R_{51}$ is hydrogen, loweralkyl, aminoalkyl, alkylaminoalkyl, dialkylaminoalkyl or alkoxyalkyl.

4. A compound selected from the group consisting of:
(2R)-2-Benzyl-3-morpholin-4-ylcarbonyl-propionyl-L-(4-thiazolyl)-Ala Amide of (2′S, 1′R, 5S)-3-ethyl-5-(1′-hydroxy-2′-amino-3′-cylcohexylpropyl)-oxazolidin-2-one,
(2R)-2-Benzyl-3-(4-trifluoroethyl-piperazin-1-ylcarbonyl)-propionyl-L-(4-thiazolyl)-Ala Amide of (2′S, 1′R, 5S)-3-ethyl-5-(1′-hydroxy-2′-amino-3′-cyclohexylpropyl)-oxazolidin-2-one,
(2R)-2-Benzyl-3-thiazol-4-ylpropionyl-L-(4-thiazolyl)-Ala Amide of (2′S, 1′R, 5S)-3-ethyl-5-(1′-hydroxy-2′-amino-3′-cyclohexylpropyl)oxazolidin-2-one,
(2S)-2-Benzyl-3-tert-butylsulfonyl-propionyl-His Amide of (2′S, 1′R, 5S)-3-ethyl-5-(1′-hydroxy-2′-amino-3′-cyclohexylpropyl)oxazolidin-2-one,
(2R)-2-Benzyl-3-((2-imidazol-1-ylethyl)-methylaminocarbonyl)propionyl-L-(4-thiazolyl)-AlaAmide of (2′S, 1′R, 5S)-3-ethyl-5-(1′-hydroxy-2′-amino-3′-cyclohexylpropyl)oxazolidin-2-one,
(2R)-2-Benzyl-3-((2-L-(4-thiazolyl)-Ala Amide of (2′S, 1′R, 5S)-3-ethyl-5-(1′-hydroxy-2′-amino-3′-cyclohexylpropyl)-oxazolidin-2-one,
and (2R)-2-Benzyl-3-(morpholinocarbonyl)-propionyl-L-(4-thiazolyl)-Ala Amide of (2S, 4S, 1′R, 2′S)-2-(2-amino-3-cyclohexyl-1-hydroxy)-4-methyltetrahydrofuran, or pharmaceutically acceptable salts or esters thereof.

5. A pharmaceutical composition for treating hypertension, comprising a pharmaceutical carrier and a therapeutically effective amount of a compound of claim 1.

6. A pharmaceutical composition for treating hypertension, comprising a pharmaceutical carrier and a therapeutically effective amount of a compound selected from the group consisting of:
(2R)-2-Benzyl-3-morpholin-4-ylcarbonyl-propionyl-L-(4-thiazolyl)-Ala Amide of (2′S, 1′R, 5S)-3-ethyl-5-(1′-hydroxy-2′-amino-3′-cylcohexylpropyl)-oxazolidin-2-one,
(2R)-2-Benzyl-3-(4-trifuluoroethyl-piperazin-1-ylcarbonyl)-propionyl-L-(4-thiazolyl)-Ala Amide of (2′S, 1′R, 5S)-3-ethyl-5-(1′-hydroxy-2′-amino-3′-cyclohexylpropyl)-oxazolidin-2-one,
(2R)-2-Benzyl-3-thiazol-4-ylpropionyl-L-(4-thiazolyl)-Ala Amide of (2′S, 1′R, 5S)-3-ethyl-5-(1′-hydroxy-2′-amino-3′-cyclohexylpropyl)oxazolidin-2-one,
(2S)-2-Benzyl-3-tert-butylsulfonyl-propionyl-His Amide of (2′S, 1′R, 5S)-3-ethyl-5-(1′-hydroxy-2′-amino-3′-cyclohexylpropyl)oxazolidin-2-one,
(2R)-2-Benzyl-3-((2-imidazol-1-ylethyl)-methylaminocarbonyl)propionyl-L-(4-thiazolyl)-AlaAmide of (2′S, 1′R, 5S)-3-ethyl-5-(1′-hydroxy-2′-amino-3′-cyclohexylpropyl)oxazolidin-2-one,
(2R)-2-Benzyl-3-((2-L-(4-thiazolyl)-Ala Amide of (2′S, 1′R, 5S)-3-ethyl-5-(1′-hydroxy-2′-amino-3′-cyclohexylpropyl)-oxazolidin-2-one,
and (2R)-2-Benzyl-3-(morpholinocarbonyl)-propionyl-L-(4-thiazolyl)-Ala Amide of (2S, 4S, 1′R, 2′S)-2-(2-amino-3-cyclohexyl-1-hydroxy)-4-methyltetrahydrofuran, or pharmaceutically acceptable salts or esters thereof.

7. A method for treating hypertension comprising administering to a host in need of such treatment a therapeutically effective amount of a compound of claim 1.

8. A method for treating hypertension comprising administering to a host in need of such treatment a therapeutically effective amount of a compound selected from the group consisting of:
(2R)-2-Benzyl-3-morpholin-4-ylcarbonyl-propionyl-L-(4-thiazolyl)-Ala Amide of (2′S, 1′R, 5S)-3-ethyl-5-(1′-hydroxy-2′-amino-3′-cylcohexylpropyl)-oxazolidin-2-one,
(2R)-2-Benzyl-3-(4-trifluoroethyl-piperazin-1-ylcarbonyl)-propionyl-L-(4-thiazolyl)-Ala Amide of (2′S, 1′R, 5S)-3-ethyl-5-(1′-hydroxy-2′-amino-3′-cyclohexylpropyl)-oxazolidin-2-one,
(2R)-2-Benzyl-3-thiazol-4-ylpropionyl-L-(4-thiazolyl)-Ala Amide of (2′S, 1′R, 5S)-3-ethyl-5-(1′-hydroxy-2′-amino-3′-cyclohexylpropyl)oxazolidin-2-one,
(2S)-2-Benzyl-3-tert-butylsulfonyl-propionyl-His Amide of (2′S, 1′R, 5S)-3-ethyl-5-(1′-hydroxy-2′-amino-3′-cyclohexylpropyl)oxazolidin-2-one,
(2R)-2-Benzyl-3-((2-imidazol-1-ylethyl)-methylaminocarbonyl)propionyl-L-(4-thiazolyl)-AlaAmide of (2′S, 1′R,

5S)-3-ethyl-5-(1′-hydroxy-2′-amino-3′-cyclohexylpropyl)oxazolidin-2-one, (2R)-2-Benzyl-3-((2-L-(4-thiazolyl)-Ala Amide of (2′S, 1′R, 5S)-3-ethyl-5-(1′-hydroxy-2′-amino-3′-cyclohexylpropyl)-oxazolidin-2-one, and (2R)-2-Benzyl-3-(morpholinocarbonyl)-propionyl-L-(4-thiazolyl)-Ala Amide of (2S, 4S, 1′R, 2′S)-2-(2-amino-3-cyclohexyl-1-hydroxy)-4-methyltetrahydrofuran, or pharmaceutically acceptable salts or esters thereof.

9. A compound of the formula:

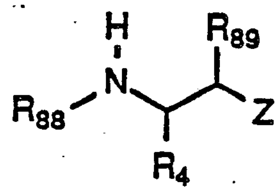

wherein $R_4$ is loweralkyl, cycloalkylmethyl or benzyl;

$R_{88}$ is hydrogen or an N-protecting group;

$R_{89}$ is $-OR_{90}$ or $-NHR_{91}$ wherein $R_{90}$ is hydrogen or an O-protecting group and $R_{91}$ is hydrogen or an N-protecting group; and

Z is

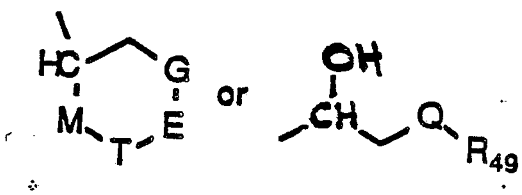

wherein M is O, S or NH, T is C=O, C=S, S, S(O), $S(O)_2$ or $CH_2$, E is O, S, $NR_6$ wherein $R_6$ is hydrogen, loweralkyl, hydroxyalkyl, hydroxy, alkoxy, amino, or alkylamino, or E is $CR_6R_{42}$ wherein $R_6$ is as defined above and $R_{42}$ is hydrogen or loweralkyl or E is $C=CR_{43}R_{44}$ wherein $R_{43}$ and $R_{44}$ are independently selected from hydrogen and loweralkyl, G is absent, $CH_2$, or $NR_{11}$ wherein $R_{11}$ is hydrogen or loweralkyl, with the proviso that when G is $NR_{11}$ then $R_6$ is loweralkyl or hydroxyalkyl, Q is $CR_{45}R_{46}$ wherein $R_{45}$ and $R_{46}$ are independently selected from hydrogen and loweralkyl or Q is $C=CR_{47}R_{48}$ wherein $R_{47}$ and $R_{48}$ are independently selected from hydrogen and loweralkyl, and $R_{49}$ is $-CH_2OH$, carboxy, alkoxycarbonyl or $-CONR_{50}R_{51}$ wherein $R_{50}$ is hydrogen or loweralkyl and $R_{51}$ is hydrogen, loweralkyl, aminoalkyl, alkylaminoalkyl, dialkylaminoalkyl or alkoxyalkyl.

10. A process of making a compound of claim 1 comprising the step of coupling a compound of the formula:

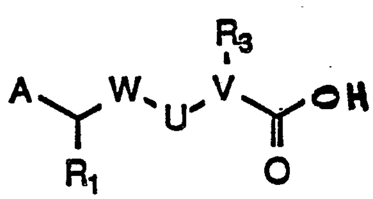

wherein A is hydrogen, loweralkyl, aminoalkyl, (alkyl)aminoalkyl, dialkylaminoalkyl, (alkoxy)aminoalkyl, (alkoxy)(alkyl)aminoalkyl, phenylalkyl, (substituted phenyl)alkyl wherein the phenyl ring is substituted with one, two or three substituents independently selected from loweralkoxy, loweralkyl, amino, alkylamino, dialkylamino, hydroxy, halo, mercapto, nitro, thioalkoxy, carboxaldehyde, carboxy, alkoxycarbonyl and carboxamide, naphthylalkyl, (substituted naphthyl)alkyl wherein the naphthyl ring is substituted with one, two or three substituents independently selected from loweralkoxy, loweralkyl, amino, alkylamino, dialkylamino,

hydroxy, halo, mercapto, nitro, thioalkoxy, carboxaldehyde, carboxy, alkoxycarbonyl and carboxamide, substituted or unsubstituted heterocyclic, where saturated heterocyclics may be unsubstituted, monosubsituted or disubstituted with hydroxy, oxo, amino, alkylamino, dialkylamino, alkoxy, polyalkoxy, loweralkyl, haloalkyl or polyhaloalkyl; unsaturated heterocyclics may be unsubstituted or monosubstituted with hydroxy, amino, alkylamino, dialkylamino, alkoxy, polyalkoxy, loweraklyl, haloalkyl or polyhaloalkyl, or A is (unsubstituted heterocyclic)alkyl or (substituted heterocyclic)alkyl wherein unsubstituted or substituted heterocyclic is as defined above, or A is $-OR_7$ or $-SR_7$ wherein $R_7$ is hydrogen, loweralkyl, aminoalkyl, (alkyl)aminoalkyl, dialkylaminoalkyl, (alkoxy)aminoalkyl, (alkoxy)(alkyl)aminoalkyl, phenylalkyl, (substituted phenyl)alkyl wherein substituted phenyl is as defined above, naphthylalkyl, (substituted naphthyl)alkyl wherein the substituted naphthyl is as defined above, substituted or unsubstituted heterocyclic as defined above, (unsubstituted heterocyclic)alkyl or (substituted heterocyclic)alkyl wherein unsubstituted or substituted heterocyclic is as defined above, (unsubstituted heterocyclic)C(O)- or (substituted heterocyclic)C(O)- wherein unsubstituted or substituted heterocyclic is as defined above; or A is $-NR_8R_9$ wherein $R_8$ and $R_9$ are independently selected from hydrogen, hydroxy, alkoxy, loweralkyl, aminoalkyl, cyanoalkyl and hydroxyalkyl; or A is

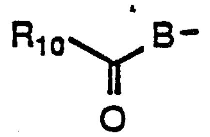

or

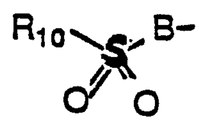

wherein B is NH, alkylamino, S, O, $CH_2$, $NHCH_2$ or $CH(OR_{52})$ wherein $R_{52}$ is hydrogen, loweralkyl or loweralkylcarbonyl, and $R_{10}$ is hydrogen, loweralkyl, cycloalkyl, phenyl, substituted phenyl as defined above, naphthyl, substituted naphthyl as defined above, alkoxy, alkenyloxy, hydroxyalkoxy, dihydroxyalkoxy, phenylalkoxy, (substituted phenyl)alkoxy wherein substituted phenyl is as defined above, naphthylalkoxy, (substituted naphthyl)alkoxy wherein substituted naphthyl is as defined above, phenylalkoxyalkyl, (substituted phenyl)alkoxyalkyl wherein substituted phenyl is as defined above, naphthylalkoxyalkyl, (substituted naphthyl)alkoxyalkyl wherein substituted naphthyl is as defined above, thioalkoxyalkyl, loweralkylsulfinylalkyl, loweralkylsulfonylalkyl, phenylthioalkyl, (substituted phenyl)thioalkyl wherein substituted phenyl is as defined above, naphthylthioalkyl, (substituted naphthyl)thioalkyl wherein substituted naphthyl is as defined above, phenylsulfonylalkyl, (substituted phenyl)sulfonylalkyl wherein substituted phenyl is as defined above, naphthylsulfonylalkyl, (substituted naphthyl)sulfonylalkyl wherein substituted naphthyl is as defined above, amino, alkylamino, dialkylamino, (hydroxyalkyl)(alkyl)amino, (dihydroxyalkyl)(alkyl)amino, aminoalkyl, alkoxycarbonylalkyl, carboxyalkyl, (N-protected)-aminoalkyl, alkylaminoalkyl, (N-protected)-(alkyl)aminoalkyl; dialkylaminoalkyl, (heterocyclic)alkyl, a substituted or unsubstituted heterocyclic as defined above, aminocycloalkyl, aminoalkylamino, (dialkylaminoalkyl) (alkyl)amino, phenylalkylamino, (substituted phenyl)alkylamino wherein substituted phenyl is as defined above, naphthylalkylamino, (substituted naphthyl)alkylamino wherein substituted naphthyl is as defined above, (phenylalkyl)(alkyl)amino, ((substituted phenyl)alkyl)(alkyl)amino wherein substituted phenyl is as defined above, (naphthylalkyl)(alkyl)-amino, ((substituted naphthyl)alkyl)(alkyl)amino wherein substituted naphthyl is as defined above, alkoxyalkyl(alkyl)amino, (polyalkoxy)alkyl(alkyl)amino, di-(alkoxyalkyl)amino, di-(hydroxyalkyl)amino, di-((polyalkoxy)alkyl)amino, ((heterocyclic)alkyl)(alkyl)amino, ((heterocyclic)alkyl)amino, (heterocyclic)(alkyl)-amino, (alkylaminoalkyl)(alkyl)amino, (dialkylaminoalkyl)(alkyl)amino, ((alkoxy)(alkyl)aminoalkyl)(alkyl)amino, ((alkoxy)aminoalkyl)(alkyl)amino, polyalkoxy or (polyalkoxy)alkyl; or A is $R_{41}CH(OH)CH_2$- or $R_{41}CH(OH)CH(OH)$- wherein $R_{41}$ is loweralkyl, cycloalkyl, phenyl, substituted phenyl as defined above, naphthyl, substituted naphthyl as defined above, phenylalkyl, (substituted phenyl)alkyl wherein substituted phenyl is as defined above, naphthylalkyl, (substituted naphthyl)alkyl wherein substituted naphthyl is as defined above, phenylalkoxyalkyl, (substituted phenyl)alkoxyalkyl wherein substituted phenyl is as defined above, naphthylalkoxyalkyl, (substituted naphthyl)alkoxyalkyl wherein substituted naphthyl is as defined above, thioalkoxyalkyl, loweralkylsulfinylalkyl, loweralkylsulfonylalkyl, phenylthioalkyl, (substituted phenyl)thioalkyl wherein substituted phenyl is as defined above, naphthylthioalkyl, (substituted naphthyl)thioalkyl wherein substituted naphthyl is as defined above, phenylsulfonylalkyl, (substituted phenyl)sulfonylalkyl wherein substituted phenyl is as defined above, naphthylsulfonylalkyl, (substituted naphthyl)sulfonylalkyl wherein substituted naphthyl is as defined above, aminoalkyl, alkoxycarbonylalkyl, carboxyalkyl, (N-protected)-aminoalkyl, alkylaminoalkyl, (N-protected)(alkyl)aminoalkyl, dialkylaminoalkyl, heterocyclicalkyl, a substituted or unsubstituted heterocyclic as defined above, aminocycloalkyl or (polyalkoxy)alkyl;

W is C=O, CHOH or $NR_2$ wherein $R_2$ is hydrogen or loweralkyl;

U is C=O, $CH_2$ or $NR_2$ wherein $R_2$ is hydrogen or loweralkyl, with the proviso that when W is CHOH then U is $CH_2$ and with the proviso that U is C=O or $CH_2$ when W is $NR_2$;

V is CH, C(OH) or C(halogen) with the proviso that V is CH when U is $NR_2$;

$R_1$ is loweralkyl, cycloalkylalkyl, benzyl, (alpha, alpha)-dimethylbenzyl, 4-methoxybenzyl, halobenzyl, 4-hydroxybenzyl, (1-naphthyl)methyl, (2-naphthyl)methyl, (unsubstituted heterocyclic)methyl, (substituted heterocyclic)methyl wherein unsubstituted or substituted heterocyclic is as defined above, phenethyl, 1-benzyloxyethyl, phenoxy, thiophenoxy or anilino, provided that B is $CH_2$ or CHOH or A is hydrogen when $R_1$ is phenoxy, thiophenoxy or anilino; and

$R_3$ is loweralkyl, loweralkenyl, ((alkoxy)alkoxy)alkyl, carboxyalkyl, (thioalkoxy)alkyl, azidoalkyl, aminoalkyl, (alkyl)aminoalkyl, dialkylaminoalkyl, (alkoxy)(alkyl)aminoalkyl, (alkoxy)aminoalkyl, benzyl or heterocyclic ring substituted methyl; or an acid halide or activated ester derivative thereof; with a compound of the formula:

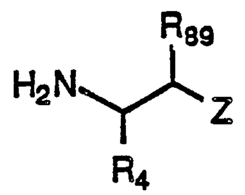

wherein $R_4$ is loweralkyl, cycloalkylmethyl or benzyl;

$R_{89}$ is $-OR_{90}$ or $-NHR_{91}$ wherein $R_{90}$ is hydrogen or an O-protecting group and $R_{91}$ is hydrogen or an N-protecting group; and

Z is

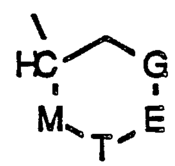

or

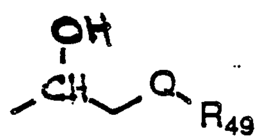

wherein M is O, S or NH, T is C=O, C=S, S, S(O), $S(O)_2$ or $CH_2$, E is O, S, $NR_6$ wherein $R_6$ is hydrogen, loweralkyl, hydroxyalkyl, hydroxy, alkoxy, amino, or alkylamino, or E is $CR_6R_{42}$ wherein $R_6$ is as defined above and $R_{42}$ is hydrogen or loweralkyl or E is $C=CR_{43}R_{44}$ wherein $R_{43}$ and $R_{44}$ are independently selected from hydrogen and loweralkyl, G is absent, $CH_2$, or $NR_{11}$ wherein $R_{11}$ is hydrogen or loweralkyl, with the proviso that when G is $NR_{11}$ then $R_6$ is loweralkyl or hydroxyalkyl, Q is $CR_{45}R_{46}$ wherein $R_{45}$ and $R_{46}$ are independently selected from hydrogen and loweralkyl or Q is $C=CR_{47}R_{48}$ wherein $R_{47}$ and $R_{48}$ are independently selected from hydrogen and loweralkyl, and $R_{49}$ is $-CH_2OH$, carboxy, alkoxycarbonyl or $-CONR_{50}R_{51}$ wherein $R_{50}$ is hydrogen or loweralkyl and $R_{51}$ is hydrogen, loweralkyl, aminoalkyl, alkylaminoalkyl, dialkylaminoalkyl or alkoxyalkyl.